(19) Europäisches Patentamt
European Patent Office
Office européen des brevets

(11) EP 4 242 207 A1

(12) **EUROPEAN PATENT APPLICATION**
published in accordance with Art. 153(4) EPC

(43) Date of publication:
**13.09.2023 Bulletin 2023/37**

(21) Application number: **21888652.1**

(22) Date of filing: **05.11.2021**

(51) International Patent Classification (IPC):
***C07D 471/00*** (2006.01) ***C07D 471/04*** (2006.01)
***C07D 401/00*** (2006.01) ***C07D 401/02*** (2006.01)
***A61K 31/517*** (2006.01) ***A61K 31/496*** (2006.01)
***A61P 35/00*** (2006.01)

(52) Cooperative Patent Classification (CPC):
**A61K 31/496; A61K 31/517; A61P 35/00; C07D 401/00; C07D 401/02; C07D 471/00; C07D 471/04**

(86) International application number:
**PCT/CN2021/128977**

(87) International publication number:
**WO 2022/095960 (12.05.2022 Gazette 2022/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.11.2020 CN 202011229677**
**01.11.2021 CN 202111280310**

(71) Applicant: **Tyligand Bioscience (Shanghai) Limited**
**Shanghai 201203 (CN)**

(72) Inventors:
- **SHANG, Erchang**
**Shanghai 201112 (CN)**
- **ZHONG, Boyu**
**Irving, TX 75038 (US)**
- **ZHANG, Tony Yantao**
**Fishers, IN 46037 (US)**
- **SONG, Guanglin**
**Shanghai 200125 (CN)**
- **WANG, Ruixiang**
**Shanghai 201299 (CN)**
- **TANG, Bingqing**
**Shanghai 200125 (CN)**
- **HU, Xubo**
**Shanghai 200120 (CN)**

(74) Representative: **Vossius & Partner Patentanwälte Rechtsanwälte mbB**
**Siebertstraße 3**
**81675 München (DE)**

# (54) KRAS INHIBITORS FOR TREATMENT OF CANCERS

(57) Compounds which have the structure represented by formula (I) and can be used as KRas inhibitors, pharmaceutical compositions comprising this type of compounds, a method of preparing this type of compounds, and uses of these compounds in the treatment of cancers.

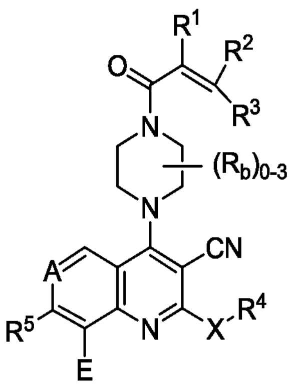

(I)

## Description

### Technical Field

The disclosure relates to the field of pharmaceutical chemistry. More specifically, the disclosure relates to a class of compounds with novel structures useful as inhibitors of KRas protein, pharmaceutical compositions comprising the compounds, methods for preparing the compounds, and uses of these compounds in the treatment of cancers.

### Background

Ras, the rat sarcoma oncogene homolog, represents a group of closely related monomeric globular proteins belonging to the GTPase protein family. Specifically, under normal physiological conditions, Ras is activated by growth factors and various other extracellular signals and is responsible for regulating functions such as cell growth, survival, migration, and differentiation. The functions of Ras proteins are performed by alternating between GDP-bound and GTP-bound states, namely "molecular switch" (Alamgeer et al., Current Opin Pharmacol. 2013, 13:394-401). GDP-bound Ras is inactive, i.e. dormant or off, and the signaling system is turned off. It is activated when exposed to some growth-promoting stimuli, for example, it can be induced by guanine nucleotide exchange factor (GEF) to release GDP and binds to GTP, and as a result, Ras is "turned on" thereby converting to the active form of Ras, which recruits and activates various downstream effectors to carry out signal transmission, and is able to transmit signals from the cell surface into the cytoplasm, thereby controlling numerous key cellular processes such as differentiation, survival, and proliferation (Zhi Tan et al., Mini-Reviews in Medicinal Chemistry, 2016, 16, 345-357).

Ras has GTPase activity, which cleaves the terminal phosphate of GTP and converts it into GDP, that is, it transforms itself into an inactive state. But intrinsic enzymetic activity of Ras is relatively low, and the conversion of GTP-Ras to GDP-Ras needs exogenous GTPase activating protein (GAP). GAP interacts with Ras and promotes the conversion of GTP to GDP. Therefore, any Ras gene mutation that affects the interaction between Ras and GAP or affects the conversion of GTP to GDP leads to prolonged activation of Ras, thereby continuously conveying signals for growth and division to cells, stimulating continuous cell proliferation, and ultimately leading to tumor formation and development.

Among the genes associated with human tumors, there are three ubiquitously expressed Ras genes H-RAS, K-RAS and N-RAS, which encode highly homologous HRas, NRas and KRas proteins of about 21 KDa, respectively. In 1982, for the first time, Ras was identified mutationally activated in cancer cell lines (Chang, E.H. et al., Proceedings of the National Academy of Sciences of the United States of America, 1982, 79(16), 4848-4852). Subsequently, large genome sequencing studies in different cancer types revealed that, Ras protein is mutated in more than 30% of cancer types, with the highest mutation rate especially in pancreatic cancer (>90%), colon cancer (45%), and lung cancer (35%). Transgenic and genetically engineered mouse models have also revealed that mutated Ras protein is sufficient to drive and induce multiple types of cancer, and Ras oncogenes are also crucial for the maintenance and progression of tumors in multiple cancer types, for example, RNA interference has been shown to slow tumor growth in Ras mutant cancer cell lines and animal models of cancer. These studies make Ras tumor proteins widely accepted as very attractive anticancer drug targets in the pharmaceutical field.

Studies have shown that KRas is the most commonly mutated member of the Ras family, and KRas mutations can be found in about 85% of Ras mutation-driven cancers; the vast majority of Ras mutations occur at codons G12, G13, and Q61, of which about 80% of KRas mutations occur at glycine of codon 12, such as G12C mutation, G12D mutation, and G13D mutation etc. KRas mutations are frequently found in pancreatic cancer, lung adenocarcinoma, colorectal cancer, gallbladder cancer, thyroid cancer, and cholangiocarcinoma, and can also be found in 25% of patients with non-small cell lung cancer (McCormick, F. et al., Clinical Cancer Research 21(8), 1797-1801, 2015).Therefore, KRas mutant protein has become the most important branch in Ras drug target study, and the development of their inhibitors is also regarded as a very promising R&D direction in anticancer/oncology drug development.

However, Ras-targeted drug development over the past three decades has shown that, due to the smooth surface of Ras protein, the lack of obvious groove or pocket structures for binding small molecule inhibitors, and its very high affinity for guanine substrates (picomolar grade), the development of its small molecule inhibitors has been caught in an insoluble dilemma, and thus Ras has long been considered as an "undruggable" target in the industry. Nevertheless, sustained efforts to target Ras mutant proteins have yielded some encouraging results, and a series of Ras inhibitors have been developed that inhibit Ras, especially KRas mutations through multiple pathways, including direct targeting of Ras, inhibition of Ras expression level, disruption of Ras protein localization, targeting synthetic lethal components, targeting Ras-GEF interactions, targeting Ras and effector interactions, and targeting dimerization of Ras (Zhi Tan et al., Mini-Reviews in Medicinal Chemistry, 2016, 16, 345-357).

Ras inhibitors that have been developed, such as those directed against the most commonly mutated protein, KRas-G12C, include allosteric covalent inhibitors, such as the 6H05 series, quinazoline series, ARS series, and tetrahydropyridopyrimidine series, as well as orthogonal binding covalent inhibitors, which are reviewed in the literature (Duan

Ni et al., Pharmacology & Therapeutics, https://doi.org/10.1016/j.pharmthera.2019.06.007).Various structural types of KRas inhibitors have also been described in several patents, such as CN10256421, US2019/0144444A1 and WO2019/110751A1.

However, the above mentioned KRas inhibitors still have problems to be solved, and their "druggability" remains unsatisfactory. For example, many KRas-dependent cancers are susceptible to develop resistance to such targeted therapeutics, have side effects such as off-target effects, chemically active metabolites, poor metabolic stability, or generate immunogenic covalent adducts (John P. O'Bryan et al., Pharmacological Research 139 (2019) 503-511; Duan Ni et al., Pharmacology & Therapeutics, https://doi.org/10.1016/j.pharmthera.2019.06.007). Therefore, there remains a clinical demand for more alternative KRas inhibitors that are expected to have comparable or improved KRas inhibitory activity to existing inhibitors, improved druggability, better safety such as fewer drug interactions or metabolic properties, improved pharmacokinetic properties, and/or greater selectivity for different patient populations or specific tumor types.

The present disclosure provides novel structural compounds having inhibitory activity against KRas mutant protein. These compounds, especially the preferred compounds of the disclosure, have the following technical effects due to their improved structural patterns as compared to the existing KRas mutant protein inhibitors of the prior art:

(1) retain comparable or enhanced, or even significantly enhanced inhibition activities for KRas mutant protein and related cancer cell proliferation;

(2) different biological activity profiles for different disease types or patient populations;

(3) improved metabolic stability, thereby bringing better pharmacokinetic properties; and

(4) improved physicochemical properties, thereby having good druggability and safety, e.g. easier to absorb in the body.

**Summary of the disclosure**

The inventors have found through researches that, compounds of formula (I) defined herein, their isomers or pharmaceutically acceptable salts or solvates are effective inhibitors for Ras mutations, especially KRas mutant protein, which can inhibit Ras mutations in cells, especially KRas activity, and can be used to treat or prevent diseases or conditions mediated by Ras mutations, especially KRas mutant protein or benefiting from inhibition of Ras mutations, especially KRas mutant protein, especially can inhibit abnormal cell proliferation by inhibiting Ras mutations, especially KRas mutant protein, thereby treating or preventing tumors or cancers.

The first aspect of the present disclosure provides a compound of formula (I), an isomers or a pharmaceutically acceptable salt or solvate thereof,

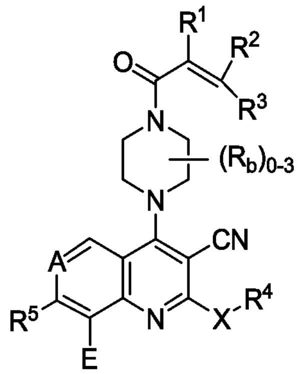

(I)

wherein,

A is selected from C-$R_a$ or N, wherein $R_a$ is selected from halogen, CN, nitro, $C_{3-6}$ cycloalkyl or $C_{1-6}$ alkyl optionally substituted by halogen;

$R^1$, $R^2$ and $R^3$ are each independently selected from H, halogen or $C_{1-6}$ alkyl, wherein the alkyl is optionally substituted by a group independently selected from halogen, $-N(R_c)_2$, $-OR_c$ or 3-8 membered heterocycloalkyl;

$R_b$ at each occurrence is independently selected from H, halogen, CN, or $C_{1-6}$ alkyl optionally substituted by halogen or CN;

X is selected from a bond, -O- or -NH-;

$R^4$ is selected from H, halogen, $C_{1-6}$ alkyl, -$C_{0-6}$ alkyl-$C_{6-10}$ aryl, -$C_{0-6}$ alkyl-$C_{3-8}$ cycloalkyl, -$C_{0-6}$ alkyl-$C_{3-8}$ cycloalkenyl, -$C_{0-6}$ alkyl-3-8 membered heterocycloalkyl, -$C_{0-6}$ alkyl-3-8 membered heterocycloalkenyl, -$C_{0-6}$ alkyl-5-10 membered heteroaryl, wherein the alkyl, aryl, cycloalkyl, cycloalkenyl, heterocycloalkyl, heterocycloalkenyl and heteroaryl groups are optionally substituted by one or more groups independently selected from halogen, $C_{1-6}$ alkyl optionally substituted by halogen, $-(CR_cR_c)_{0-6}-SR_c$, $-(CR_cR_c)_{0-6}-(CO)_{0-1}-OR_c$, $-(CR_cR_c)_{0-6}-(CO)_{0-1}-N(R_c)_2$, wherein the $-(CR_cR_c)_{0-6}-(CO)_{0-1}-N(R_c)_2$ attached to the aryl, cycloalkyl, cycloalkenyl, heterocycloalkyl, heterocycloalkenyl or heteroaryl, via the group connected to N and together with the ring atom to which it is attached and the adjacent atom, optionally form a 4-7 membered nitrogen-containing heterocyclic ring;

$R_c$ at each occurrence is independently selected from H or $C_{1-6}$ alkyl optionally substituted by halogen, or wherein two $R_c$ attached to the same carbon or nitrogen atom, together with the carbon atom or nitrogen atom to which they are attached, independently form a 3-6 membered cyclic group;

E is selected from halogen, -O-$R_d$ or -N($R_d$)$_2$-, wherein each $R_d$ is independently H or $C_{1-6}$ alkyl optionally substituted by halogen;

$R^5$ is

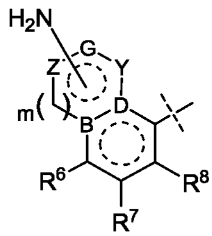

;

m is 0 or 1; 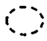 represents an aromatic ring;

B and D are each independently selected from N or C;

Z, G and Y are each independently selected from C, N, O or S;

$R^6$, $R^7$ and $R^8$ are each independently selected from H, halogen and $C_{1-6}$ alkyl optionally substituted by halogen;

with the proviso that B and D are not both N; and at most three of Z, G, Y, D and B are not C.

The disclosure further provides a compound of formula (II), an isomer, a pharmaceutically acceptable salt or a solvate thereof,

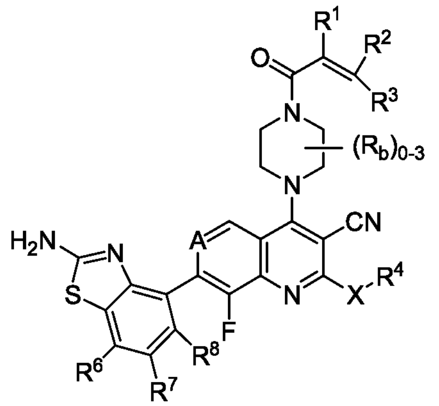

(II)

wherein, $R^1$, $R^2$, $R^3$, $R^4$, $R^6$, $R^7$, $R^8$, A and $R^b$ each has the meaning given by the formula (I) above.

The second aspect of the present disclosure provides a pharmaceutical combination comprising a compound of formula (I) or (II), an isomer or a pharmaceutically acceptable salt or a solvate thereof.

The third aspect of the present disclosure provides a compound of formula (I) or formula (II), an isomer or a pharmaceutically acceptable salt or a solvate thereof, used as a medicine.

The fourth aspect of the present disclosure provides a compound of formula (I) or formula (II), an isomer or a pharmaceutically acceptable salt or a solvate thereof for the treatment and/or prevention of diseases mediated by a Ras mutation, particularly a KRas mutation, preferably KRas G12C, KRas G12D or KRas G13D mutation, and most preferably KRas G12C mutaition.

The fifth aspect of the present disclosure provides use of a compounds of formula (I) or formula (II), an isomer or a pharmaceutically acceptable salt or a solvate thereof or a pharmaceutical composition comprising them in the manufacture of a medicament for treating and/or preventing diseases mediated by a Ras mutation, particularly a KRas mutation, preferably KRas G12C, KRas G12D or KRas G13D mutation, and most preferrably KRas G12C mutation.

The sixth aspect of the present disclosure provides a method for treating and/or preventing diseases mediated by a Ras mutation, particularly a KRas mutation, preferably KRas G12C, KRas G12D or KRas G13D mutation, and most preferably KRas G12C mutation, comprising administering a therapeutically effective amounts of a compounds of formula (I) or formula (II), an isomers or a pharmaceutically acceptable salt or a solvate thereof or a pharmaceutical combination comprising them, to a subject in need thereof.

The seventh aspect of the present disclosure provides a method for preparing a compound of formula (I) or formula (II), an isomer or a pharmaceutically acceptable salt or a solvate thereof.

The eighth aspect of the present disclosure provides a pharmaceutical combination comprising a compound of formula (I) or formula (II), an isomer or a pharmaceutically acceptable salt or a solvate thereof and one or more other pharmaceutical active agents.

## Detailed description

### Definitions

Unless defined otherwise, the terms used in the specification and claims have the meanings indicated below. In the case that a specific term or phrase is not specifically defined, it should not be regarded as uncertain or unclear, but should be understood according to the ordinary meaning in the art. Many of the groups defined herein are optionally substituted, and the list of substituents given in this definition section is only exemplary and does not intend to limit the substituents defined elsewhere in this specification and claims.

The term "Ras mutation" or "Ras mutant protein" used herein refers to a protein encoded and expressed by the Ras gene in which one or more codons are mutated, typically including, but not limited to Ras proteins mutated at glycine at coon G12, ay glycine at codon G13 or at glutamine at codon Q61, such as mutated HRas, NRas, or KRas. Mutations of these residues in the active site of Ras impair the intrinsic or GAP-catalyzed GTPase activity of Ras, resulting in the persistence of GTP-bound Ras.

For the purposes of the present disclosure, "Ras mutation" or "Ras mutant protein" are used interchangeably and generally refer to mutated HRas, NRas, or KRas, such as, but not limited to HRas-G12C (glycine to cysteine mutation at codon G12), NRas-G12C, KRas-G12C, KRas-G12D (glycine to aspartate mutation at codon G12), KRas-G13D (glycine to aspartate mutation at codon G13); specifically refer to the KRas mutant protein, and more specifically refer to the KRas-G12C mutant protein, KRas-G12D mutant protein, KRas-G13D mutant protein, and most specifically refer to the

KRas-G12C mutant protein.

The term "treatment" or "treating" as used herein refers to the administration of one or more of the compounds of formula (I), their isomers, or their pharmaceutically acceptable salts or solvates to a subject, such as a mammal such as a human suffering from the disease or having symptoms of the disease, for use to cure, alleviate, relieve, or affect the disease or the symptoms of the disease. In a specific embodiment of this disclosure, the disease is a Ras mutation-mediated disease as defined below, especially a tumor or cancer.

The term "prevention" or "preventing" as used herein is well known in the art and refers to the administration of one or more of the compounds of formula (I) described herein, their isomers, or their pharmaceutically acceptable salts or solvates, to a subject, such as a mammal such as a human suspected of suffering from or susceptible to the diseases mediated by Ras mutations, especially cancers or tumors as defined herein, to reduce the risk of suffering from the defined diseases. The term "prevention" or "preventing" encompasses the use of the compounds of the present disclosure prior to the diagnosis or determination of any clinical and/or pathological symptoms.

The terms "inhibit" and "reduce" as used herein, or any variations of these terms, refer to the ability of a biologically active agent to reduce the signaling activity of the target by interacting directly or indirectly with the target, and refer to any measurable reduction or complete inhibition of target activity. For example, compared to normal conditions, the activity (e.g., KRas activity) may be reduced by approximately, at most approximately, or at least approximately 5%, 10%, 15%, 20%, 25%, 30%, 35%, 40%, 45%, 50%, 55%, 60%, 65%, 70%, 75%, 80%, 85%, 90%, 95%, 99%, or more, or any range derivable therein.

The term "selective inhibition" as used herein refers to the ability of a biological active agent to preferentially reduce the signaling activity of a target of interest over off-target signaling activity by interacting directly or indirectly with the target. As far as the compound of formula (I) of the present disclosure is concerned, it has the ability to selectively inhibit G12 or G13 mutations of KRas, HRas or NRas proteins, such as G12C mutation, G12D mutation and G13D mutation, relative to various types of mutations occurring at one or more codons of Ras protein, and preferably selectively inhibit G12C mutation of KRas protein. For example, as compared to another specific Ras mutation, the present disclosure has at least 5%, 10%, 15%, 20%, 25%, 30%, 35%, 40%, 45%, 50%, 55%, 60%, 65%, 70%, 75%, 80%, 85%, 90%, 95%, 99% or more, or any range derivable therein, of better activity, for a specific Ras mutation, or as compared to the activity for another specific Ras mutation, has at least 1-, 2-, 3-, 4-, 5-, 10-, 25-, 50-, 100-, 250-, or 500-fold better activity for a specific Ras mutation (e.g. KRas-G12C).

The term "Ras mutation-mediated disease" as used herein refers to a disease in which Ras mutation promotes the occurrence and development of the disease, or inhibition of Ras mutation will reduce the incidence of the disease and reduce or eliminate disease symptoms. For the purposes of the present disclosure, "Ras mutation-mediated disease" refers preferably to KRas mutation-mediated disease, most preferably to KRas-G12C-mediated disease, and more preferably to cancers or tumors.

The term "cancer" or "tumor" as used herein refers to abnormal cell growth and proliferation, either malignant or benign, and all precancerous cells and carcinoma cells and tissues. For all aspects of the present disclosure, the cancer or tumor includes but not limited to lung adenocarcinoma, lung cancer, bone cancer, pancreatic cancer, skin cancer, head and neck cancer, skin or intraocular melanoma, uterine cancer, ovary cancer, rectum cancer, cancer in anal region, stomach cancer, colon cancer, breast cancer, carcinoma of fallopian tube, endometrial cancer, cervical cancer, carcinoma of vagina, carcinoma of vulva, Hodgkin’s disease, esophagus cancer, carcinoma of small intestine, carcinoma of endocrine system, thyroid cancer, parathyroid cancer, adrenal gland cancer, soft tissue sarcoma, urethral cancer, penis cancer, prostate cancer, chronic or acute leukemia, lymphocytic lymphoma, bladder cancer, carcinoma of kidney or ureter, renal cell carcinoma, carcinoma of renal pelvis, central nervous system tumor (CNS), primary CNS lymphoma, spine tumor, brainstem glioma, or pituitary adenoma.

For all aspects of the present disclosure, preferably, the cancer or tumor described is associated with a Ras mutation, particularly a KRas mutation, preferably a KRas G12C, KRas G12D or KRas G13D mutation, most preferably a KRas G12C mutation, including but not limited to the tumor types described above and its preferred scope. Particularly preferred tumors of the present disclosure include lung cancer, lung adenocarcinoma, colon cancer, rectum cancer, pancreatic cancer, endometrial cancer, cholangiocarcinoma, leukemia and ovary cancer.

The terms "subject", "individual" or "patient" as used herein refers to vertebrates. In specific embodiments, vertebrates are mammals. Mammals include but are not limited to farm animals (e.g., cattle), sport animals, pets (e.g., guinea pigs, cats, dogs, rabbits, and horses), primates, mice, and rats. In specific embodiments, mammals are humans.

The term "effective amount" as used herein refers to an amount or dose that is generally sufficient to produce a beneficial therapeutic effect on a cancer or tumor patient in need of the treatment. Those skilled in the art can determine the effective amount or dosage of the active ingredient in the present disclosure by conventional methods in combination with conventional influencing factors.

The term "pharmaceutical combination" used herein refers to the compounds of the present disclosure in combination with other active agents for the purpose of the present disclosure. The other active agents may be one or more additional compounds of the present disclosure, or may be second or additional (e.g., third) compound which is

compatible with the compounds of the present disclosure, i.e. do not adversely affect each other, or have complementary activities, such as these active agents are known to modulate other bioactive pathways, or modulate different components of the bioactive pathways involved in the compounds of the present disclosure, or even overlap with the biological targets of the compounds of the present disclosure. Such active agents are combined appropriately in effective amounts to achieve the desired purposes. The other active agents may be co-administered with the compound of the present disclosure in a single pharmaceutical composition, or administered separately from the compound of the present disclosure in separate discrete units, either simultaneously or sequentially when administered separately. The sequential administration may be close or distant in time.

In one aspect, other active agents that can be used in combination with the compounds of the present disclosure include but are not limited to, chemotherapeutic agents, therapeutic antibodies, and radiation therapy, such as alkylating agents, antimetabolites, cell cycle inhibitors, mitotic inhibitors, topoisomerase inhibitors, antihormonal drugs, angiogenesis inhibitors or cytotoxic agents.

The term "pharmaceutically acceptable" as used herein refers to molecular entities and compositions that do not produce adverse, allergic or other adverse effects when administered to animals such as humans in appropriate amounts.

The term "pharmaceutically acceptable salt" as used herein refers to those salts which retain the biological effectiveness and properties of the parent compound and which are not biologically or otherwise undesirable, including acid addition salts and base addition salts. "Pharmaceutically acceptable acid addition salts" may be formed from compounds having a free base with inorganic or organic acids, inorganic acids such as hydrochloric acid, hydrobromic acid, sulfuric acid, nitric acid, carbonic acid, phosphoric acid, etc., organic acids may be selected from aliphatic, aliphatic, aromatic, aromatic-aliphatic, heterocyclic, carboxylic and sulfonic organic acids, such as formic acid, acetic acid, propionic acid, glycolic acid, gluconic acid, lactic acid, pyruvate, oxalic acid, malic acid, maleic acid, malonic acid, succinic acid, fumaric acid, tartaric acid, citric acid, aspartic acid, ascorbic acid, glutamic acid, anthranilic acid, benzoic acid, cinnamic acid, mandelic acid, pamoic acid, phenylacetic acid, methanesulfonic acid, ethanesulfonic acid, benzenesulfonic acid, p-toluene sulfonic acid, salicylic acid, etc. "Pharmaceutically acceptable base addition salts" include those derived from inorganic bases such as salts of sodium, potassium, lithium, ammonium, calcium, magnesium, iron, zinc, copper, manganese, and aluminum, as well as salts derived from pharmaceutically acceptable organic nontoxic bases, including but not limited to primary amines, secondary amines, and tertiary amines, and substituted ammonium, including naturally occurring substituted amines, cyclic amines, and alkaline ion exchange resins, such as ammonia, isopropylamine, trimethylamine, diethylamine, triethylamine, tripropylamine, ethanolamine, diethanolamine, 2-dimethylaminoethanol, 2-diethylaminoethanol, tromethamine, dicyclohexylamine, lysine, arginine, histidine, caffeine, procaine, hybamine, choline, betaine, ethanediamine, glucosamine, methylglucosamine, triethanolamine, theobromine, purine, piperazine, piperidine, N-ethylpiperidine, and polyamine resins etc.

The term "isomer" as used herein refers to any stereoisomer, enantiomeric mixture, including racemate, diastereomeric mixture, geometric isomer, atropisomer, and/or tautomer that may exist in the structure of a compound. The determination and separation methods of the stereochemistry of the isomers are well known to those skilled in the art (S. P. Parker, Ed., McGraw-Hill Dictionary of Chemical Terms (1984) McGraw-Hill Book Company, New York; and Eliel, E. and Wilen, S., "Stereochemistry of Compounds", John Wiley & Sounds, Inc., New York, 1994). The present disclosure therefore covers all possible isomeric forms of the compounds of formula (I) defined above, and pharmaceutically acceptable salts or solvates thereof.

The "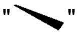" or "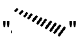" used in the structural formula or structural fragment of a compound herein indicates the stereocenter, i.e., the absolute configuration of the chiral center, and accordingly in the naming of the compounds or intermediates provided by the present disclosure, R and S indicate the absolute configuration of the chiral center. The determination of the absolute configuration is well known to those skilled in the art.

The " " used in the structural fragments shown in present disclosure indicates that the attached bond is the bond that the fragment connecting to the rest structure of the molecule.

The term "solvate" as used herein refers to a solvent addition form containing stoichiometric or non-stoichiometric amounts of solvent, including any solvated form of the compounds of the present disclosure, including, for example, solvates with water, such as hydrates, or a solvate with an organic solvent, such as methanol, ethanol or acetonitrile, i.e. as methanolate, ethanolate or acetonitrile-olate, respectively; or in any polymorphic form. It should be understood that such solvates of the compounds of the present disclosure also include solvates of the pharmaceutically acceptable salts of the compounds of the present disclosure.

The term "isotopic variant" as used herein refers to a compound that contains unnatural proportions of isotopes on one or more of the atoms constituting the compound. The compounds of the present disclosure may contain unnatural proportions of atomic isotopes at one or more atoms constituting the compounds, thereby forming isotopic variants of the compounds of the present disclosure or pharmaceutically acceptable salts thereof, whether radioactive or not, are intended to within the scope of the present disclosure. Examples of isotopes that can be incorporated into the compounds

of the present disclosure and pharmaceutically acceptable salts thereof include, for example, $^{2}H$, $^{3}H$, $^{13}C$, $^{14}C$, $^{15}N$, $^{17}O$, $^{18}O$, $^{31}P$, $^{32}P$, $^{35}S$, $^{18}F$ and $^{36}Cl$. It should be understood that isotopic variations of the compounds of the present disclosure and pharmaceutically acceptable salts thereof may generally be prepared by conventional methods using appropriate isotopic variations of suitable reagents. For example, certain isotopic variations of the compounds of the present disclosure incorporating radioisotopes (e.g., $^{3}H$ or $^{14}C$) and their pharmaceutically acceptable salts may be used for tissue distribution studies of drugs and/or substrates. Tritiated, i.e, $^{3}H$ and carbon-14, i.e. $^{14}C$ isotopes are particularly preferred due to ease of preparation and detectability. Furthermore, substitution with isotopes such as deuterium, i.e. 2H, may afford certain therapeutic advantages resulting from greater metabolic stability, for example increased *in vivo* half-life or reduced dosage requirements, and thus may be preferred in certain circumstances. In addition, the compounds of the present disclosure substituted with positron emitting isotopes (e.g. $^{11}C$, $^{18}F$, $^{15}O$ and $^{13}N$) can be prepared and can be used in positron tomography (PET) studies for substrate acceptor occupancy detection.

The term "metabolite" as used herein refers a product produced by metabolism of a specific compound *in vivo.* Such products may, for example, result from oxidation, reduction, hydrolysis, amidation, deamidation, esterification, de-esterification, enzymatic cleavage, etc. of the administered compound. Identification and analysis of metabolite products is performed in a manner well known to those skilled in the art.

The term "prodrug" as used herein refers to a compound that can be converted under physiological conditions or by solvolysis to a biologically active compound described herein, e.g., a compound of formula (I). Accordingly, the term "prodrug" refers to a pharmaceutically acceptable precursor of a biologically active compound. In some aspects, a prodrug is inactive when administered to a subject, but is converted to an active compound in vivo, e.g., by hydrolysis. Prodrug compounds generally offer the advantages of solubility, histocompatibility, or delayed release in mammalian organisms (see e.g., Bundgard, H., Design of Prodrugs (1985), pp. 7-9, 21-24 (Elsevier, Amsterdam). Discussions of prodrugs can be found in Higuchi, T. et al' s ACS Symposium Series, Volume 14, and "Bioreversible Carriers in Drug Design", Edward B. Roche (B. Roche), Pharmaceutical Association & Pergamon Press, 1987, USA, the entire contents of which are incorporated herein as a reference. The term "prodrug" also refers to any covalently bonded carrier that releases active compounds in vivo when administered to mammalian subjects. Prodrugs of the active compounds as described herein are generally prepared by modifying functional groups present on the active compound such that the modification is cleaved to the parent active compound during routine manipulation or in vivo. Prodrugs include compounds wherein a hydroxyl, amino or thiol group is bonded to any group that cleaves to form a free hydroxyl, free amino or free thiol when the prodrug is administered to a mammal. Examples of prodrugs include, but are not limited to, acetate, formate and benzoate derivatives of hydroxy functional groups, or acetamide, formamide and benzamide derivatives of amine functional groups in active compounds. In some embodiments, the prodrugs include phosphate-containing prodrugs, borate-containing prodrugs, phosphorothioate-containing prodrugs, sulfate-containing prodrugs, peptide-containing prodrugs prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs and 5-fluorocytosine and 5-fluorouridine prodrugs.

The term "pharmaceutically acceptable excipient or carrier" used herein refers to one or more compatible solid or liquid fillers or gel substances that are suitable for human use and have sufficient purity and low toxicity, examples of which include, but are not limited to cellulose and its derivatives (e.g., sodium carboxymethylcellulose, cellulose acetate, etc.), gelatin, talc, solid lubricants (e.g., magnesium stearate), calcium sulfate, vegetable oil, polyols (e.g., propylene glycol, glycerol, mannitol, sorbitol, etc.), emulsifiers (e.g., Tween), wetting agents (e.g., sodium dodecyl sulfate), coloring agents, flavoring agents, stabilizers, antioxidants, preservatives, etc.

The term "halogen" or "halo" as used herein means F, Cl, Br or I. Furthermore, the term "halogen-substituted" groups is intended to include monohalogenated or polyhalogenated groups in which one or more same or different halogens replace one or more hydrogens in the group.

The term "alkyl" as used herein refers to a linear or branched saturated hydrocarbon group composed of carbon atoms and hydrogen atoms. Specifically, the alkyl group has 1-10, such as 1 to 6, 1 to 5, 1 to 4, 1 to 3 or 1 to 2 carbon atoms. For example, as used herein, the term "$C_1$-$C_6$ alkyl" refers to a linear or branched saturated hydrocarbon group having 1 to 6 carbon atoms, examples include methyl, ethyl, propyl (including n-propyl and isopropyl), butyl (including n-butyl, isobutyl, sec-butyl, or tert-butyl), pentyl (including n-pentyl, isopentyl, neopentyl), n-hexyl, 2-methylpentyl, etc. Specific alkyl groups have 1 to 3 carbon atoms.

The term "alkoxy" as used herein refers to the group-O-alkyl, wherein the alkyl group has the meaning described herein. Specifically, the term includes the group -O-$C_{1-6}$alkyl, and more specifically the -O-$C_{1-3}$alkyl. Representative examples of alkoxy include, but are not limited to, methoxy, ethoxy, propoxy (including *n*-propoxy, isopropoxy), butoxy (including *n*-butoxy, isobutoxy, *tert*-butoxy), pentoxy (including *n*-pentoxy, isopropoxy, neopentoxy), hexoxy (including n-hexoxy, isohexoxy), etc. Specific alkoxyl groups have 1 to 3 carbon atoms.

The term "alkylthio" as used herein refers to -S-alkyl, wherein the alkyl is as defined above for "alkyl".. Specifically, the term includes the group -S-$C_{1-6}$ alkyl, and more specifically -S-$C_{1-3}$ alkyl. Representative examples of alkylthio include, but are not limited to, methylthio, ethylthio, propylthio (including *n*-propylthio, isopropylthio), butylthio (including

*n*-butylthio, isobutylthio, *tert*-butylthio), pentylthio (including n-pentylthio, isopentylthio, neopentylthio), hexylthio (including n-hexylthio, isohexylthio), etc. Specific alkylthio groups have 1 to 3 carbon atoms.

The term "halogen-substituted $C_1$-$C_6$ alkyl" as used herein refers to the $C_1$-$C_6$ alkyl group described above, in which one or more (e.g., 1, 2, 3, 4, or 5) hydrogen atoms are replaced by halogens. Those skilled in the art should understand that halogens can be either the same or different and can be located on the same or different C atoms when there is more than one halogen substituent. Examples of "halogen-substituted $C_1$-$C_6$ alkyl" groups are $-CH_2F$, $-CHF_2$, $-CF_3$, $-CCl_3$, $-C_2F_5$, $-C_2Cl_5$, $-CH_2CF_3$, - $CH_2Cl$, $-CH_2CH_2CF_3$, or $-CF(CF_3)_2$, etc.

The term "cycloalkyl" as used herein refers to monocyclic, fused polycyclic, bridged polycyclic, or spiro non-aromatic saturated monovalent hydrocarbon ring structures having the specified number of ring atoms. The cycloalkyl may have 3 to 12 carbon atoms (i.e., $C_3$-$C_{12}$ cycloalkyl), e.g., 3 to 10, 3 to 8, 3 to 7, 3 to 6, and 5 to 6 carbon atoms. Examples of suitable cycloalkyl include, but are not limited to, monocyclic structures, such as cyclopropyl, cyclobutyl, cyclopentyl, cyclohexyl, cycloheptyl, or cyclooctyl; or polycyclic (e.g., dicyclic) structures, including spiro, fused, or bridging systems, such as bicyclic[1.1.1]pentyl, bicyclo[2.2.1]heptyl, spiro[3.4]octyl, bicyclo[3.1.1]hexyl, bicyclo[3.1.1]heptyl, or bicyclo[3.2.1]octyl, etc.

The term "cycloalkenyl" as used herein refers to a monocyclic, fused polycyclic, bridged polycyclic, or spiro non-aromatic unsaturated hydrocarbon ring structure with a specified number of ring atoms containing at least one (e.g., 1, 2, or 3) carbon-carbon double bond. The cycloalkenyl may have 3 to 12 carbon atoms (ie, $C_3$-$C_{12}$ cycloalkenyl), such as 3 to 10, 3 to 8, 3 to 7, 3 to 6, and 5 to 6 carbon atoms. Examples of suitable cycloalkenyl include, but are not limited to, monocyclic structures such as cyclopropenyl, cyclobutenyl, cyclopentenyl, cyclopentadienyl, cyclohexenyl, cyclohexadienyl, cycloheptenyl, cycloheptadienyl, cycloheptatrienyl, or cyclooctenyl.

The term "heterocycloalkyl" as used herein refers to a monocyclic, fused polycyclic, spirocyclic or bridged polycyclic non-aromatic saturated ring structure comprising one or more (e.g., 1, 2, 3 or 4) heteroatoms independently selected from O, N and S, or their N-oxides, or their S-oxides or S-dioxides. A heterocycloalkyl may have 3 to 12 ring members (may be referred to as a 3-12 membered heterocycloalkyl), for example 3 to 10 ring members, 3 to 8 ring members, 3 to 7 ring members, 4 to 7 ring members, 4 to 6 ring members, 5 to 6 ring members. Heterocycloalkyl typically contains up to 4 (e.g., 1, 2, 3 or 4) heteroatoms. Examples of suitable heterocycloalkyl include, but are not limited to azetidinyl, oxetanyl, thietanyl, pyrrolidinyl (such as 1-pyrrolidinyl, 2-pyrrolidinyl and 3-pyrrolidinyl), tetrahydrofuranyl (such as 1- tetrahydrofuryl, 2-tetrahydrofuryl and 3-tetrahydrofuryl), tetrahydrothiophenyl (such as 1-tetrahydrothiophenyl, 2-tetrahydrothiophenyl and 3-tetrahydrothiophenyl), piperidyl (such as 1-piperidyl, 2-piperidyl, 3-piperidyl and 4-piperidyl), tetrahydropyranyl (such as 4-tetrahydropyranyl), tetrahydrothiopyranyl (such as 4-tetrahydrothiopyranyl), morpholinyl (e.g. morpholino), thiomorpholinyl, dioxanyl, piperazinyl or azepanyl, diazepanyl such as 1,4-diazepanyl, 3,6-diaza-bicyclo[3.1.1]heptyl or 3-aza-bicyclo[3.2.1]octyl. The atom in the heterocycloalkyl that is bonded to the rest of the compound can be a carbon atom or a heteroatom, as long as it is chemically feasible.

Preferred heterocycloalkyl such as

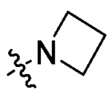 , 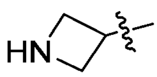 , 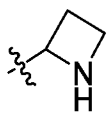 , 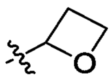 , 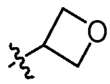 ,

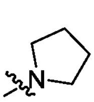 , 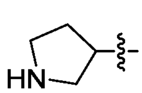 , 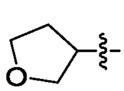 , 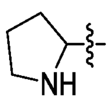 , 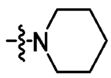 , 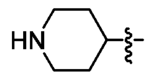 , 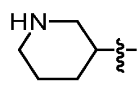 , 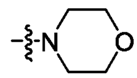 ,

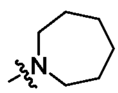 , 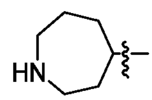 ;

it should be understood that structures with asymmetric centers cover their racemic and/or single enantiomeric forms, e.g.,

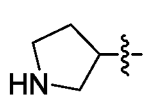

may be

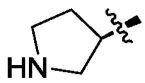 and/or 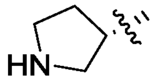 .

The term "heterocycloalkenyl" as used herein refers to a "heterocycloalkyl" as defined herein comprising at least one (e.g., 1, 2 or 3) double bond. Examples of suitable heterocycloalkenyl include, but are not limited to,

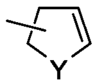 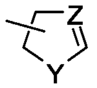 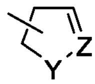 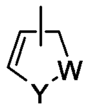

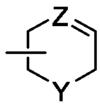 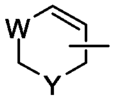 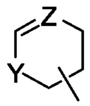 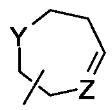

wherein each W is selected from $CH_2$, NH, O, and S; each Y is selected from NH, O, C(=O), $SO_2$, and S; and each Z is selected from N and CH provided that each ring contains at least one atom selected from N, O or S. Such as pyrrolinyl (such as 1-pyrrolinyl, 2-pyrrolidinyl, 3-pyrrolinyl, 4-pyrrolinyl or 5-pyrrolinyl), dihydrofuryl (such as 1-dihydrofuranyl, 2 -dihydrofuryl, 3-dihydrofuryl, 4-dihydrofuryl or 5-dihydrofuryl), dihydrothienyl (e.g., 1-dihydrothienyl, 2-dihydrothienyl, 3-dihydrothienyl or 4-dihydrothienyl), tetrahydropyridyl (e.g., 1-, 2-, 3-, 4-, 5- or 6-tetrahydropyridyl), tetrahydropyranyl (e.g., 4 -tetrahydropyranyl) or tetrahydrothiopyranyl (e.g., 4-tetrahydrothiopyranyl).

The term "aryl" as used herein refers to a monovalent aromatic hydrocarbon group derived by removing a hydrogen atom from a single carbon atom in an aromatic ring system. Specifically, aryl refers to a monocyclic or fused polycyclic aromatic ring structure with a specified number of ring atoms. Specifically, the term includes groups containing 6 to 14, e.g., 6 to 10, preferably 6 ring members. Specific aryl groups include phenyl and naphthyl, and the most specific aryl group is phenyl.

The term "heteroaryl" as used herein refers to a monocyclic or fused polycyclic aromatic ring with specified members of ring atoms comprising one or more (e.g., 1, 2, 3 or 4) heteroatoms independently selected from O, N and S, or N-oxide, or S-oxide or S-dioxide thereof. Specifically, this aromatic ring structure may have 5 to 10 ring members. A heteroaryl may be, for example, a 5-6 membered single ring, or fused of two 6-membered rings, fused of two 5-membered rings, fused of a 6-membered ring and a 5-membered ring, or fused of a 5-membered ring and a 4-membered ring. The heteroaryl ring may generally contain up to 4 heteroatoms, more usually up to 3 heteroatoms, more usually up to 2, for example a single heteroatom independently selected from O, N and S, wherein N and S can be in an oxidized state such as N-oxide, S=O, or $S(O)_2$. In one embodiment, the heteroaryl ring contains at least one ring nitrogen atom, at least one ring sulfur atom, or at least one ring oxygen atom. For example, heteroaryl may be a fused ring containing 1, 2, 3 or 4 heteroatoms independently selected from N, O or S, i.e. examples include benzofuran, benzothiophene, indole, benzimidazole, indazole, benzotriazole, pyrrolo[2,3-b]pyridine, pyrrolo[2,3-c]pyridine, pyrrolo[3,2-c]pyridine, pyrrolo[3,2-b]pyridine, imidazo[4,5-b]pyridine, imidazo[4,5-c]pyridine, pyrazolo[4,3-d]pyridine, pyrazolo[4,3-c]pyridine, pyrazolo[3,4-c]pyridine, pyrazolo[3,4-b]pyridine, isoindole, purine, indolizine, imidazo[1,2-a]pyridine, imidazo[1,5-a]pyridine, pyrazolo[1,5-a]pyridazine, pyrrolo[1,2-b]pyrimidine, imidazo[1,2-c]pyrimidine, 5H-pyrrolo[3,2-b]pyrazine, 1H-pyrazolo[4,3-b]pyrazine, 1H-pyrazolo[3,4-d]pyrimidine, 7H-pyrrole[2,3-d]pyrimidine, quinoline, isoquinoline, cinnoline, quinazoline, quinoxaline, phthalazine, 1,6-naphthyridine, 1,7-naphthyridine, 1,8-naphthyridine, 1,5-naphthyridine, 2,6-naphthyridine, 2,7-naphthyridine, pyrido[3,2-d]pyrimidine, pyrido[4,3-d]pyrimidine, pyrido[3,4-d]pyrimidine, pyrido[2,3-d]pyrimidine, pyrido[2,3-b]pyrazine, pyrido[3,4-b]pyrazine, pyrimido[5,4-d]pyrimidine, pyrazino[2,3-b]pyrazine and pyrimido[4,5-d]pyrimidine. For example, a heteroaryl may be a 5-6 membered heteroaryl containing 1 or 2 heteroatoms independently selected from N, O or S. Examples of suitable 5-membered monocyclic heteroaryl include, but are not limited to, pyrrolyl, furyl, thienyl, imidazolyl, furazanyl, oxazolyl, oxadiazolyl, oxatriazolyl, isoxazolyl, thiazolyl, isothiazolyl, pyrazolyl, triazolyl and tetrazolyl; examples of suitable 6-membered monocyclic heteroaryl include, but are not limited to, pyridyl, pyrazinyl, pyridazinyl, pyrimidinyl and triazinyl. The atom in the heteroaryl group that is bonded to the rest of the compound can be a carbon atom or a heteroatom, as long as it is chemically feasible.

A substituent described as "optionally substituted" means that the group may be unsubstituted or substituted by one or more (e.g., 0, 1, 2, 3, 4 or 5 or more, or any range derived therefrom) of the substituents listed for that group, wherein the substituents may be the same or different. In one embodiment, an optionally substituted group is substituted with 1 substituent. In another embodiment, an optionally substituted group is substituted with 2 substituents. In another embodiment, an optionally substituted group is substituted with 3 substituents. In another embodiment, an optionally substituted group is substituted with 4 substituents.

Those of ordinary skill in the art of organic synthesis understand that stable chemically feasible heterocycle, whether aromatic or nonaromatic, in which the maximum number of heteroatoms or the type of heteroatoms contained are determined by the size of the ring, the degree of unsaturation, and the valence of the heteroatoms. In general, a heterocycle can have from 1 to 4 heteroatoms, provided that the heterocycle or heteroaromatic ring is chemically feasible and stable.

The term "hydroxyl" as used herein refers to the -OH group.

The term "thiol" as used in this paper refers to the -SH group.

The term "nitro" as used herein refers to the $-NO_2$ group.

The term "optionally substituted" used herein means, unless otherwise indicated, that a group may be unsubstituted or substituted by one or more of the substituents listed for that group (e.g., 0, 1, 2, 3, 4 or 5 or more, or any range derived therefrom), in which the substituents may be the same or different. In one embodiment, an optionally substituted group has 1 substituent. In another embodiment, an optionally substituted group has 2 substituents. In another embodiment, an optionally substituted group has three substituents. In another embodiment, an optionally substituted group has four substituents. In another embodiment, an optionally substituted group has five substituents.

Unless otherwise specified, $C_{n-n+m}$ or $C_n$-$C_m$ in the compound definition of the present disclosure includes various cases of n to n+m carbons, such as $C_{1-6}$ including $C_1$, $C_2$, $C_3$, $C_4$, $C_5$, and $C_6$, and also includes any range from n to n+m, such as $C_{0-6}$ including $C_1$, $C_2$, $C_3$, $C_4$, $C_5$, $C_6$, $C_{0-1}$, $C_{0-2}$, $C_{0-3}$, $C_{0-4}$, $C_{0-5}$, $C_{1-2}$, $C_{1-3}$, $C_{1-4}$, $C_{2-3}$, etc., and $C_{1-6}$ including $C_{1-2}$, $C_{1-3}$, $C_{1-4}$, $C_{2-6}$, $C_{3-6}$, etc. Similarly, the n-membered to n+m-membered in the compound definition of the present disclosure represents the number of ring atoms from n to n+m, for example, a 3-12-membered ring includes a 3-membered ring, a 4-membered ring, a 5-membered ring, a 6-membered ring, and a 12-membered ring, etc., also includes any range from n to n+m members, for example, 3-12-membered rings include 3-6-membered ring, 3-8-membered ring, 3-9-membered ring, 4-7-membered ring, 4-5-membered ring, 5-6-membered ring, 5-7-membered ring, 5-8-membered ring, 5-9-membered ring, 6-7-membered ring, 6-8-membered ring and 6-10-membered ring.

As used in this specification and the following claims, the word "comprise" and variations of the word such as "include" and "contain", means "including but not limited to" and is not intended to exclude such as other additive, ingredient, integer, or step. When an element is described as comprising a plurality of ingredients, steps or conditions, it should be understood that the element may also be described as comprising any combination of the plurality of ingredients, steps or conditions, or as "consisting of a plurality or combination of ingredients, steps or conditions" or "consists essentially of a plurality or combination of ingredients, steps or conditions".

It should be understood that when describing herein the compounds of the present disclosure, pharmaceutical compositions comprising the compounds, pharmaceutical combinations, kits and related uses and methods, the dosages referred to are based on the weight of the free form, excluding any salts, hydrates or solvates, unless the specification states that the dosage is based on the weight of the salt, hydrate or solvate.

## Compounds of the disclosure

The terms "invented compound" and "compound of the disclosure", etc. used throughout this disclosure, unless otherwise stated, encompass the compounds of formula (I) or formula (II) defined in the various embodiments and preferred embodiments herein, including their isomers, including atropisomers, enantiomer mixtures, especially racemates, diastereomer mixtures, geometric isomers, tautomers, solvates, metabolites, isotopes variants, salts (e.g. pharmaceutically acceptable salts), and prodrugs.

Therefore, each of the above isomers and derivatives of the compounds of formula I are included in the scope of the present disclosure and their respective meanings, preparations and specific examples are defined in the "Definitions" section above, or are well-known to those skilled in the art. In some embodiments, metabolites, isotopic variants or prodrugs, and any combination thereof are excluded as appropriate. However, preference is given to substantially pure enantiomers (enantiomer pure) or diastereomers of the compounds of formula (I) or formula (II) and/or pharmaceutically acceptable salts thereof.

The present disclosure also covers N-oxides of the compounds of formula (I) or formula (II) provided these compounds contain a basic nitrogen atom, such as those present in nitrogen-containing heterocycles. Some compounds of the present disclosure may exist in polymorphic or amorphous form and therefore fall within the scope of the present disclosure.

In one aspect, the disclosure provides a compound of formula (I), an isomer or a pharmaceutically acceptable salt or a solvate thereof,

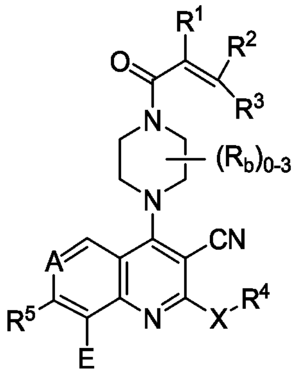

(I)

wherein,

A is selected from C-$R_a$ or N, wherein $R_a$ is selected from halogen, CN, nitro, $C_{3-6}$ cycloalkyl or $C_{1-6}$ alkyl optionally substituted by halogen;

$R^1$, $R^2$ and $R^3$ are each independently selected from H, halogen or $C_{1-6}$ alkyl, wherein the alkyl is optionally substituted by a group independently selected from halogen, -$N(R_c)_2$, -$OR_c$ or 3-8 membered heterocycloalkyl;

$R_b$ at each occurrence is independently selected from H, halogen, CN, or $C_{1-6}$ alkyl optionally substituted by halogen or CN;

X is selected from a bond, -O- or -NH-;

$R^4$ is selected from H, halogen, $C_{1-6}$ alkyl, -$C_{0-6}$ alkyl-$C_{6-10}$ aryl, -$C_{0-6}$ alkyl-$C_{3-8}$ cycloalkyl, -$C_{0-6}$ alkyl-$C_{3-8}$ cycloalkenyl, -$C_{0-6}$ alkyl-3-8 membered heterocycloalkyl, -$C_{0-6}$ alkyl-3-8 membered heterocycloalkenyl, -$C_{0-6}$ alkyl-5-10 membered heteroaryl, wherein the alkyl, aryl, cycloalkyl, cycloalkenyl, heterocycloalkyl, heterocycloalkenyl and heteroaryl groups are optionally substituted by one or more groups independently selected from halogen, $C_{1-6}$ alkyl optionally substituted by halogen, -$(CR_cR_c)_{0-6}$-$SR_c$, -$(CR_cR_c)_{0-6}$-$(CO)_{0-1}$-$OR_c$, -$(CR_cR_c)_{0-6}$-$(CO)_{0-1}$-$N(R_c)_2$, wherein the -$(CR_cR_c)_{0-6}$-$(CO)_{0-1}$-$N(R_c)_2$ attached to the aryl, cycloalkyl, cycloalkenyl, heterocycloalkyl, heterocycloalkenyl or heteroaryl, via the group connected to N and together with the ring atom to which it is attached and the adjacent atom, optionally forms a 4-7 membered nitrogen-containing heterocyclic ring;

$R_c$ at each occurrence is independently selected from H or $C_{1-6}$ alkyl optionally substituted by halogen, or wherein two $R_c$ attached to the same carbon or nitrogen atom, together with the carbon atom or nitrogen atom to which they are attached, independently form a 3-6 membered cyclic group;

E is selected from halogen, -O-$R_d$ or -$N(R_d)_2$-, wherein each $R_d$ is independently H or $C_{1-6}$ alkyl optionally substituted by halogen;

$R^5$ is

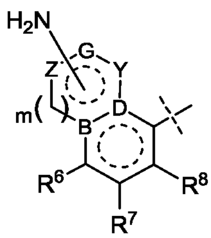

;

m is 0 or 1;

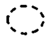 represents an aromatic ring;

B and D are each independently selected from N or C;

Z, G and Y are each independently selected from C, N, O or S;

$R^6$, $R^7$ and $R^8$ are each independently selected from H, halogen and $C_{1-6}$ alkyl optionally substituted by halogen;

with the proviso that B and D are not both N; and at most three of Z, G, Y, D and B are not C;

or an isomer, a pharmaceutically acceptable salt or a solvate thereof.

In one embodiment of the compounds of formula (I), A is N.

In one embodiment of the compounds of formula (I), A is C-$R_a$, wherein $R_a$ is selected from the halogen.

In one embodiment of the compounds of formula (I), A is C-$R_a$, wherein $R_a$ is F or Cl, preferably Cl.

In one embodiment of the compounds of formula (I), $R^1$ is selected from H or halogen.

In one embodiment of the compounds of formula (I), $R^1$ is H or F.

In one embodiment of the compounds of formula (I), both $R^2$ and $R^3$ are H.

In one embodiment of the compounds of formula (I), $R_b$ is H.

In one embodiment of the compounds of formula (I), $R_b$ is not H and either one $R_b$ or two $R_b$ are present.

In one embodiment of the compounds of formula (I), there is one $R_b$ and $R_b$ is $C_{1-6}$ alkyl.

In a specific embodiment, there is one $R_b$ and $R_b$ is $C_{1-6}$ alkyl, preferably $C_{1-3}$ alkyl; in a more specific embodiment, there is one $R_b$ and $R_b$ is $-CH_3$; in a more specific embodiment, there is one $R_b$ and $R_b$ is 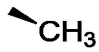 ; in a more specific embodiment, there is one $R_b$ and $R_b$ is 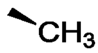 , and the manner of attaching to the piperazine ring is

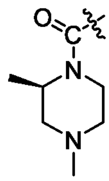 ;

in a more specific embodiment, there is one $R_b$ and $R_b$ is 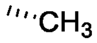 , and the manner of attaching to the piperazine ring is

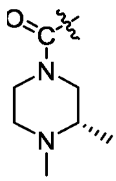 .

In one embodiment of the compounds of formula (I), there is one $R_b$ and $R_b$ is CN; in a specific embodiment, there is one $R_b$ and $R_b$ is CN and attached to the carbon atom ortho to the amide nitrogen.

In one embodiment of the compounds of formula (I), there is one $R_b$ and $R_b$ is $C_{1-6}$ alkyl, preferably $C_{1-3}$ alkyl, substituted by CN; in a specific embodiment, there is one $R_b$ and $R_b$ is cyanomethyl; in a more specific embodiment, there is one $R_b$ and $R_b$ is cyanomethyl, and the manner of attaching to the piperazine ring is

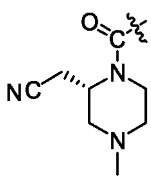 or 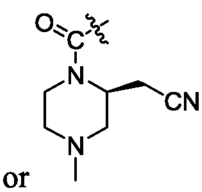 .

In one embodiment of the compounds of formula (I), there are two $R_b$ and $R_b$ is $C_{1-6}$ alkyl, preferably $C_{1-3}$ alkyl;

in a specific embodiment, there are two $R_b$ and $R_b$ is $-CH_3$; in a more specific embodiment, there are two $R_b$ and $R_b$ is $-CH_3$, and the manner of attaching to the piperazine ring is

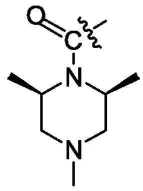,

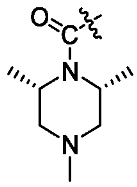, 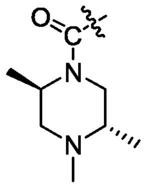 or 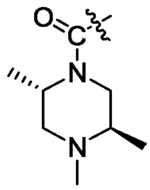.

In one embodiment of the compounds of formula (I), X is a bond.

In one embodiment of the compounds of formula (I), X is -O- or -NH-.

In one embodiment of the compounds of formula (I), X is -O-.

In one embodiment of the compounds of formula (I), X is -NH-.

In one embodiment of the compounds of formula (I), $R^4$ is selected from H, halogen, $C_{1-6}$ alkyl, $-C_{0-3}$alkyl-phenyl, $-C_{0-3}$alkyl-$C_{3-6}$cycloalkyl, $-C_{0-3}$alkyl-$C_{3-6}$cycloalkenyl, $-C_{0-3}$alkyl-4-6 membered heterocycloalkyl comprising 1, 2, or 3 heteroatoms independently selected from N, O or S, $-C_{0-3}$alkyl-5-6 membered heterocycloalkenyl comprising 1, 2, or 3 heteroatoms independently selected from N, O or S, and $-C_{0-3}$alkyl-5-6 membered heteroaryl comprising 1, 2, or 3 heteroatoms independently selected from N, O or S, wherein the alkyl, phenyl, cycloalkyl, cycloalkenyl, heterocycloalkyl, heterocycloalkenyl and heteroaryl are optionally substituted by 1, 2 or 3 groups independently selected from halogen, $C_{1-6}$ alkyl optionally substituted by halogen, $-(CR_cR_c)_{0-6}-SR_c$, $-(CR_cR_c)_{0-6}-OR_c$, $-(CR_cR_c)_{0-6}-N(R_c)_2$, wherein the $-(CR_cR_c)_{0-6}-N(R_c)_2$ attached to the phenyl, cycloalkyl, cycloalkenyl, heterocycloalkyl, heterocycloalkenyl or heteroaryl, via the group connected to N and together with the ring atom to which it is attached and the adjacent atom, optionally form a 4-7 membered nitrogen-containing heterocyclic ring.

In one embodiment of the compounds of formula (I), $R^4$ is selected from H, halogen, $C_{1-6}$ alkyl, $-C_{0-3}$alkyl-phenyl, $-C_{0-3}$alkyl-$C_{3-6}$cycloalkyl, $-C_{0-3}$alkyl-4-6 membered heterocycloalkyl comprising 1, 2 or 3 heteroatoms independently selected from N, O or S and $-C_{0-3}$alkyl-5-6 membered heteroaryl comprising 1, 2 or 3 heteroatoms independently selected from N, O or S, where the alkyl, phenyl, cycloalkyl, heterocycloalkyl, and heteroaryl are optionally substituted by 1, 2, or 3 groups independently selected from halogen, $C_{1-6}$ alkyl optionally substituted by halogen, $-(CR_cR_c)_{0-6}-SR_c$, $-(CR_cR_c)_{0-6}-OR_c$, $-(CR_cR_c)_{0-6}-N(R_c)_2$, wherein the $-(CR_cR_c)_{0-6}-N(R_c)_2$ attached to the phenyl, cycloalkyl, heterocycloalkyl or heteroaryl, via the group connected to N and together with the ring atom to which it is attached and the adjacent atom, optionally form a 4-7 membered nitrogen-containing heterocyclic ring.

In one embodiment of the compounds of formula (I), $R^4$ is selected from H, halogen, $C_{1-6}$ alkyl, phenyl, $C_{3-6}$cycloalkyl, 4-6 membered heterocycloalkyl comprising 1, 2 or 3 heteroatoms independently selected from N, O or S, and 5-6 membered heteroaryl comprising 1, 2 or 3 heteroatoms independently selected from N, O or S, where the alkyl, phenyl, cycloalkyl, heterocycloalkyl, and heteroaryl are optionally substituted by 1, 2 or 3 groups independently selected from halogen, $C_{1-6}$ alkyl optionally substituted by halogen, $-(CR_cR_c)_{0-6}-OR_c$, $-(CR_cR_c)_{0-6}-N(R_c)_2$, wherein the $-(CR_cR_c)_{0-6}-N(R_c)_2$ attached to the phenyl, cycloalkyl, heterocycloalkyl or heteroaryl, via the group connected to N and together with the ring atom to which it is attached and the adjacent atom, optionally form a 4-7 membered nitrogen-containing heterocyclic ring.

In one embodiment of the compounds of formula (I), $-X-R^4$ is H.

In one embodiment of the compounds of formula (I), $-X-R^4$ is not H.

In one embodiment of the compounds of formula (I), $-X-R^4$ is -OH.

In one embodiment of the compounds of formula (I), $-X-R^4$ is $-NH_2$.

In one embodiment of the compounds of formula (I), $-X-R^4$ is a halogen; in a specific embodiment, $- X-R^4$ is either Cl or F.

In one embodiment of the compounds of formula (I), $R^4$ is a $C_{1-6}$ alkyl, optionally substituted by 1, 2 or 3 groups independently selected from halogen, $C_{1-6}$ alkyl, hydroxyl, $C_{1-6}$ alkoxy, and $-(CR_cR_c)_{0-6}-N(R_c)_2$. In a specific embodiment, exemplary $R^4$ includes but is not limited to $-CH_3$, $-CH_2CH_3$, $- CH_2CH_2CH_3$, $-CH(CH_3)CH_3$, $-CH_2CH(CH_3)CH_3$,

$-CH_2CH_2CH_2CH_3$, $-C(CH_3)(CH_3)(CH_3)$, $-CH_2F$, $-CHF_2$, $-CF_3$, $-CH_2CF_3$, $-CH_2CH_2CF_3$, $-CH(CF_3)CH_3$, $-CH_2CH(CF_3)CH_3$, $-CH_2CH_2CH_2CF_3$, $-C(CF_3)(CH_3)(CH_3)$, $-CH_2OH$, $-CH_2CH_2OH$, $-CH_2CH_2CH_2OH$, $-CH_2CH(OH)CH_3$, $-CH_2CH(CH_2OH)CH_3$, $-CH_2CH_2CH_2CH_2OH$, $-C(CH_2OH)(CH_3)(CH_3)$, $-CH_2OCH_3$, $-CH_2OCH_2CH_3$, $-CH_2CH_2OCH_3$, $-CH_2CH_2OCH_2CH_3$, $-CH_2CH_2CH_2OCH_3$, $-CH_2CH(OCH_3)CH_3$, $-CH_2CH(CH_2OCH_3)CH_3$, $-CH_2CH_2CH_2CH_2OCH_3$, $-NH_2$, $-NHCH_3$, $-NHCH_2CH_3$, $-N(CH_3)_2$, $-N(CH_3)(CH_2CH_3)$, $-N(CH_2CH_3)_2$, $-CH_2NH_2$, $-CH_2NHCH_3$, $-CH_2N(CH_3)_2$, $-CH_2NHCH_2CH_3$, $-CH_2N(CH_3)(CH_2CH_3)$, $-CH_2N(CH_2CH_3)_2$, $-CH_2CH_2NH_2$, $-CH_2CH_2NHCH_3$, $-CH_2CH_2N(CH_3)_2$, $-CH(CH_3)N(CH_3)_2$, $CH_2CH_2NHCH_2CH_3$, $-CH_2CH_2N(CH_2CH_3)_2$, $-CH_2CH_2CH_2NH_2$, $-CH_2CH_2CH_2NHCH_3$, $-CH_2CH_2CH_2N(CH_3)_2$, $-CH_2CH(NH_2)CH_3$, $-CH_2CH(CH_2NH_2)CH_3$, $-CH_2CH_2CH_2CH_2NH_2$, $-CH_2CH_2CH_2CH_2NHCH_3$ and $-CH_2CH_2CH_2CH_2N(CH_3)_2$.

In a preferred embodiment, $R^4$ is $C_{1-6}$ alkyl, optionally substituted by 1, 2, or 3 groups independently selected from halogen, $C_{1-6}$ alkyl, and $C_{1-6}$ alkoxy; more preferably, $R^4$ is selected from $-CH_3$, $-CH_2CH_3$, $-CH_2CH_2CH_3$, $-CH(CH_3)CH_3$, $-CH_2OCH_3$, $-CH_2CH_2OCH_3$, $-CH_2CH_2OCH_2CH_3$ and $-CF_3$.

In one embodiment of the compounds of formula (I), $-X-R^4$ is $C_{1-6}$ alkyl, and the $C_{1-6}$ alkyl has the meaning as given for the embodiments, preferred or more preferred embodiments for the $C_{1-6}$ alkyl of $R^4$ as described above. In one embodiment of the compounds of formula (I), $-X-R^4$ is $-O-C_{1-6}$ alkyl, and the $C_{1-6}$ alkyl has the meaning as given for the embodiments, preferred or more preferred embodiments for the $C_{1-6}$ alkyl of $R^4$ as described above. In one embodiment of the compounds of formula (I), $-X-R^4$ is the $-NH-C_{1-6}$ alkyl, and the $C_{1-6}$ alkyl has the meaning as given for the embodiments, preferred or more preferred embodiments for the $C_{1-6}$ alkyl of $R^4$ as described above.

In one embodiment of the compounds of formula (I), $R^4$ is $-C_{0-3}$ alkyl-phenyl, preferably phenyl, which is optionally substituted by 1, 2 or 3 groups independently selected from halogens, $C_{1-6}$ alkyl optionally substituted by halogen, $-(CR_cR_c)_{0-6}-OR_c$ and $-(CR_cR_c)_{0-6}-N(R_c)_2$. In a specific embodiment, exemplary substituents include but are not limited to F, Cl, Br, I, $-CH_3$, $-CH_2CH_3$, $-CH_2CH_2CH_3$, $-CH(CH_3)CH_3$, $-CH_2CH(CH_3)CH_3$, $-CH_2CH_2CH_2CH_3$, $-C(CH_3)(CH_3)(CH_3)$, $-CH_2F$, $-CHF_2$, $-CF_3$, $-CH_2CF_3$, $-CH_2CH_2CF_3$, $-CH(CF_3)CH_3$, $-CH_2CH(CF_3)CH_3$, $-CH_2CH_2CH_2CF_3$, $-C(CF_3)(CH_3)(CH_3)$, $-CH_2OH$, $-CH_2CH_2OH$, $-CH_2CH_2CH_2OH$, $-CH_2CH(OH)CH_3$, $-CH_2CH(CH_2OH)CH_3$, $-CH_2CH_2CH_2CH_2OH$, $-C(CH_2OH)(CH_3)(CH_3)$, $-OH$, $-OCH_3$, $-OCH_2CH_3$, $-OCH_2CH_2CH_3$, $-OCH(CH_3)(CH_3)$, $-CH_2OCH_3$, $-CH_2OCH_2CH_3$, $-CH_2CH_2OCH_3$, $-CH_2CH_2OCH_2CH_3$, $-CH_2CH_2CH_2OCH_3$, $-CH_2CH(OCH_3)CH_3$, $-CH_2CH(CH_2OCH_3)CH_3$, $-CH_2CH_2CH_2CH_2OCH_3$, $-NH_2$, $-NHCH_3$, $-NHCH_2CH_3$, $-N(CH_3)_2$, $-N(CH_3)(CH_2CH_3)$, $-N(CH_2CH_3)_2$, $-CH_2NH_2$, $-CH_2NHCH_3$, $-CH_2N(CH_3)_2$, $-CH_2NHCH_2CH_3$, $-CH_2N(CH_3)(CH_2CH_3)$, $-CH_2N(CH_2CH_3)_2$, $-CH_2CH_2NH_2$, $-CH_2CH_2NHCH_3$, $-CH_2CH_2N(CH_3)_2$, $-CH(CH_3)N(CH_3)_2$, $CH_2CH_2NHCH_2CH_3$, $-CH_2CH_2N(CH_2CH_3)_2$, $-CH_2CH_2CH_2NH_2$, $-CH_2CH_2CH_2NHCH_3$, $-CH_2CH_2CH_2N(CH_3)_2$, $-CH_2CH(NH_2)CH_3$, $-CH_2CH(CH_2NH_2)CH_3$, $-CH_2CH_2CH_2CH_2NH_2$, $-CH_2CH_2CH_2CH_2NHCH_3$ and $-CH_2CH_2CH_2CH_2N(CH_3)_2$.

In a preferred embodiment, $R^4$ is phenyl substituted by 1, 2 or 3 groups independently selected from halogen, $C_{1-6}$ alkyl optionally substituted by halogen, and $-(CR_cR_c)_{0-6}-N(R_c)_2$. Exemplary substituents include but are not limited to F, Cl, Br, I, $-CH_3$, $-CH_2CH_3$, $-CH_2CH_2CH_3$, $-CH(CH_3)CH_3$, $-CH_2CH(CH_3)CH_3$, $-CH_2CH_2CH_2CH_3$, $-C(CH_3)(CH_3)(CH_3)$, $-CH_2F$, $-CHF_2$, $-CF_3$, $-CH_2CF_3$, $-CH_2CH_2CF_3$, $-CH(CF_3)CH_3$, $-CH_2CH(CF_3)CH_3$, $-CH_2CH_2CH_2CF_3$, $-C(CF_3)(CH_3)(CH_3)$, $-NH_2$, $-NHCH_3$, $-NHCH_2CH_3$, $-N(CH_3)_2$, $-N(CH_3)(CH_2CH_3)$, $-N(CH_2CH_3)_2$, $-CH_2NH_2$, $-CH_2NHCH_3$, $-CH_2N(CH_3)_2$, $-CH_2NHCH_2CH_3$, $-CH_2N(CH_3)(CH_2CH_3)$, $-CH_2N(CH_2CH_3)_2$, $-CH_2CH_2NH_2$, $-CH_2CH_2NHCH_3$, $-CH_2CH_2N(CH_3)_2$, $-CH(CH_3)N(CH_3)_2$, $CH_2CH_2NHCH_2CH_3$, $-CH_2CH_2N(CH_2CH_3)_2$, $-CH_2CH_2CH_2NH_2$, $-CH_2CH_2CH_2NHCH_3$, $-CH_2CH_2CH_2N(CH_3)_2$, $-CH_2CH(NH_2)CH_3$, $-CH_2CH(CH_2NH_2)CH_3$, $-CH_2CH_2CH_2CH_2NH_2$, $-CH_2CH_2CH_2CH_2NHCH_3$ and $-CH_2CH_2CH_2CH_2N(CH_3)_2$; more preferably, $R^4$ is selected from

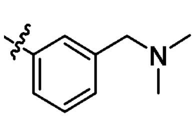 , 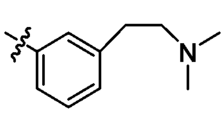 ,

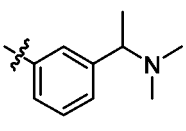 .

In one embodiment of the compounds of formula (I), $R^4$ is $-C_{0-3}$ alkyl-phenyl, preferably phenyl, substituted by $-(CR_cR_c)_{0-6}-N(R_c)_2$, wherein each $R_c$ is independently H or $C_{1-6}$ alkyl, and one of the $R_c$ on N together with the atom on the benzene ring to which it is attached and the adjacent atom form a 4-7 membered nitrogen-containing heterocycle. In a specific embodiment, $R^4$ is selected from

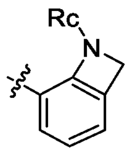, 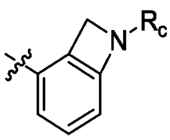, 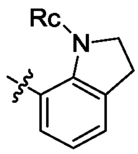, 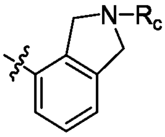, 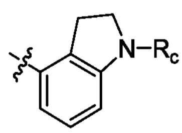, 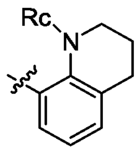, 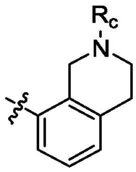,

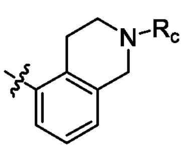, 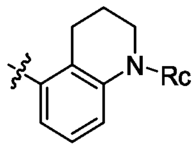, 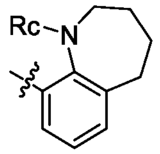, 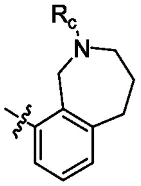, 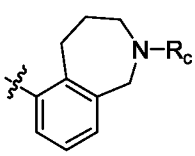, 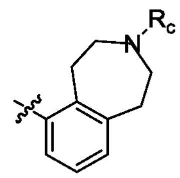,

wherein each $R_c$ is independently selected from H, $-CH_3$, $-CH_2CH_3$, $-CH_2CH_2CH_3$, $-CH(CH_3)CH_3$, - $CH_2CH(CH_3)CH_3$, $-CH_2CH_2CH_2CH_3$ and $-C(CH_3)(CH_3)(CH_3)$.

In a preferred embodiment, $R^4$ is

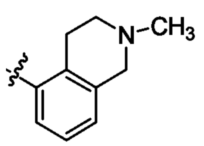
.

In one embodiment of the compounds of formula (I), $-X-R^4$ is $-C_{0-6}$ alkyl-phenyl, preferably $-C_{0-3}$ alkyl-phenyl, and preferably phenyl, wherein the phenyl has the meaning as given for the embodiments, preferred or more preferred embodiments for the phenyl of $R^4$ as described above. In one embodiment of the compounds of formula (I), $-X-R^4$ is $-O-C_{0-6}$ alkyl-phenyl, preferably $-O-C_{0-3}$ alkyl-phenyl, and more preferably -O-phenyl, wherein the phenyl has the meaning as given for the embodiments, preferred or more preferred embodiments for the phenyl of $R^4$ as described above. In one embodiment of the compounds of formula (I), $-X-R^4$ is $-NH-C_{0-6}$ alkyl-phenyl, preferably $C_{0-3}$ alkyl-phenyl, and more preferably -NH-phenyl, wherein the phenyl has the meaning as given for the embodiments, preferred or more preferred embodiments for the phenyl of $R^4$ as described above.

In one embodiment of the compounds of formula (I), $R^4$ is a $-C_{0-3}$ alkyl-$C_{3-6}$ cycloalkyl, preferably a $-C_{3-6}$ cycloalkyl, optionally substituted by 1, 2 or 3 groups independently selected from halogen, $C_{1-6}$alkyl optionally substituted by halogen, $-(CR_cR_c)_{0-6}-OR_c$ and $-(CR_cR_c)_{0-6}-N(R_c)_2$. In specific embodiments, Exemplary substituents include but are not limited to: F, Cl, Br, I, $-CH_3$, $-CH_2CH_3$, - $CH_2CH_2CH_3$, $-CH(CH_3)CH_3$, $-CH_2CH(CH_3)CH_3$, $-CH_2CH_2CH_2CH_3$, $-C(CH_3)(CH_3)(CH_3)$, $-CH_2F$, - $CHF_2$, $-CF_3$, $-CH_2CF_3$, $-CH_2CH_2CF_3$, $-CH(CF_3)CH_3$, $-CH_2CH(CF_3)CH_3$, $-CH_2CH_2CH_2CF_3$, - $C(CF_3)(CH_3)(CH_3)$, $-CH_2OH$, $-CH_2CH_2OH$, $-CH_2CH_2CH_2OH$, $-CH_2CH(OH)CH_3$, - $CH_2CH(CH_2OH)CH_3$, $-CH_2CH_2CH_2CH_2OH$, $-C(CH_2OH)(CH_3)(CH_3)$, -OH, $-OCH_3$, $-OCH_2CH_3$, - $OCH_2CH_2CH_3$, $-OCH(CH_3)(CH_3)$, $-CH_2OCH_3$, $-CH_2OCH_2CH_3$, $-CH_2CH_2OCH_3$, - $CH_2CH_2OCH_2CH_3$, $-CH_2CH_2CH_2OCH_3$, $-CH_2CH(OCH_3)CH_3$, $-CH_2CH(CH_2OCH_3)CH_3$, - $CH_2CH_2CH_2CH_2OCH_3$, $-NH_2$, $-NHCH_3$, $-NHCH_2CH_3$, $-N(CH_3)_2$, $-N(CH_3)(CH_2CH_3)$, $-N(CH_2CH_3)_2$, $-CH_2NH_2$, $-CH_2NHCH_3$, $-CH_2N(CH_3)_2$, $-CH_2NHCH_2CH_3$, $-CH_2N(CH_3)(CH_2CH_3)$, - $CH_2N(CH_2CH_3)_2$, $-CH_2CH_2NH_2$, $-CH_2CH_2NHCH_3$, $-CH_2CH_2N(CH_3)_2$, $-CH(CH_3)N(CH_3)_2$, $CH_2CH_2NHCH_2CH_3$, $-CH_2CH_2N(CH_2CH_3)_2$, $-CH_2CH_2CH_2NH_2$, $-CH_2CH_2CH_2NHCH_3$, - $CH_2CH_2CH_2N(CH_3)_2$, $-CH_2CH(NH_2)CH_3$, $-CH_2CH(CH_2NH_2)CH_3$, $-CH_2CH_2CH_2CH_2NH_2$, - $CH_2CH_2CH_2CH_2NHCH_3$ and $-CH_2CH_2CH_2CH_2N(CH_3)_2$.

In a preferred embodiment, $R^4$ is selected from cyclopropyl, cyclobutyl, cyclopentyl and cyclohexyl; more preferably cyclopropyl.

In one embodiment of the compounds of formula (I), $-X-R^4$ is $-C_{0-6}$ alkyl-$C_{3-6}$ cycloalkyl, preferably $-C_{0-3}$alkyl-$C_{3-6}$cycloalkyl, more preferably $C_{3-6}$cycloalkyl, wherein the cycloalkyl has the meaning as given for the embodiments, preferred or more preferred embodiments for the cycloalkyl of $R^4$ as described above. In one embodiment of the compounds of formula (I), $-X-R^4$ is $-O-C_{0-6}$alkyl-$C_{3-6}$ cycloalkyl, preferably $-O-C_{0-3}$alkyl-$C_{3-6}$cycloalkyl, and more preferably $-O-C_{3-6}$cycloalkyl, wherein the cycloalkyl has the meaning as given for the embodiments, preferred or more preferred embodiments for the cycloalkyl of $R^4$ as described above. In one embodiment of the compounds of formula (I), $-X-R^4$ is $-NH-C_{0-6}$alkyl-$C_{3-6}$cycloalkyl, preferably $-NH-C_{0-3}$alkyl-$C_{3-6}$cycloalkyl, and more preferably $-NH-C_{3-6}$cycloalkyl, wherein the cycloalkyl has the meaning as given for the embodiments, preferred or more preferred embodiments for the cycloalkyl of $R^4$ as described above. In one embodiment of the compounds of formula (I), $R^4$ is $-C_{0-3}$alkyl-4-6 membered hetero-

cycloalkyl comprising 1, 2 or 3 heteroatoms independently selected from N, O or S, preferably a 4-6 membered heterocycloalkyl comprising 1, 2 or 3 heteroatoms independently selected from N, O or S, wherein the heterocycloalkyl is optionally substituted by 1, 2 or 3 groups independently selected from halogen, $C_{1-6}$ alkyl optionally substituted by halogen, $-(CR_cR_c)_{0-6}-OR_c$ and $-(CR_cR_c)_{0-6}-N(R_c)_2$. In specific embodiments, exemplary substituents include but are not limited to: F, Cl, Br, I, $-CH_3$, $-CH_2CH_3$, $-CH_2CH_2CH_3$, $-CH(CH_3)CH_3$, $-CH_2CH(CH_3)CH_3$, $-CH_2CH_2CH_2CH_3$, $-C(CH_3)(CH_3)(CH_3)$, $-CH_2F$, $-CHF_2$, $-CF_3$, $-CH_2CF_3$, $-CH_2CH_2CF_3$, $-CH(CF_3)CH_3$, $-CH_2CH(CF_3)CH_3$, $-CH_2CH_2CH_2CF_3$, $-C(CF_3)(CH_3)(CH_3)$, $-CH_2OH$, $-CH_2CH_2OH$, $-CH_2CH_2CH_2OH$, $-CH_2CH(OH)CH_3$, $-CH_2CH(CH_2OH)CH_3$, $-CH_2CH_2CH_2CH_2OH$, $-C(CH_2OH)(CH_3)(CH_3)$, $-OH$, $-OCH_3$, $-OCH_2CH_3$, $-OCH_2CH_2CH_3$, $-OCH(CH_3)(CH_3)$, $-CH_2OCH_3$, $-CH_2OCH_2CH_3$, $-CH_2CH_2OCH_3$, $-CH_2CH_2OCH_2CH_3$, $-CH_2CH_2CH_2OCH_3$, $-CH_2CH(OCH_3)CH_3$, $-CH_2CH(CH_2OCH_3)CH_3$, $-CH_2CH_2CH_2CH_2OCH_3$, $-NH_2$, $-NHCH_3$, $-NHCH_2CH_3$, $-N(CH_3)_2$, $-N(CH_3)(CH_2CH_3)$, $-N(CH_2CH_3)_2$, $-CH_2NH_2$, $-CH_2NHCH_3$, $-CH_2N(CH_3)_2$, $-CH_2NHCH_2CH_3$, $-CH_2N(CH_3)(CH_2CH_3)$, $-CH_2N(CH_2CH_3)_2$, $-CH_2CH_2NH_2$, $-CH_2CH_2NHCH_3$, $-CH_2CH_2N(CH_3)_2$, $-CH(CH_3)N(CH_3)_2$, $CH_2CH_2NHCH_2CH_3$, $-CH_2CH_2N(CH_2CH_3)_2$, $-CH_2CH_2CH_2NH_2$, $-CH_2CH_2CH_2NHCH_3$, $-CH_2CH_2CH_2N(CH_3)_2$, $-CH_2CH(NH_2)CH_3$, $-CH_2CH(CH_2NH_2)CH_3$, $-CH_2CH_2CH_2CH_2NH_2$, $-CH_2CH_2CH_2CH_2NHCH_3$ and $-CH_2CH_2CH_2CH_2N(CH_3)_2$.

In one embodiment of the compounds of formula (I), the 4-6 membered heterocycloalkyl in $R^4$ is selected from azetidinyl, oxetanyl, thietanyl, pyrrolidinyl (e.g., 1-pyrrolidinyl, 2-pyrrolidinyl, and 3-pyrrolidinyl), tetrahydrofuryl (e.g., 1-tetrahydrofuryl, 2-tetrahydrofuryl, and 3-tetrahydrofuryl), tetrahydrothiophenyl (e.g., 1-tetrahydrothiophenyl, 2-tetrahydrothiophenyl, and 3-tetrahydrothiophenyl), piperidinyl (e.g., 1-piperidinyl, 2-piperidinyl, 3-piperidinyl, and 4-piperidinyl), tetrahydropyranyl (e.g., 4-tetrahydropyranyl), tetrahydrothiopyranyl (e.g., 4-tetrahydrothiopyranyl), morpholinyl (e.g., morpholino), thiomorpholyl, dioxanyl, or piperazinyl.

In a preferred embodiment, $R^4$ is selected from

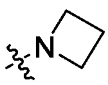 , 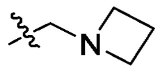 , 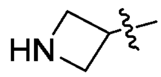 , 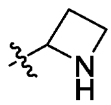 ,

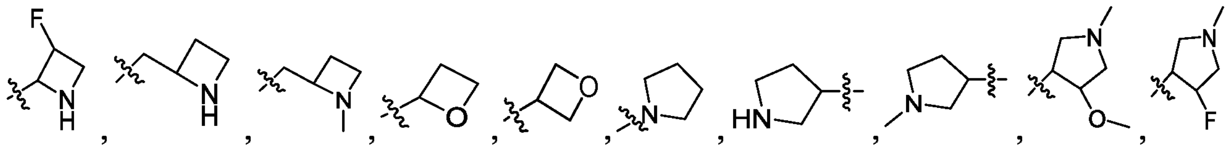

, , , , , , , , , ,

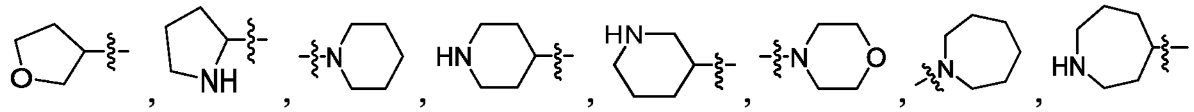

, , , , , , , ;

most preferably, $R^4$ is selected from

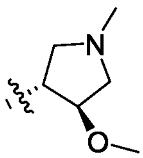 , 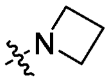 , 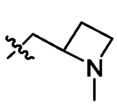 , 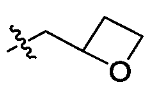 and 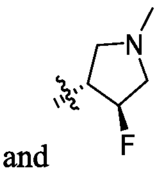 .

In one embodiment of the compounds of formula (I), $-X-R^4$ is $-C_{0-6}$alkyl-4-6 membered heterocycloalkyl comprising 1, 2 or 3 heteroatoms independently selected from N, O or S, preferably $-C_{0-3}$alkyl-4-6 membered heterocycloalkyl comprising 1, 2 or 3 heteroatoms independently selected from N, O or S, more preferably 4-6 membered heterocycloalkyl comprising 1, 2 or 3 heteroatoms independently selected from N, O or S, wherein the heterocycloalkyl has the meaning as given for the embodiments, preferred or more preferred embodiments for the heterocycloalkyl of $R^4$ as described above. In one embodiment of the compounds of formula (I), $-X-R^4$ is $-O-C_{0-6}$alkyl-4-6 membered heterocycloalkyl comprising 1, 2 or 3 heteroatoms independently selected from N, O or S, preferably $-O-C_{0-3}$alkyl-5-6 membered heterocycloalkyl comprising 1, 2 or 3 heteroatoms independently selected from N, O or S, more preferably a 4-6 membered heterocycloalkyl comprising 1, 2 or 3 heteroatoms independently selected from N, O or S, wherein the heterocycloalkyl has the meaning as given for the embodiments, preferred or more preferred embodiments for the heterocycloalkyl of $R^4$ as described above. In one embodiment of the compounds of formula (I), $-X-R^4$ is $-NH-C_{0-6}$alkyl- 4-6 membered

heterocycloalkyl comprising 1, 2 or 3 heteroatoms independently selected from N, O or S, preferably -NH-$C_{0-3}$alkyl-5-6 membered heterocycloalkyl comprising 1, 2 or 3 heteroatoms independently selected from N, O or S, more preferably a 4-6 membered heterocycloalkyl comprising 1, 2 or 3 heteroatoms independently selected from N, O or S, wherein the heterocycloalkyl has the meaning as given for the embodiments, preferred or more preferred embodiments for the heterocycloalkyl of $R^4$ as described above.

In one embodiment of the compounds of formula (I), $R^4$ is -$C_{0-3}$alkyl-5-6 heteroaryl comprising 1, 2 or 3 heteroatoms independently selected from N, O or S, preferably 5-6 heteroaryl comprising 1, 2 or 3 heteroatoms independently selected from N, O or S, wherein the heteroaryl is optionally substituted by 1, 2 or 3 groups independently selected from halogen, $C_{1-6}$ alkyl optionally substituted by halogen, $-(CR_cR_c)_{0-6}-OR_c$ and $-(CR_cR_c)_{0-6}-N(R_c)_2$. In specific embodiments, exemplary substituents include but are not limited to F, Cl, Br, I, $-CH_3$, $-CH_2CH_3$, $-CH_2CH_2CH_3$, $-CH(CH_3)CH_3$, $-CH_2CH(CH_3)CH_3$, $-CH_2CH_2CH_2CH_3$, $-C(CH_3)(CH_3)(CH_3)$, $-CH_2F$, $-CHF_2$, $-CF_3$, $-CH_2CF_3$, $-CH_2CH_2CF_3$, $-CH(CF_3)CH_3$, $-CH_2CH(CF_3)CH_3$, $-CH_2CH_2CH_2CF_3$, $-C(CF_3)(CH_3)(CH_3)$, $-CH_2OH$, $-CH_2CH_2OH$, $-CH_2CH_2CH_2OH$, $-CH_2CH(OH)CH_3$, $-CH_2CH(CH_2OH)CH_3$, $-CH_2CH_2CH_2CH_2OH$, $-C(CH_2OH)(CH_3)(CH_3)$, $-OH$, $-OCH_3$, $-OCH_2CH_3$, $-OCH_2CH_2CH_3$, $-OCH(CH_3)(CH_3)$, $-CH_2OCH_3$, $-CH_2OCH_2CH_3$, $-CH_2CH_2OCH_3$, $-CH_2CH_2OCH_2CH_3$, $-CH_2CH_2CH_2OCH_3$, $-CH_2CH(OCH_3)CH_3$, $-CH_2CH(CH_2OCH_3)CH_3$, $-CH_2CH_2CH_2CH_2OCH_3$, $-NH_2$, $-NHCH_3$, $-NHCH_2CH_3$, $-N(CH_3)_2$, $-N(CH_3)(CH_2CH_3)$, $-N(CH_2CH_3)_2$, $-CH_2NH_2$, $-CH_2NHCH_3$, $-CH_2N(CH_3)_2$, $-CH_2NHCH_2CH_3$, $-CH_2N(CH_3)(CH_2CH_3)$, $-CH_2N(CH_2CH_3)_2$, $-CH_2CH_2NH_2$, $-CH_2CH_2NHCH_3$, $-CH_2CH_2N(CH_3)_2$, $-CH(CH_3)N(CH_3)_2$, $CH_2CH_2NHCH_2CH_3$, $-CH_2CH_2N(CH_2CH_3)_2$, $-CH_2CH_2CH_2NH_2$, $-CH_2CH_2CH_2NHCH_3$, $-CH_2CH_2CH_2N(CH_3)_2$, $-CH_2CH(NH_2)CH_3$, $-CH_2CH(CH_2NH_2)CH_3$, $-CH_2CH_2CH_2CH_2NH_2$, $-CH_2CH_2CH_2CH_2NHCH_3$ and $-CH_2CH_2CH_2CH_2N(CH_3)_2$.

In one embodiment of the compounds of formula (I), the 5-6 membered heteroaryl in $R^4$ is selected from pyrrolyl, furyl, thiophenyl, imidazolyl, furazanyl, oxazolyl, oxadiazolyl, oxotriazolyl, isoxazolyl, thiazolyl, isothiazolyl, pyrazolyl, triazolyl and tetrazolyl, pyridyl, pyrazinyl, pyridazinyl, pyrimidinyl, and triazinyl.

In a preferred embodiment, $R^4$ is selected from,

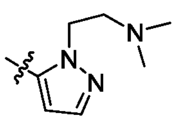, 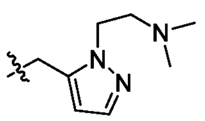, 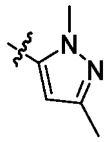,

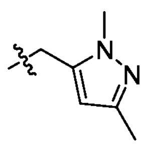, 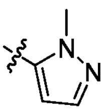, 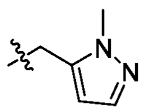, 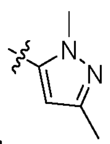, 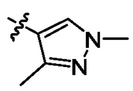, 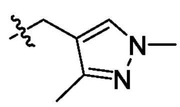, 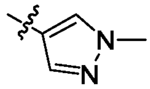, 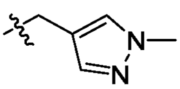.

Most preferably, $R^4$ is selected from

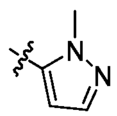, 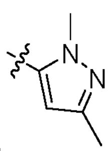 and 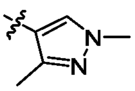.

In one embodiment of the compounds of formula (I), -X-$R^4$ is -$C_{0-6}$alkyl-5-6 membered heteroaryl comprising 1, 2 or 3 heteroatoms independently selected from N, O or S, preferably -$C_{0-3}$alkyl-5-6 membered heteroaryl comprising 1, 2 or 3 heteroatoms independently selected from N, O or S, more preferably 5-6 membered heteroaryl comprising 1, 2 or 3 heteroatoms independently selected from N, O or S, wherein the heteroaryl has the meaning as given for the embodiments, preferred or more preferred embodiments for the heteroaryl of $R^4$ as described above. In one embodiment of the compounds of formula (I), -X-$R^4$ is -O-$C_{0-6}$alkyl-5-6 membered heteroaryl comprising 1, 2 or 3 heteroatoms independently selected from N, O or S, preferably -O-$C_{0-3}$alkyl-5-6 membered heteroaryl comprising 1, 2 or 3 heteroatoms independently selected from N, O or S, and more preferably 5-6 membered heteroaryl comprising 1, 2 or 3 heteroatoms independently selected from N, O or S, wherein the heteroaryl has the meaning as given for the embodiments, preferred or more preferred embodiments for the heteroaryl of $R^4$ as described above. In one embodiment of the compounds of formula (I), -X-$R^4$ is -NH-$C_{0-6}$alkyl-5-6 heteroaryl comprising 1, 2 or 3 heteroatoms independently selected from N, O or S, preferably -NH-$C_{0-3}$alkyl-5-6 heteroaryl comprising 1, 2 or 3 heteroatoms independently selected from N, O or S, and more preferably 5-6 heteroaryl comprising 1, 2 or 3 heteroatoms independently selected from N, O or S, wherein the

heteroaryl has the meaning as given for the embodiments, preferred or more preferred embodiments for the heteroaryl of $R^4$ as described above.

In one embodiment of the compounds of formula (I), the $-(CR_cR_c)_{0\text{-}6}\text{-}SR_c$, $-(CR_cR_c)_{0\text{-}6}\text{-}(CO)_{0\text{-}1}\text{-}OR_c$, $-(CR_cR_c)_{0\text{-}6}\text{-}(CO)_{0\text{-}1}\text{-}N(R_c)_2$ as the substituent on $R^4$ are each preferably $-(CR_cR_c)_{0\text{-}6}\text{-}SR_c$, $-(CR_cR_c)_{0\text{-}6}\text{-}OR_c$, $-(CR_cR_c)_{0\text{-}6}\text{-}N(R_c)_2$; and more preferably $-(CR_cR_c)_{0\text{-}3}\text{-}SR_c$, $-(CR_cR_c)_{0\text{-}3}\text{-}OR_c$, $-(CR_cR_c)_{0\text{-}3}\text{-}N(R_c)_2$.

In one embodiment of the compounds of formula (I), E is halogen and preferably F.

In one embodiment of the compounds of formula (I), E is $-O\text{-}R_d$, wherein $R_d$ is $C_{1\text{-}6}$alkyl optionally substituted by halogen, preferably $C_{1\text{-}6}$alkyl, such as but not limited to methyl, ethyl, propyl, isopropyl, trifluoromethyl, difluoromethyl, trifluoroethyl, etc.

In one embodiment of the compounds of formula (I), $R^5$ is

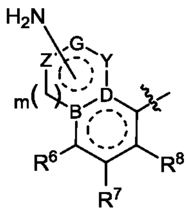

,

wherein, at most two of Z, G, Y, B, D are not C. Exemplary $R^5$ includes but is not limited to

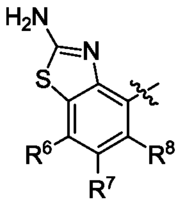

,

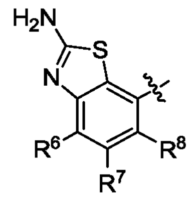

,

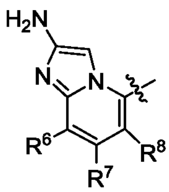

,

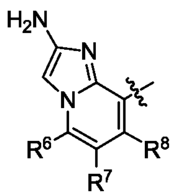

,

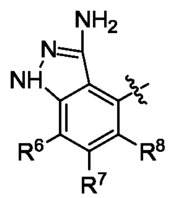

,

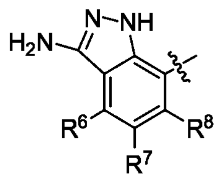

,

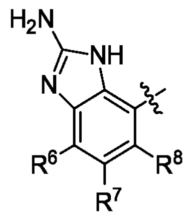

,

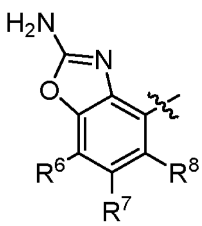

,

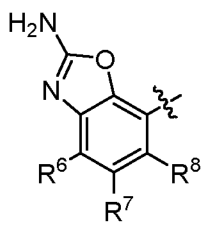

,

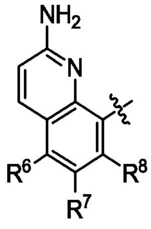

,

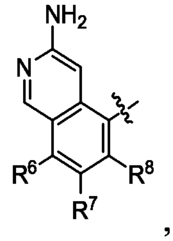

,

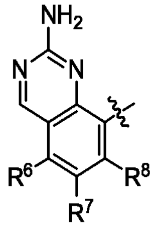

.

In a preferred embodiment, $R^5$ is

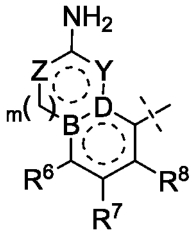

,

wherein at most two of Z, Y, B, D are not C.

Exemplary $R^5$ includes but is not limited to

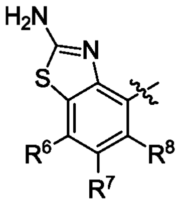 , 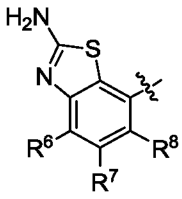 , 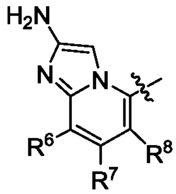 ,

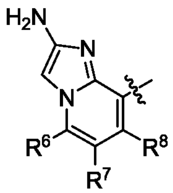 , 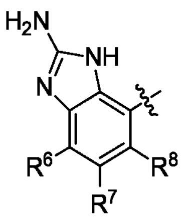 , 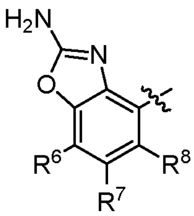 , 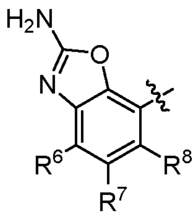 , 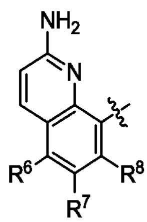 , 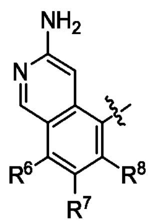 ,

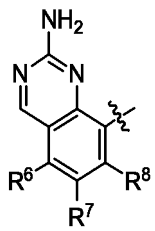 .

In a more preferred embodiment, $R^5$ is

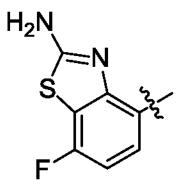 , 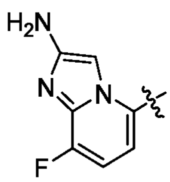 , 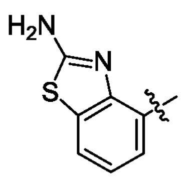 ,

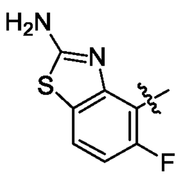 , 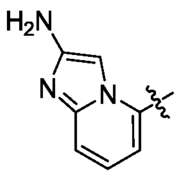 , 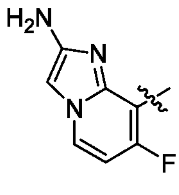 , 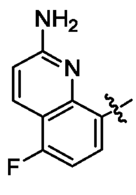 , 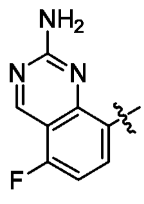 ,

most preferably $R^5$ is

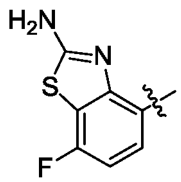

.

In a preferred embodiment, $R^5$ is

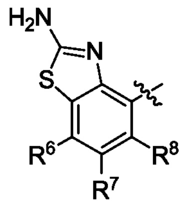

,

more preferably

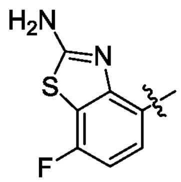

.

In one embodiment of the compounds of formula (I), at least one of $R^6$, $R^7$ and $R^8$ is halogen, preferably F. In a more preferred embodiment, $R^6$ is F and $R^7$ and $R^8$ are H.

In one embodiment of the compounds of formula (I), at least one of $R^6$, $R^7$ and $R^8$ is halogen-substituted $C_{1-6}$ alkyl, preferably $-CF_3$.

In one embodiment of the compounds of formula (I), $R_c$ is H or $C_{1-6}$ alkyl.

The present disclosure also provides a compound of formula (II), an isomer, a pharmaceutically acceptable salt or a solvate thereof,

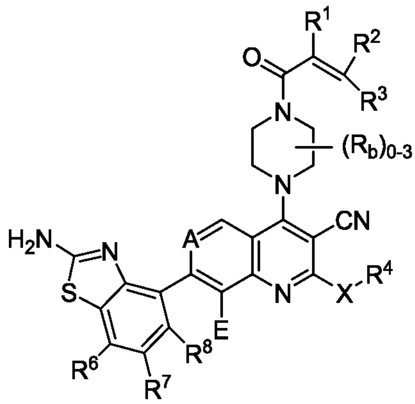

(II)

wherein $R^1$, $R^2$, $R^3$, $R^4$, $R^6$, $R^7$, $R^8$, A, E, $R_b$ each is as defined above for the compound of formula (I).

In a preferred embodiment of the compounds of formula (II),

A is selected from C-$R_a$ or N, wherein $R_a$ is selected from halogen;

$R^1$, $R^2$ and $R^3$ are each independently selected from H, halogen or $C_{1-6}$ alkyl;

$R_b$, at each occurrence is independently selected from H, halogen, CN or $C_{1-6}$ alkyl optionally substituted by halogen or CN;

X is selected from bond, -O- or -NH-;

$R^4$ is selected from H, halogen, $C_{1-6}$ alkyl, -$C_{0-3}$ alkyl-$C_{6-10}$ aryl, -$C_{0-3}$ alkyl-$C_{3-8}$ cycloalkyl, -$C_{0-3}$ alkyl-$C_{3-8}$ cycloalkenyl, -$C_{0-3}$ alkyl-3-8 heterocycloalkyl comprising 1, 2 or 3 heteroatoms independently selected from N, O or S, -$C_{0-3}$ alkyl-3-8 heterocycloalkenyl comprising 1, 2, or 3 heteroatoms independently selected from N, O or S, -$C_{0-3}$ alkyl-5-10 heteroaryl comprising 1, 2, or 3 heteroatoms independently selected from N, O or S, wherein the alkyl, aryl, cycloalkyl, cycloalkenyl, heterocycloalkyl, heterocycloalkenyl and heteroaryl are optionally substituted by 1, 2 or 3 groups independently selected from: halogen, $C_{1-6}$ alkyl optionally substituted by halogen, $-(CR_cR_c)_{0-6}-OR_c$, $-(CR_cR_c)_{0-6}-N(R_c)_2$, wherein the $-(CR_cR_c)_{0-6}-N(R_c)_2$ attached to the aryl, cycloalkyl, cycloalkenyl, heterocycloalkyl, heterocycloalkenyl or heteroaryl, via the group connected to N and together with the ring atom to which it is attached and the adjacent atom, optionally form a 4-7 membered nitrogen-containing heterocycle;

$R_c$ is at each occurrence independently selected from H or $C_{1-6}$ alkyl optionally substituted by halogen;

E is selected from halogen, -O-$R_d$ or -N$(R_d)_2$-, wherein $R_d$ is independently selected from H or $C_{1-6}$ alkyl optionally substituted by halogen;

$R^6$, $R^7$ and $R^8$ are each independently selected from H, halogen and $C_{1-6}$ alkyl optionally substituted by halogen;

or an isomer, a pharmaceutically acceptable salt or a solvate thereof.

In a preferred embodiment of the compounds of formula (II), A is C-$R_a$, wherein $R_a$ is selected from F or Cl, and preferably Cl.

In a preferred embodiment of the compounds of formula (II), $R_b$ is H.

In a preferred embodiment of the compounds of formula (II), $R_b$ is not H and either one $R_b$ or two $R_b$ are present.

In a preferred embodiment of the compounds of formula (II), -X-$R^4$ is H.

In a preferred embodiment of the compounds of formula (II), -X-$R^4$ is not H.

In a preferred embodiment of the compounds of formula (II), at least one of $R^6$, $R^7$ and $R^8$ is halogen, preferably F; more preferably $R^6$ is F, and $R^7$ and $R^8$ are H.

In a preferred embodiment of the compounds of formula (II), E is halogen and preferably F.

In a preferred embodiment of the compounds of formula (II), E is -O-$R_d$, wherein $R_d$ is $C_{1-6}$ alkyl optionally substituted by halogen, preferably $C_{1-6}$ alkyl, e.g., but not limited to methyl, ethyl, propyl, isopropyl, trifluoromethyl, difluoromethyl, trifluoroethyl, etc.

In a preferred embodiment of the compounds of formula (II), $R^4$ is selected from H, halogen, $C_{1-6}$ alkyl, phenyl, $C_{3-6}$cycloalkyl, 4-6 membered heterocycloalkyl comprising 1, 2 or 3 heteroatoms independently selected from N, O or S, and 5-6 membered heteroaryl comprising 1, 2 or 3 heteroatoms independently selected from N, O or S, wherein the alkyl, aryl, cycloalkyl, heterocycloalkyl, and heteroaryl are optionally substituted by 1, 2, or 3 groups independently selected from: halogen, $C_{1-6}$ alkyl optionally substituted by halogen, $-(CR_cR_c)_{0-6}-OR_c$, and $-(CR_cR_c)_{0-6}-N(R_c)_2$, wherein the $-(CR_cR_c)_{0-6}-N(R_c)_2$ attached to the aryl, cycloalkyl, cycloalkenyl, heterocycloalkyl, heterocycloalkenyl or heteroaryl, via the group connected to N and together with the ring atom to which it is attached and the adjacent atom, optionally form a 4-7 membered nitrogen-containing heterocycle.

In a preferred embodiment of the compounds of formula (II), $R^4$ is selected from halogen, preferably F or Cl.

In a preferred embodiment of the compounds of formula (II), $R^4$ is selected from $C_{1-6}$ alkyl, e.g., methyl, ethyl, propyl, isopropyl, etc., optionally substituted by 1, 2 or 3 groups independently selected from: halogen, $C_{1-6}$ alkyl optionally substituted by halogen, $-(CR_cR_c)_{0-6}-OR_c$, and $-(CR_cR_c)_{0-6}-N(R_c)_2$.

In a preferred embodiment of the compounds of formula (II), $R^4$ is selected from phenyl, optionally substituted by halogen, $C_{1-6}$ alkyl optionally substituted by halogen, $-(CR_cR_c)_{0-6}-OR_c$, and $-(CR_cR_c)_{0-6}-N(R_c)_2$.

In a preferred embodiment of the compounds of formula (II), $R^4$ is selected from the $C_{3-6}$ cycloalkyl, optionally substituted by 1, 2 or 3 groups independently selected from halogen, $C_{1-6}$ alkyl optionally substituted by halogen, $-(CR_cR_c)_{0-6}-OR_c$, and $-(CR_cR_c)_{0-6}-N(R_c)_2$.

In a preferred embodiment of the compounds of formula (II), $R^4$ is selected from a 4-6 membered heterocycloalkyl comprising 1, 2 or 3 heteroatoms independently selected from N, O or S, and optionally substituted by 1, 2 or 3 groups independently selected from halogen, $C_{1-6}$ alkyl optionally substituted by halogen, $-(CR_cR_c)_{0-6}-OR_c$, and $-(CR_cR_c)_{0-6}-N(R_c)_2$.

In a preferred embodiment of the compounds of formula (II), $R^4$ is selected from 5-6-membered heteroaryl

comprising 1, 2 or 3 heteroatoms independently selected from N, O or S, and optionally substituted by 1, 2 or 3 groups independently selected from: halogen, $C_{1-6}$ alkyl optionally substituted by halogen, $-(CR_cR_c)_{0-6}-OR_c$, and $-(CR_cR_c)_{0-6}-N(R_c)_2$.

In each of the above embodiments, the $-(CR_cR_c)_{0-6}-N(R_c)_2$ attached to the aryl, cycloalkyl, heterocycloalkyl or heteroaryl, via the group connected to N and together with the ring atom to which it is attached and the adjacent atom, optionally form a 4-7 membered nitrogen-containing heterocycle.

In a preferred embodiment of the compounds of formula (II), $R_c$ is at each occurrence independently selected from the H or $C_{1-6}$ alkyl.

The present disclosure also provides an embodiment of the compounds of formula (II), wherein $R^1$, $R^2$, $R^3$, $R^4$, $R^6$, $R^7$, $R^8$, A, E and $R_b$ each has the meaning in the embodiments, preferred, more preferred or most preferred embodiments given above for the compound of formula (I).

It should be noted that the compounds of the present disclosure cover each of the above independent embodiments or specific embodiments, and also cover embodiments resulting from any combination or subcombination of the above-mentioned various embodiments or specific embodiments, and also cover embodiments resulting from any combination of any of the above preferred or exemplary embodiments.

Specific embodiments of the compounds of the present disclosure include the following specific compounds or their isomers, pharmaceutically acceptable salts or solvates,

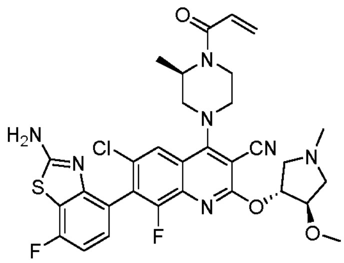
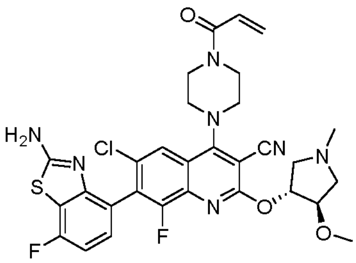
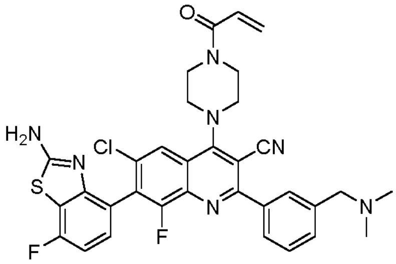

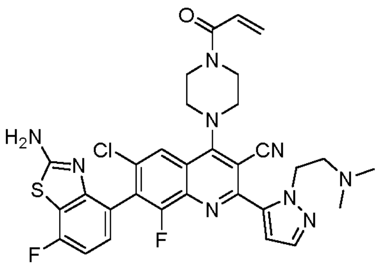
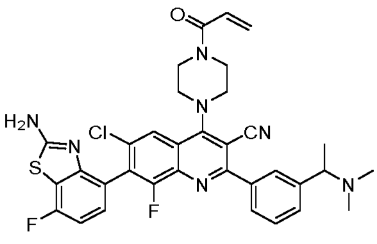
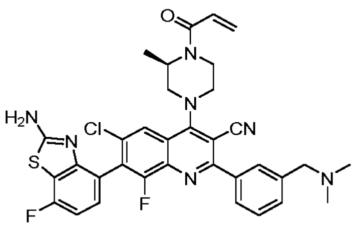

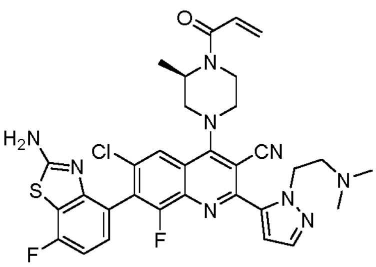
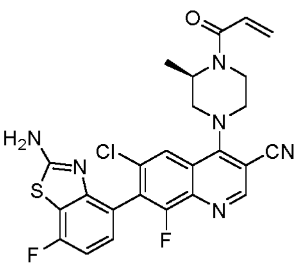
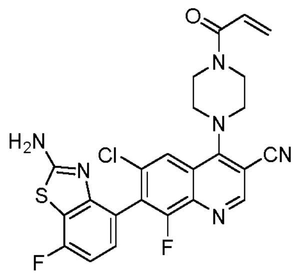

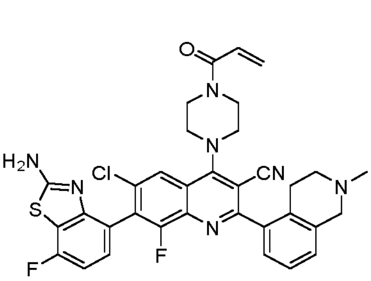
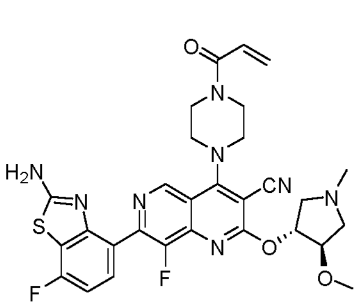
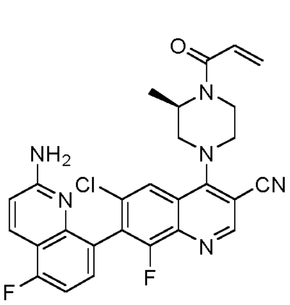

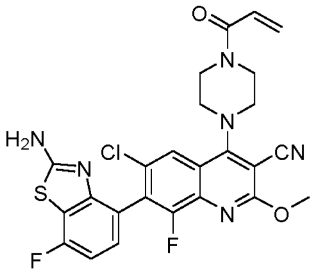
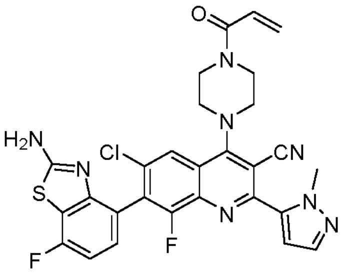
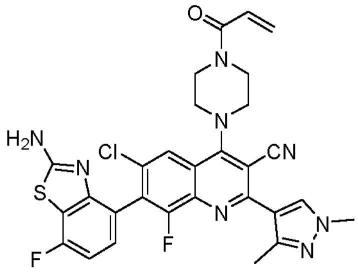

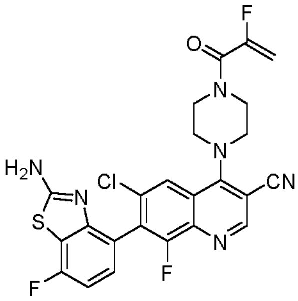
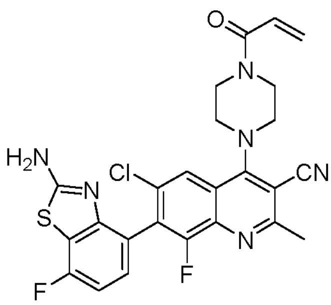
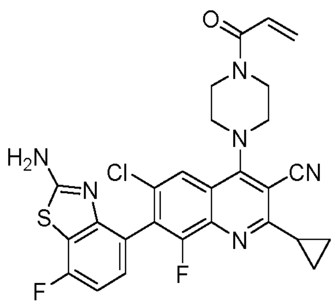

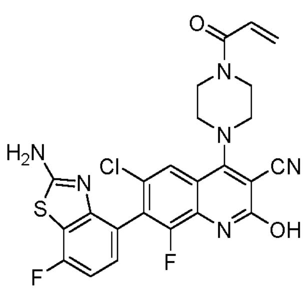
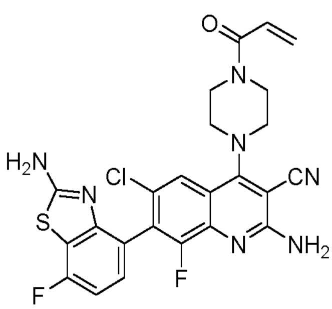
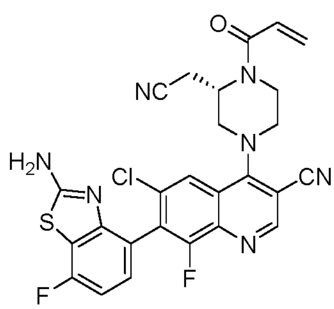

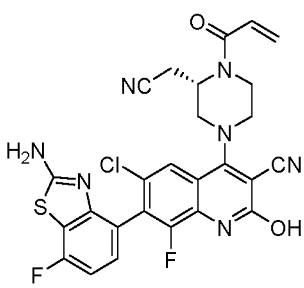
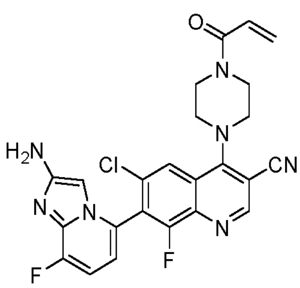
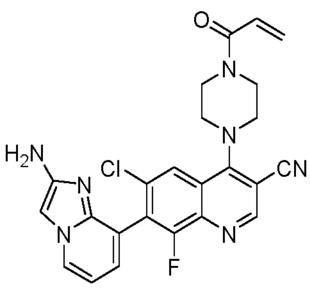

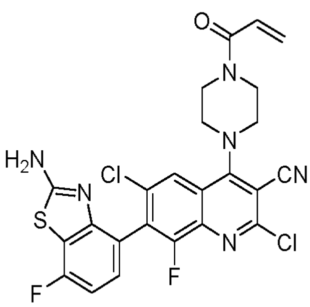
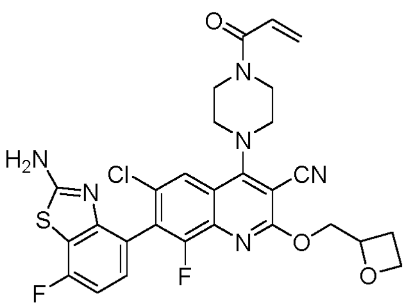
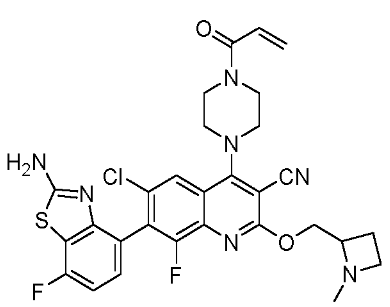

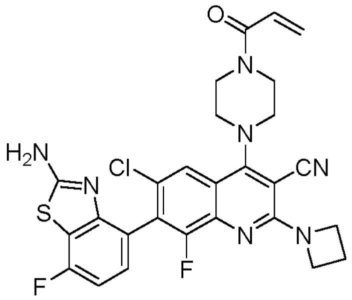
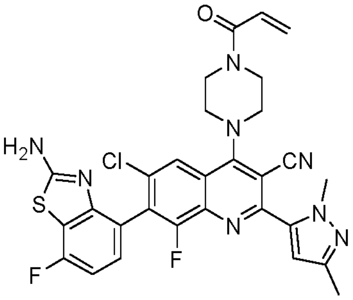
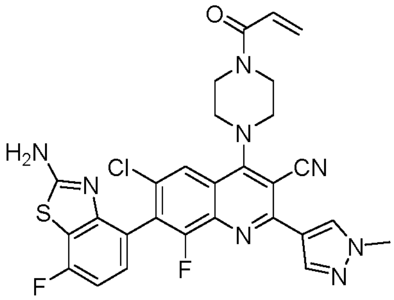

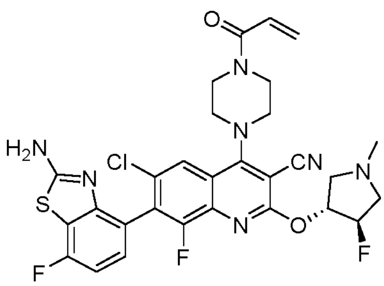
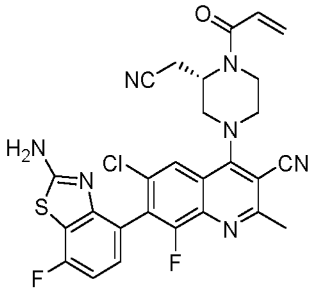
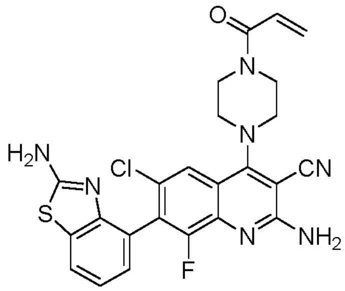

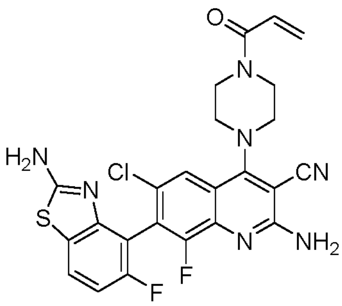
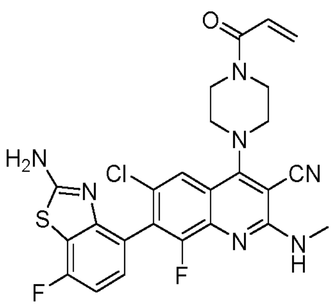
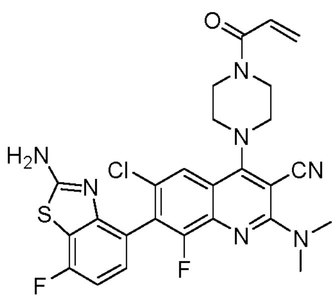

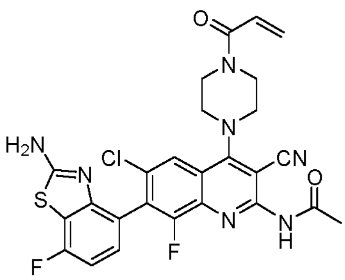
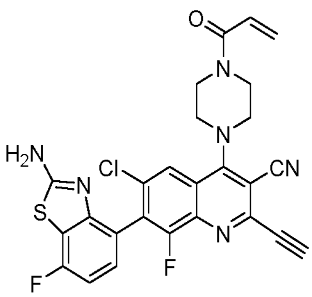
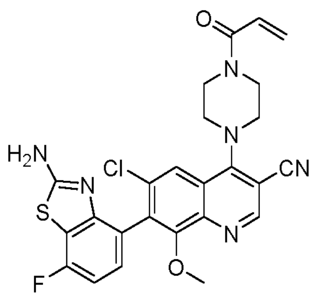
.

The compounds and their various specific embodiments defined above are inhibitors of a Ras mutation, particularly a KRas mutation, which include mutations on codons G12, G13 and Q61, such as G12C mutation, G12D mutation, G13D mutation. The compounds of the present disclosure, particularly those specific examples herein, show proliferation inhibitory activity in cell assays against Ras mutated, particularly KRas G12C mutated cells, as shown in the activity examples below. Therefore, the compounds of this disclosure can be used to treat or prevent diseases mediated by a Ras mutation, preferably a KRas mutation, and most preferably KRas G12C mutation, such as diseases or conditions that can be treated by inhibiting a Ras mutation, preferably a KRas mutation, most preferably KRas G12C mutation, or diseases or conditions in which a Ras mutation, preferably a KRas mutation, most preferably KRas G12C mutation activity plays a role or is involved, especially to treat or prevent tumors or cancers by inhibiting a Ras mutation, preferably a KRas mutation, and most preferably KRas G12C mutation.

In addition to showing inhibitory activity against the KRas G12C mutation, some compounds of the present disclosure also show inhibitory activity against the KRas G12D mutation and others show inhibitory activity against the KRas G13D mutation.

In addition to showing inhibitory activity against Ras mutation and preferably KRas mutation, the compounds

defined in this disclosure and their various specific embodiments, especially exemplary compounds, due to their improved structural patterns, retain comparable or enhanced, or even significantly enhanced inhibitory activity against KRas mutant protein and related cancer cell proliferation as compared with existing KRas mutant protein inhibitors in the prior art; have different biological activity profiles and can be used for new indications; have improved metabolic stability resulting in better pharmacokinetic properties; and have improved physicochemical properties resulting in good druggability, such as easier absorption in vivo, etc..

Based on the above, the present disclosure also provides technical solutions in the following aspects.

In one aspect, the present disclosure provides the compounds, isomers or pharmaceutically acceptable salts or solvates thereof, for use as medicaments.

In another aspect, the present disclosure provides the compounds, isomers or pharmaceutically acceptable salts or solvates thereof for treating and/or preventing diseases mediated by a Ras mutation, preferably a KRas mutation.

## Pharmaceutical compositions and uses thereof

In another aspect, the present disclosure provides pharmaceutical compositions comprising the compounds of formula (I) or formula (II), isomers or pharmaceutically acceptable salts or solvates thereof as defined above, as well as pharmaceutical carriers, diluents, or excipients. The pharmaceutical compositions of the present disclosure may be used to treat or prevent diseases mediated by a Ras mutation, particularly a KRas mutation, such as KRas G12C, KRas G12D or KRas G13D mutation, such as tumors or cancers.

The pharmaceutical compositions of the disclosure may be formulated by techniques known to a skilled person in the art, such as those disclosed in Remington's Pharmaceutical Sciences 20th edition.

The administration and application of the pharmaceutical compositions of the present disclosure are in accordance with good medical practice. Factors to consider in this context include specific disorders treated, specific mammals treated, clinical situation of individual patients, cause of the disorders, location of the agent delivery, method of administration, arrangement of administration, and other factors well-known to physician practitioners. The optimal dose level and frequency of administration of the pharmaceutical compositions of the present disclosure will be determined by clinical trials required in the pharmaceutical field. Typically, for example, the daily dose administered orally ranges from approximately 0.001 mg to approximately 100 mg per kg of patient weight, usually 0.01 mg to approximately 50 mg per kg of body weight, e.g., 0.1 to 10 mg per kg of body weight, preferably approximately 0.01 to approximately 35 mg per kg of body weight, administered as a single dose or divided doses. For a 70 kg human subject, suitable dose ranges from approximately 0.07 to approximately 7000 mg/day, preferably approximately 0.7 to approximately 2500 mg/day. It should be understood that it may be necessary to use doses beyond these limits in some cases.

The compositions of the present disclosure may be administered in any suitable manner, including oral, topical (including buccal and sublingual), rectal, vaginal, transdermal, parenteral, subcutaneous, intraperitoneal, intrapulmonary, intradermal, intrathecal, inhaled, and epidural and intranasal, and, if local therapy is required, intralesional. Parenteral infusion includes intramuscular, intravenous, intraarterial, intraperitoneal, or subcutaneous administration. In some embodiments, oral administration is used.

The compositions of the disclosure can be administered in any convenient form such as tablets, powders, capsules, lozenges, granules, solutions, dispersions, suspensions, syrups, sprays, suppositories, gels, emulsions, patches, etc. The compositions may contain conventional components in pharmaceutical formulations such as diluents (e.g., glucose, lactose, or mannitol), carriers, pH regulators, buffers, sweeteners, fillers, stabilizers, surfactants, humectants, lubricants, emulsifiers, suspending agents, preservatives, antioxidants, opacifiers, glidants, processing aids, colorants, flavoring agents, other known additives, and other agents. Suitable carriers and excipients are well-known by a skilled person in the art and detailed in e.g., Ansel, Howard C., et al, Ansel's Pharmaceutical Dosage Forms and Drug Delivery Systems. Philadelphia: Lippincott, Williams& Wilkins, 2004.

## Treatment methods and uses

As mentioned above, the compounds of formula (I) or formula (II) of the present disclosure and the compounds of various specific embodiments, particularly those specifically prepared and characterized in the Examples, show inhibitory effects on a Ras mutation, particularly a KRas mutation, such as KRas G12C, KRas G12D, or KRas G13D mutation.

Therefore, in one aspect, the present disclosure provides a method of suppressing a Ras mutation in cells, particularly a KRas mutation, preferably KRas G12C, KRas G12D or KRas G13D mutation, and most preferably KRas G12C mutation, comprising contacting the cells with a compound of formula (I) or formula (II), an isomer or pharmaceutically acceptable salt or solvate thereof of the present disclosure to suppress the Ras mutation in cells, particularly KRas mutation, preferably KRas G12C, KRas G12D or KRas G13D mutation, and most preferably KRas G12C mutation.

Based on the same properties, the disclosure correspondingly provides a method for inhibiting abnormal cell

growth in a mammal, comprising administering to the mammal a therapeutically effective amount of a compound of formula (I) or formula (II) of the disclosure, an isomer or a pharmaceutically acceptable salt or a solvate thereof, or a pharmaceutical composition comprising a compound of formula (I) or formula (II) of the disclosure, an isomer or a pharmaceutically acceptable salt or a solvate thereof.

In another aspect, the present disclosure provides a method for the treatment and/or prevention of diseases mediated by a Ras mutation, particularly KRas mutation, preferably KRas G12C, KRas G12D or KRas G13D mutation, and most preferably KRas G12C mutation, comprising administering to a subject in need thereof a therapeutically effective amount of a compound of formula (I) or formula (II) of the present disclosure, an isomers or a pharmaceutically acceptable salt or a solvate thereof, or a pharmaceutical composition comprising a compound of formula (I) or formula (II), an isomers or a pharmaceutically acceptable salt or a solvate thereof of the present disclosure.

In another aspect, the disclosure provides the use of a compound of formula (I) or formula (II) of the disclosure, an isomer or a pharmaceutically acceptable salt or a solvate thereof, or a pharmaceutical composition comprising the compound of formula (I) or formula (II), an isomer or a pharmaceutically acceptable salt or a solvate thereof, for inhibiting a Ras mutation in cells, particularly a KRas mutation, preferably KRas G12C, KRas G12D or KRas G13D mutation, most preferably KRas G12C mutation, or for inhibiting abnormal cell growth in a mammal, or for treating and/or preventing diseases mediated by a Ras mutation, especially a KRas mutation, preferably KRas G12C, KRas G12D or KRas G13D mutation, and most preferably KRas G12C mutation.

In another aspect, the disclosure provides the use of a compound of formula (I) or formula (II) of the disclosure, an isomer or a pharmaceutically acceptable salt or a solvate thereof, or a pharmaceutical composition comprising the compound of formula (I) or formula (II), an isomer or a pharmaceutically acceptable salt or a solvate thereof in the manufacture of medicaments for the treatment and/or prevention of diseases mediated by a Ras mutation, in particular a KRas mutation, preferrably KRas G12C, KRas G12D or KRas G13D mutation, and most preferrably KRas G12C mutation.

For each method and use technical solution provided by the disclosure, said abnormal cell growth or disease mediated by a Ras mutation, particularly a KRas mutation, preferably KRas G12C, KRas G12D or KRas G13D mutation, and most preferably KRas G12C mutation, refers in particular to cancers or tumors. Examples of the cancers or tumors include but are not limited to, lung cancer, lung adenocarcinoma, bone cancer, pancreatic cancer, skin cancer, head and neck cancer, skin or intraocular melanoma, uterine cancer, ovary cancer, rectum cancer, cancer in anal region, gastric cancer, colon cancer, breast cancer, carcinoma of fallopian tube, endometrial cancer, cervical cancer, carcinoma of vagina, carcinoma of vulva, Hodgkin's disease, esophagus cancer, carcinoma of small intestine, carcinoma of endocrine system, thyroid cancer, parathyroid cancer, adrenal gland cancer, soft tissue sarcoma, urethral cancer, penis cancer, prostate cancer, chronic or acute leukemia, lymphocytic lymphoma, bladder cancer, carcinoma of kidney or ureter, renal cell carcinoma, carcinoma of renal pelvis, central nervous system tumor (CNS), primary CNS lymphoma, spine tumor, brainstem glioma, or pituitary adenoma.

For each method and use technical solution provided by the disclosure, said abnormal cell growth or diseases mediated by a Ras mutation, particularly a KRas mutation, preferably KRas G12C, KRas G12D or KRas G13D mutation, is preferably selected from lung adenocarcinoma, lung cancer, colon cancer, rectum cancer, pancreatic cancer, cholangiocarcinoma, endometrial cancer, ovary cancer, leukemia; and most preferably from lung adenocarcinoma, colon cancer, rectum cancer, pancreatic cancer, cholangiocarcinoma.

Therefore, in a preferred embodiment in this respect, the present disclosure provides the technical solutions of the above methods and uses for treating or preventing cancers or tumors by inhibiting the KRas-G12C mutation. In a further preferred embodiment, the present disclosure provides technical solutions of the above methods and uses for treating or preventing lung adenocarcinoma, colon cancer, rectum cancer, pancreatic cancer, and cholangiocarcinoma by inhibiting the KRas-G12C mutation.

## Pharmaceutical combinations

A compound of the disclosure may be administered as the sole active ingredient or in combination with another drug or therapy.

Thus, in another aspect, the disclosure provides a pharmaceutical combination comprising, or consisting of, a compound of formula (I) or formula (II) of the disclosure, an isomers or a pharmaceutically acceptable salt or a solvate thereof and other active agents. This pharmaceutical combination is used to inhibit abnormal cell growth in a mammal or to treat and/or prevent diseases mediated by a Ras mutation, preferably a KRas mutation.

The other active agents may be one or more additional compounds of the disclosure, or may be a second or other (e.g., third) compounds compatible with the compound of the disclosure that do not adversely affect each other, or have complementary activities, for example, these active agents may be compounds known to modulate other bioactive pathways, or may be compounds that modulate different components of the bioactive pathways involved in the compounds of the disclosure, or may even overlap with the biological targets of the compounds of the disclosure.

In a specific embodiment, other active agents that may be combined with the compounds of the present disclosure include but are not limited to, chemotherapeutic agents, therapeutic antibodies, and radiotherapy, such as alkylating agents, antimetabolites, cell cycle inhibitors, mitotic inhibitors, topoisomerase inhibitors, antihormones, angiogenesis inhibitors, cytotoxic agents.

Other active agents used in combination with the disclosure may be administered simultaneously, separately or sequentially with the compounds of the disclosure by the same or different routes of administration. The other active agents may be dosed together with the compound of the disclosure in a single pharmaceutical composition or separately in discrete units different from the compound of the disclosure, for example, a combination product, preferably in the form of a kit, and may be carried out simultaneously or successively when administered separately, and the successive administration may be close or distant in time. They may be prepared and/or formulated by the same or different manufacturers. Moreover, the compounds of the present disclosure and other active agents may be added to the combination therapy (i) before delivering the combination product to the physician (for example, in the case of a kit containing the compounds of the present disclosure and other drugs), (ii) by the physician (or under the guidance of a physician) immediately before administration; (iii) by the patient himself, for example, during the sequential administration of the compounds of the present disclosure and other active agents.

The compounds of the disclosure may also be combined with antitumor therapies, which include but are not limited to surgery, radiation therapy, transplantation (e.g., stem cell transplantation, bone marrow transplantation), tumor immunotherapy, and chemotherapy.

Thus, in another aspect, the present disclosure also provides a kit comprising two or more separate pharmaceutical compositions, at least one containing the compound of formula (I) or formula (II) of the present disclosure, an isomer or a pharmaceutically acceptable salt or a solvate thereof, and devices containing each such composition, such as containers, sub-bottles or discrete foil packs, such as blister packs for packaging tablets, capsules, etc. The kit of the disclosure is particularly suitable for the administration of different dosage forms, such as oral and parenteral dosage forms, or for the administration of different compositions at different dosage intervals.

For the technical solutions of the pharmaceutical compositions, pharmaceutical combinations or kits of the disclosure, the abnormal cell growth or diseases mediated by a Ras mutation, in particular a KRas mutation, preferably KRas G12C, KRas G12D or KRas G13D mutation, and most preferably KRas G12C mutation, are as defined above for the methods and uses of the disclosure.

For the compounds, pharmaceutical compositions, methods, uses, pharmaceutical combinations and kits of the disclosure, the compound of formula (II), an isomer or a pharmaceutically acceptable salt or a solvate thereof as mentioned above, is preferred, and more preferably the compound as defined in the specific embodiments of formula (II) and the specific compounds as listed above, namely Compounds 1-25.

When describing the dose of a drug or its pharmaceutically acceptable salt in this disclosure, it is understood that this dose is based on the weight of the free base and does not include any of its hydrates or solvate unless the specification states that the dose is based on the weight of the salt, hydrate, or solvate.

## Description of figures

Figure 1 shows the antitumor activity and effect on body weight of Example 9 in a mouse xenograft model of human non-small cell lung cancer NCI-H358.

Figure 2 shows the antitumor activity and effect on body weight of Example 9 in a BALB/c nude mouse subcutaneously xenograft model of human pancreatic cancer Mia PaCa-2 cells.

## Preparation of the compounds of the present disclosure

In another aspect, the present disclosure provides a method for preparing the compounds as defined herein.

The compounds of formula (I) and formula (II) of the present disclosure or isomers or pharmaceutically acceptable salts or solvates thereof can be prepared in a variety of ways, including the methods given below, the methods given in examples or similar methods. The following illustrates general synthetic schemes for the synthesis of the compounds of the present disclosure.

Suitable reaction conditions for each reaction step of each general synthetic scheme are known to those skilled in the art or can be routinely determined. The process steps used to synthesize the compounds of the present disclosure can be under reaction conditions known per se (including those specifically mentioned), in the absence or usually in the presence of a solvent or diluent (including, for example, those solvents or diluents that are inert to the reagent used and dissolve the reagent used), in the absence or presence of catalysts, condensing agents or neutralizers (for example,

ion exchangers, such as cation exchangers, such as H + form), depending on the properties of the reaction and/or the reactants at a reduced, normal or elevated temperature (including, e.g., about -100°C to about 190°C, e.g., about -78°C to about 150°C, e.g., about 0°C to about 125°C, room temperature, - 20°C to 40°C or reflux temperature), under atmospheric pressure or in a closed container, under pressure when appropriate, and/or in an inert atmosphere such as argon or nitrogen.

Depending on the reactivity of the compounds used, the above reaction will normally be carried out at a temperature between room temperature and boiling temperature of the solvent used.

The raw materials and reagents used in the preparation of these compounds are generally commercially available, or can be prepared by methods described below, methods similar to those given below, or methods known in the art.

Unless otherwise stated in the description of the method, solvents suitable for any particular reaction include: those specifically mentioned, or, for example, water; esters, such as lower alkanoic acid lower alkyl esters, such as ethyl acetate; ethers, such as aliphatic ethers, such as diethyl ether, or cyclic ethers, such as tetrahydrofuran or dioxane; liquid aromatic hydrocarbons, such as benzene or toluene; alcohols, such as methanol, ethanol, or 1- or 2-propanol; nitriles, such as acetonitrile; halogenated hydrocarbons, such as dichloromethane or chloroform; amides, such as dimethylformamide, N-methylpyrrolidine-2-one or dimethylacetamide; bases, such as heterocyclic nitrogen bases, such as pyridine or triethylamine; carboxylic acid anhydrides, such as lower alkanoic acid anhydrides, such as acetic anhydride; cyclic, linear or branched hydrocarbons, such as cyclohexane, hexane or isopentane; or mixtures of these solvents, such as aqueous solutions. Such solvent mixtures can also be used for workup, for example by chromatography or partitioning.

If necessary, the raw materials and intermediates in the synthesis reaction process can be separated and purified by conventional techniques, including but not limited to filtration, distillation, crystallization, chromatography etc. Where the intermediates and final products are obtained in solid form, the purification can also be performed by recrystallization or aging. The materials can be characterized by conventional means including physical constants and spectral data.

The reaction mixture can be worked-up by conventional techniques, e.g., by mixing with water, separating the phases and, if necessary, the crude product can be purified by chromatography.

Those skilled in the art can recognize whether there are stereocenters in formula (I) and formula (II). At all stages of the reaction, the resulting mixture of isomers can be separated into individual isomers, such as diastereomers or enantiomers, or into any desired mixture of isomers, such as racemates or mixtures of diastereomers, see for example, "Stereochemistry of Organic Compounds" by E.L. Eliel, S. H. Wilen, and L.N. Mander's (Wiley-Interscience, 1994).

In some specific cases, it may be necessary to protect specific reactive groups, which may be present in formula (I) or formula (II) and may compete or interfere with the reaction, from side reactions with other reactive groups, using appropriate protecting groups. By way of example only, if one or more groups in a compound of formula (I) or formula (II) is or contains the group C(O)OH, $NH_2$ or OH and the group has a similar or even more strong reactivity than the desired reaction position, it is advantageous to protect these groups before the desired reaction occurs. In these cases, additional deprotection steps may be necessary to remove these protecting groups after the desired reaction is complete. Suitable protecting groups and the use of such suitable protecting groups to protect and deprotect different substituents are well-known by those in the art, examples can be found in T. W. Greene and P. G. M. Wuts, Protective Groups in Organic Synthesis (3rd Edition), John Wiley & Sons, NY (1999).

The present disclosure also relates to a preparation method in which a compound that can be obtained in the form of an intermediate in each of the preparation methods described above and any step in the following processes, is used as a starting material and the remaining process steps are carried out, or in which the starting materials are formed in situ under the reaction conditions or are used in the form of derivatives, for example in protected form or salt form, or the compounds obtainable according to the method of the disclosure are produced under the conditions of the method and are further processed in situ.

**Synthetic scheme I:**

Compounds of the disclosure can be prepared according to the following exemplary schemes, or analogously, wherein the variables are as defined above if not stated otherwise.

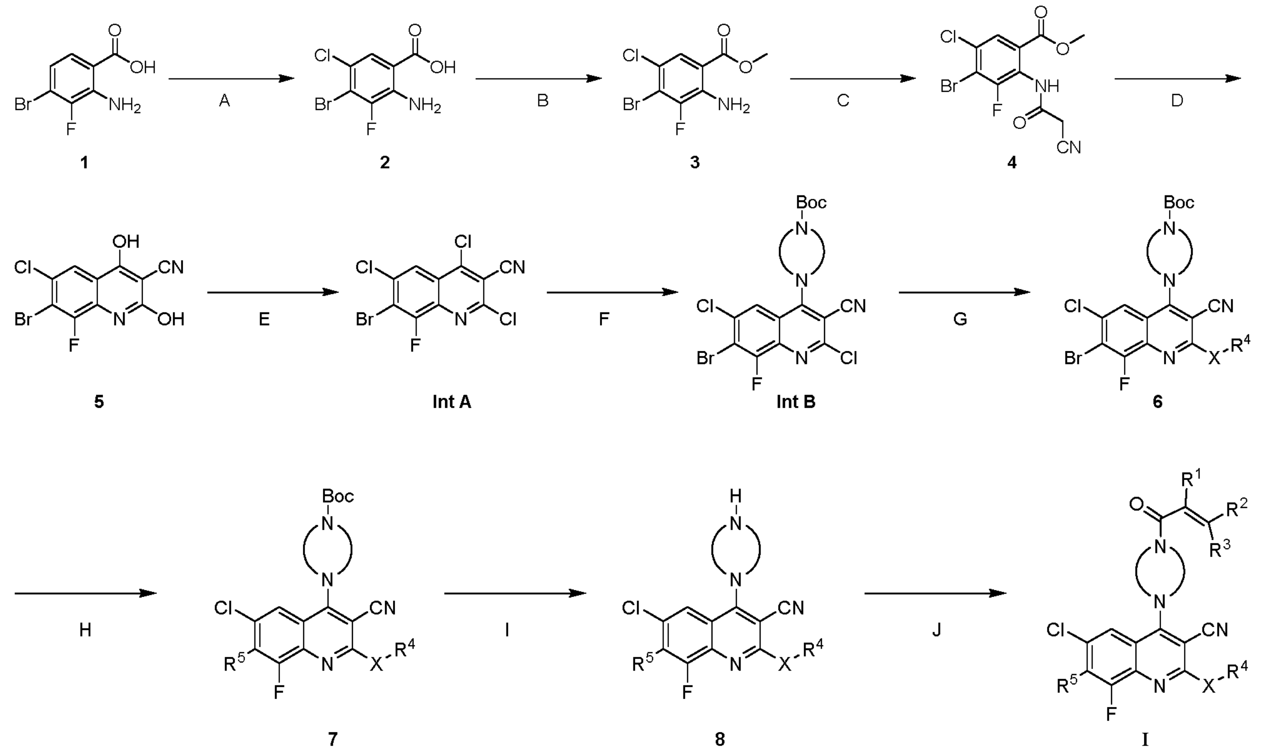


1 2 3 4

5 Int A Int B 6

7 8 I

The compounds of formula (I) or formula (II) are prepared through synthesis scheme I in the present disclosure. Compound 2 is obtained by chlorination of the aromatic compound 1 in Step A. Compound 3 is obtained by methyl-esterification in Step B followed by condensation of amino and acid in Step C to obtain Compound 4. Compound 4 is cyclized under basic conditions to afford Compound 5 which is then chlorinated to afford the intermediate Int A. Key intermediate Int A is subjected to nucleophilic substitution reaction with a piperazine or piperazine derivative carrying a single protecting group under basic conditions to afford intermediate Int B. Int B, via aromatic nucleophilic substitution (when X = O) or metal-catalyzed coupling (when X = direct linkage), affords Compound 6. In Step H, $R^5$ group (with a protecting group) is introduced into Compound 6 via a catalytic coupling reaction to afford Compound 7. Compound 7 is deprotected to afford Compound 8, which is acylated with an acyl chloride or carboxylic acid to afford the compounds of general formula I.

Typical reaction conditions and reagents used for the chlorination, esterification, condensation, cyclization, nucleophilic substitution, catalytic coupling and acylation reactions involved in Scheme I are well known in the art and fall within the scope of conventional practice for those skilled in the art, or can be determined by one skilled in the art based on the typical conditions for such reactions in the art, the starting materials used and characteristics of target products and upon suitable modifications.

**Synthetic scheme II:**

The compounds of the present disclosure with -X-$R^4$ as H can be synthesized according to the following illustrative general scheme.

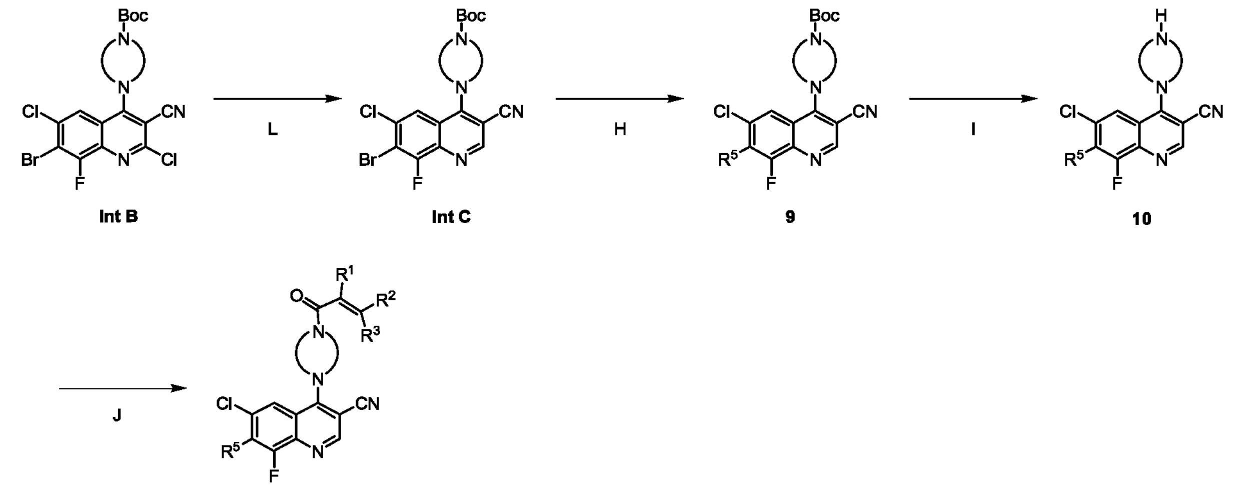


Int B Int C 9 10

In Scheme 2, the compound Int B is dechlorinated by catalytic reduction to give intermediate Int C, which undergoes the same reaction Steps H, I, J as illustrated in Scheme I to give the compound with -X-$R^4$ as H.

**Synthetic scheme III:**

The compounds of the present disclosure with A as N can be synthesized according to the following illustrative general scheme.

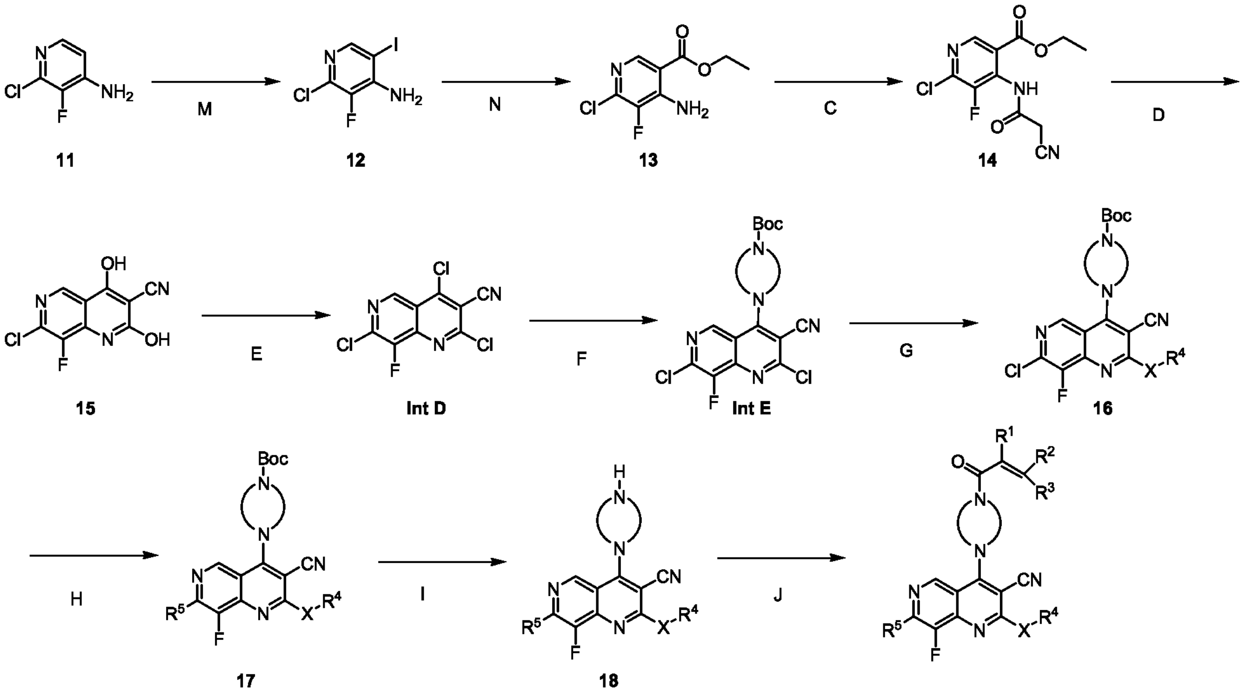


11 12 13 14 15 Int D Int E 16 17 18

In this Scheme, Compound 11 undergoes iodination to give Compound 12, which is further subjected to metal-catalyzed carbonylation reaction to give Compound 13. Starting from Compound 13, the same Steps C~J in Scheme I is performed to give the compound of the present disclosure with A as N.

**Synthetic Scheme IV:**

The compounds of the present disclosure where A is N and -X-$R^4$ is H can be synthesized according to the following illustrative general scheme.

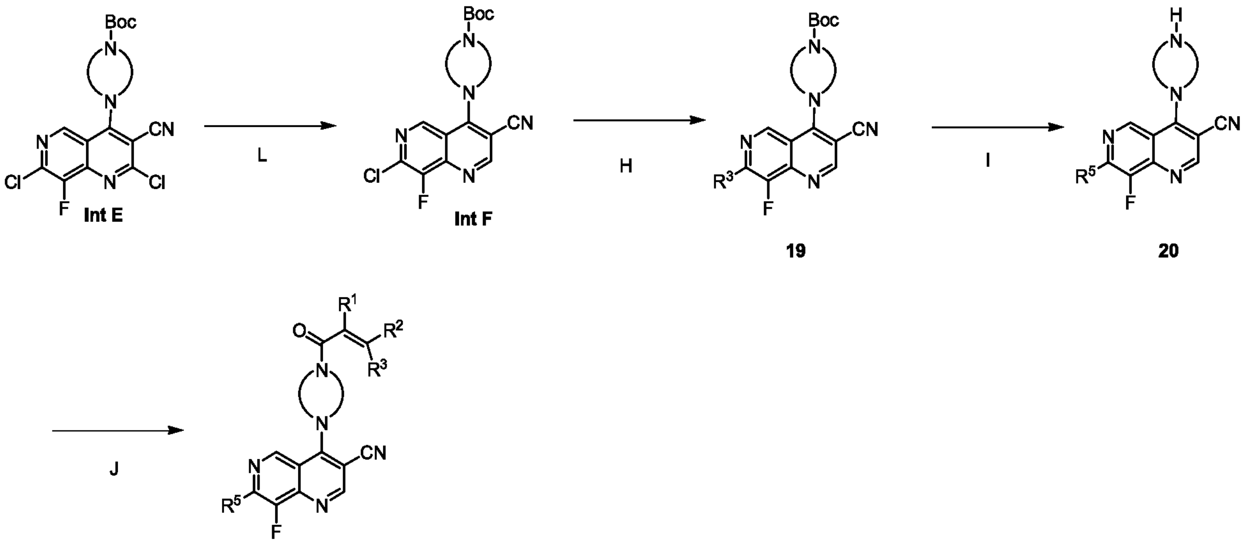

Int E
Int F
19
20

In this Scheme, starting from intermediate Int E, the same process as in Scheme II is performed to synthesize the compound of the present disclosure with A is N and $-X-R^4$ is H.

In the above synthesis Schemes I, II, III and IV,

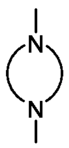

represents the piperazine ring, and the typical reaction conditions for each reaction involved are well known in the art and fall within the scope of conventional practice for those skilled in the art, or can be determined by one skilled in the art based on the typical conditions for such reactions in the art, the starting materials used and characteristics of target products and upon suitable modifications.

## Synthetic examples

The present invention will be further described below in conjunction with examples. It should be noted that the following examples are illustrative and should not be considered as limiting the protection scope of the present invention.

In the description of the embodiments and the specific examples that follow, the following abbreviations are used: Boc (tert-butoxycarbonyl); n-BuLi (n-butyllithium); t-BuOK (potassium tert-butoxide); $CDCl_3$ (deuterochloroform); DCM (dichloromethane); DIEA (N,N-diisopropylethylamine); DME (ethylene glycol dimethyl ether); DMF (N,N-dimethylformamide); DMSO (dimethylsulfoxide); DMSO-d6 (hexadeuteriodimethylsulfoxide); EA (ethyl acetate); EDTA-K2 (dipotassium ethylenediaminetetraacetic acid); EtOH (ethanol); FCC (flash column chromatography); g (gram); h (hour); HATU (2-(7-azabenzotriazole)-N,N,N',N'-tetramethyluronium hexafluorophosphate); HCl (hydrogen chloride); HLM (human liver microsomes); $H_2O$ (water); $H_2SO_4$ (sulfuric acid); IV (intravenous administration); $K_2CO_3$ (potassium carbonate); KOH (potassium hydroxide); LCMS (LC-MS); LC-MS/MS (LC-MS-MS); MeCN (acetonitrile); MeOH (methanol); Methanol-d4 (tetradeuteromethanol); mg (mg); MHz (megahertz); min (minutes); mL (milliliter); mmol (mmol); m/z (mass/charge); $N_2$ (nitrogen); NaCl (sodium chloride); NaH (sodium hydride); $NaHCO_3$ (sodium bicarbonate); NaOH (sodium hydroxide); $Na_2SO_3$ (sodium sulfite); $Na_2SO_4$ (sodium sulfate); $NCCH_2CO_2H$ (2-cyanoacetic acid); NCS (chlorosuccinimide); $NH_4Cl$ (ammonium chloride); NIS (iodosuccinimide); NMI (N-methylimidazole); NMP (N-methylpyrrolidone); NMR (nuclear magnetic resonance); Pd/C (palladium on carbon); $Pd_2(dba)_3$(tris(dibenzylideneacetone)dipalladium); $Pd(dppf)Cl_2$ (1,1'-bis-diphenylphosphine ferrocene palladium dichloride); $Pd(PPh_3)_4$ (tetrakistriphenylphosphine palladium); PE (petroleum ether); PEG (polyethylene glycol); PO (oral administration); $POCl_3$ (phosphorus oxychloride); r.t. (room temperature); $SiO_2$ (silica gel); TCFH (N,N,N',N'-tetramethylchloroformamidine hexafluorophosphate); TEA (triethylamine); TFA (trifluoroacetic acid); THF (tetrahydrofuran); TLC (thin layer chromatography); TsOH (p-toluenesulfonic acid); $TsOH.H_2O$ (p-toluenesulfonic acid monohydrate); μL (microliter); μM (micromolar concentration); pmol (micromole); v/v (liquid volume ratio); w/w (mass ratio); Xantphos (4,5-bis-diphenylphosphine-9,9-dimethylxanthene).

The names and structures of the synthesized compounds are given in the following embodiments. Any deviation

between name and structure is unintentional, in which case the structure is decisive.

The experimental methods for which specific conditions are not indicated in the following examples usually follow the conventional conditions for this type of reaction or the conditions recommended by the manufacturer. Unless otherwise specified, percentages and parts are percentages by weight and parts by weight. Unless otherwise specified, ratios of the liquid are by volume.

Unless otherwise specified, the experimental materials and reagents used in the following examples can be obtained from commercial channels, prepared according to the methods of prior art, or prepared according to a method similar to that disclosed in the present application.

In the following examples, $^1$H-NMR spectra were recorded with Bruker (400 MHz) and chemical shifts are expressed in $\delta$ (ppm) relative to the deuterated solvent peak ($CDCl_3$: $\delta$ = 7.26 ppm; $CD_3OD$: $\delta$ = 3.31 ppm; DMSO-*d6*: $\delta$ = 2.50 ppm); mass spectroscopy were recorded with an Aglient 1100 liquid chromatography + Aglient G6100 mass spectrometry LCMS instrument.

**Synthesis of Intermediate a**

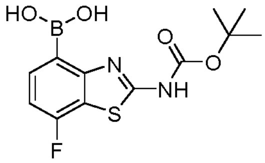

(2-((*tert*-Butoxycarbonyl)amino)-7-fluorobenzothiazol-4-yl)boronic acid

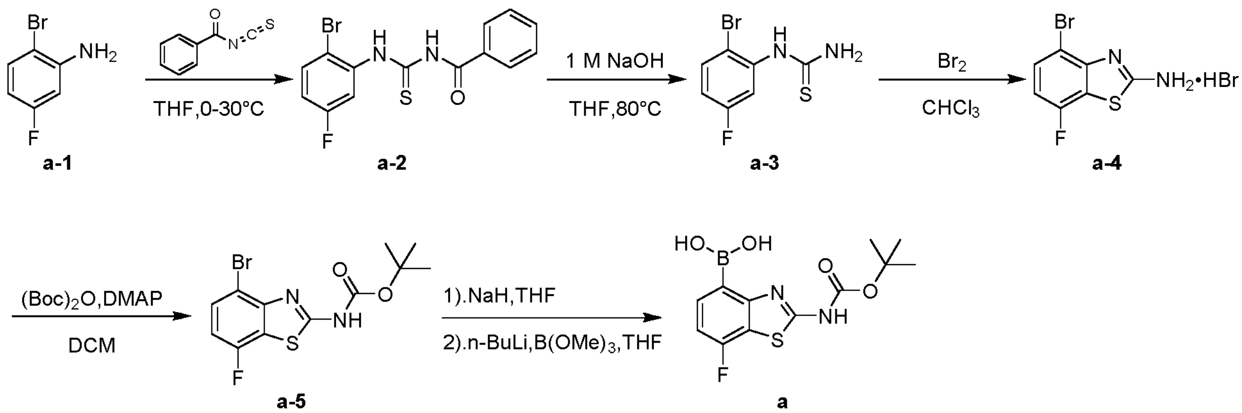


a-1 a-2 a-3 a-4 a-5 a

**Step A:** *N*-((2-Bromo-5-fluorophenyl)carbamothioyl)benzamide

Benzoyl isothiocyanate (28 g, 210 mmol) was dissolved in THF (300 mL) in a round bottom flask with a magnetic stirring bar. After cooling in an ice bath, a solution of 2-bromo-5-fluoroaniline (40 g, 210 mmol) in THF (100 mL) was added dropwise to the system under stirring. After the addition was complete, the stirring was continued for 1 h. LCMS showed the reaction was complete. Concentration under reduced pressure provided the crude product which was used directly in the next step. LCMS (m/z): 353.1 (M + H).

**Step B:** 1-(2-Bromo-5-fluorophenyl)thiourea

To a solution of *N*-((2-bromo-5-fluorophenyl)carbamothioyl)benzamide (crude from Step A) in THF (300 mL) was added a solution of NaOH (16.8 g, 420 mmol) in water (150 mL) under stirring at room temperature, heated to 80°C and reacted for 16 h. After concentration under reduced pressure, the resulting residue was diluted with EA (200 mL), and washed with water (150 mL). The organic phase and aqueous phase were separated and the aqueous phase was then extracted twice with EA (100 mL $\times$ 2). The combined organic layers were washed with saturated sodium chloride

aqueous solution, dried over $Na_2SO_4$, and filtrated. The filtrate was concentrated under reduced pressure to give a crude which was suspended in petroleum ether (200mL), and filtered to give 1-(2-bromo-5-fluorophenyl) thiourea (40 g, yield 77%) as a white solid. LCMS (m/z): 249.0, 251.0 (M + H).

**Step C:** 4-Bromo-7-fluorobenzo[*d*]thiazol-2-amine • hydrobromide

A solution of bromine (23 g, 141 mmol) in chloroform (70 mL) was slowly added dropwise to an ice-cooled solution of 1-(2-bromo-5-fluorophenyl)thiourea (35 g, 141 mmol) in chloroform (300 mL). After the addition was complete, the ice bath was removed and the reaction was heated to 70°C and reacted for 48 h. The reaction was complete, concentrated under reduced pressure, suspended in EA (100 mL), and filtered to give 4-bromo-7-fluorobenzo[*d*]thiazol-2-amine hydrobromide (30.5 g, yield 66%) as a white solid. LCMS (m/z): 247.0, 249.0 (M + H).

**Step D:** *tert*-Butyl (4-bromo-7-fluorobenzo[*d*]thiazol-2-yl)carbamate

A solution of di-tert-butyl dicarbonate (61 g, 280 mmol) in DCM (60 mL) was slowly added to a solution of 4-bromo-7-fluorobenzo[*d*]thiazol-2-amine·hydrobromide (30 g, 93 mmol) and 4-dimethylaminopyridine (14 g, 112 mmol) in DCM (300 mL) at room temperature. The reaction was continued at r.t. for 3 h and LCMS monitored the completion of the reaction. After concentration under reduced pressure, the reaction was diluted with EA (200 mL), and washed with water (150 mL). The organic phase and aqueous phase were separated and the aqueous phase was then extracted twice with EA (200 mL × 2). The combined organic phases were washed with saturated sodium chloride aqueous solution, dried over anhydrous $Na_2SO_4$, filtered, and concentrated under reduced pressure. The residue was suspended in PE/EA (20:1), and filtered to give *tert*-butyl (4-bromo-7-fluorobenzo[*d*]thiazol-2-yl)carbamate (18 g, yield 56%) as a white solid. $^1H$ NMR (400 MHz, Chloroform-*d*) δ 9.29 (s, 1H), 7.54 (dd, *J*= 8.6, 4.8Hz, 1H), 6.94 - 6.81 (m, 1H), 1.52 (s, 9H). LCMS (m/z): 291.0, 293.0 (M + H).

**Step E:** (2-((*tert*-Butoxycarbonyl)amino)-7-fluorobenzothiazol-4-yl)boronic acid

NaH (60% w/w, 3g, 75.6 mmol) was added to an ice-cooled solution of *tert*-butyl (4-bromo-7-fluorobenzo[*d*]thiazol-2-yl)carbamate (17.5 g, 50.4 mmol) in THF (200 mL) with stirring under nitrogen. The mixture was stirred at same temperature for an additional 30 min and then transferred to a -68°C dry ice/ethanol bath. *n*-Butyllithium (30.2 mL, 75.6 mmol, 2.5 M/THF) was slowly added, stirred for 10 min, trimethyl borate (156 g, 151 mmol) was added, and the resulting mixture was stirred for an additional 30 min. The reaction was quenched with saturated aqueous ammonium chloride (50 mL), diluted with EA (200 mL), washed with water (150 mL), separated the aqueous phase and organic phase and the aqueous phase was extracted twice with EA (100 mL × 2). The combined organic layers were dried over anhydrous $Na_2SO_4$, filtered, and the filtrate was concentrated under reduced pressure to give the crude product. The crude product was suspended in petroleum ether and filtered to afford (2-((tert-butoxycarbonyl)amino)-7-fluorobenzo[*d*]thiazol-4-yl)boronic acid (10.7 g, yield 68%) as a white solid. $^1H$ NMR (400 MHz, DMSO-$d_6$) δ 12.25 (s, 1H), 8.50 (s, 2H), 7.84 (dd, *J* = 8.0, 6.4 Hz, 1H), 7.22 - 7.13 (m, 1H), 1.54 (s, 9H). LCMS (m/z): 313.2 (M + H).

**Synthesis of intermediate Int A**

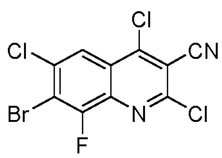

7-Bromo-2,4,6-trichloro-8-fluoroquinoline-3-carbonitrile

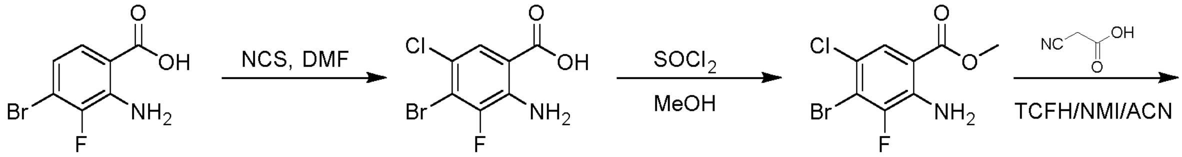


**A-1** **A-2** **A-3**

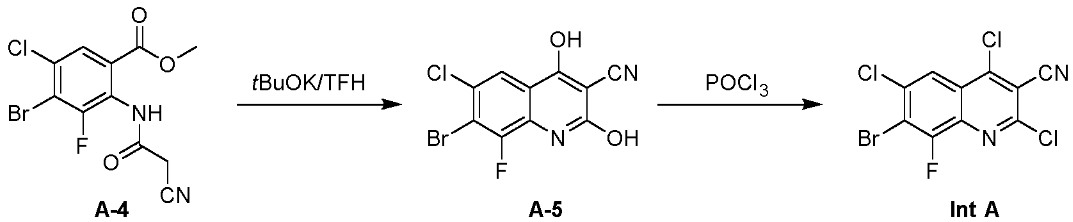


**A-4** **A-5** **Int A**

**Step A:** 2-Amino-4-bromo-5-chloro-3-fluorobenzoic acid

NCS (13.7 g, 102.6 mmol) was added in portions to a solution of 2-amino-4-bromo-3-fluorobenzoic acid (20 g, 85.5 mmol) in DMF (140 mL) at r.t. The reaction was then heated to 75°C and stirred at this temperature for 20 h. The reaction solution was cooled to room temperature and poured into 700 mL of ice-water mixture. The resulting precipitate was collected by filtration, washed with water (500 mL) and petroleum ether (150 mL) respectively, and dried under vacuum to give 2-amino-4-bromo-5-chloro-3-fluorobenzoic acid (20 g, yield 87%) as a pale-yellow solid. LCMS (m/z): 268.0 (M + H).

**Step B:** Methyl 2-amino-4-bromo-5-chloro-3-fluorobenzoate

2-Amino-4-bromo-5-chloro-3-fluorobenzoic acid (20g, 74.5 mmol) was dispersed into methanol (150 mL). Under stirring and cooling in ice-water bath, sulfoxide chloride (50 mL) was added dropwise to the reaction solution. After the addition was complete, the reaction mixture was placed at 65°C for reaction for 20h. TLC monitored the reaction was complete. The solvent was concentrated to dry to give the target product methyl 2-amino-4-bromo-5-chloro-3-fluorobenzoate (15.8 g, yield 75%). LCMS (m/z): 282.0 (M + H).

**Step C:** Methyl 4-bromo-5-chloro-2-(2-cyanoacetamido)-3-fluorobenzoate

Methyl 2-amino-4-bromo-5-chloro-3-fluorobenzoate (15.8 g, 55.9 mmol), TCFH (18.8 g, 67.1 mmol) and cyanoacetic acid (5.2 g, 61.3 mmol) were dissolved in acetonitrile (100 mL) at room temperature at room temperature, and added with *N*-methylimidazole (6.7 mL, 83.9 mmol) under stirring. The reaction mixture was heated to 60°C and stirred for 20 h. TLC monitored the reaction was complete. The reaction solution was cooled to r.t. and poured into water (500 mL). The resulting white precipitate was collected by filtration, washed with water (500 mL) and EA/PE (V/V=1:4, 150 mL) respectively, and dried under vacuum to give methyl 4-bromo-5-fluoro-2-(2-cyanoacetamido)-3-fluorobenzoate (15 g, yield 76%) as a white solid. LCMS (m/z): 349.0 (M + H).

**Step D:** 7-Bromo-6-chloro-8-fluoro-2,4-dihydroxyquinoline-3-carbonitrile

The compound methyl 4-bromo-5-chloro-2-(2-cyanoacetamido)-3-fluorobenzoate (6g, 17.2 mmol) was dispersed in THF (120 mL), and added in portions with potassium tert-butoxide (3.7 g, 32.6 mmol) in ice bath under stirring. After the addition was complete, the reaction mixture was stirred in ice bath for 1 h. LCMS monitored the reaction was complete. After the solvent was removed by distillation under reduced pressure, the reaction was added with water (200 mL), adjusted to pH=7 with hydrochloric acid (1 N in water), and filtered to collect solid. The solid was washed successively with water (100 mL) and petroleum ether (150 mL), and dried under vacuum to afford 7-bromo-6-chloro-8-fluoro-2,4-dihydroxyquinoline-3-carbonitrile (5.4 g, yield 99%) as a white solid. LCMS (m/z): 317.0 (M + H).

**Step E:** 7-Bromo-2,4,6-trichloro-8-fluoroquinoline-3-carbonitrile

The compound 7-Bromo-6-chloro-8-fluoro-2,4-dihydroxyquinoline-3-carbonitrile (5.4 g, 17 mmol) was dispersed in phosphorous oxychloride (100 mL) under stirring at room temperature. The reaction was heated to 120°C and stirred

for 24 h. After cooling to r.t., the mixture was concentrated under vacuum to remove most of phosphorus oxychloride, and acetonitrile (20 mL) was added. The solution of the product in acetonitrile was slowly poured into water (250 mL), and yellow solid precipitated. The solid was collected by filtration, washed successively with water (200 mL) and petroleum ether (100 mL), and dried under vacuum to afford 7-bromo-2,4,6-trichloro-8-fluoroquinoline-3-carbonitrile (5.4 g, yield 90%) as a yellow solid. LCMS (m/z): 353.0 (M + H).

**Synthesis of intermediate Int B1**

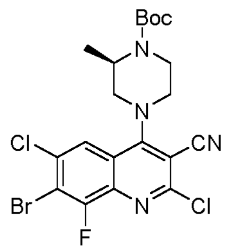

*tert*-Butyl (*R*)-4-(7-bromo-2,6-dichloro-3-cyano-8-fluoroquinolin-4-yl)-2-methylpiperazine-1 - carboxylate

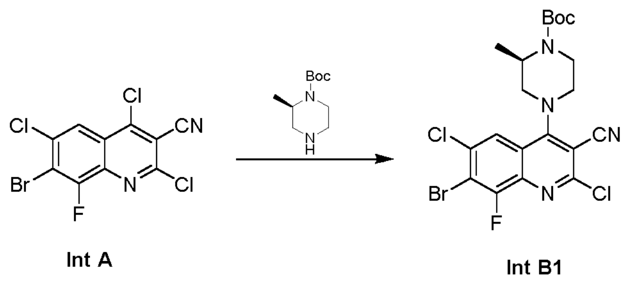

To a solution of 7-bromo-2,4,6-trichloro-8-fluoroquinoline-3-carbonitrile (intermediate Int A, 7.8 g, 22 mmol) in THF (100 mL) were added DIEA (7.3 mL, 44 mmol) and tert-butyl *(R)-2*-methylpiperazine-1-carboxylate (4.4 g, 22 mmol) successively under stirring at r.t. The resulting mixture was continued stirring for 1 h at r.t. After the reaction was complete, the reaction was concentrated by rotary evaporator to give the crude product which was washed with PE/THF mixed solvent (V/V = 10:1, 500 mL) to afford *tert*-butyl (R)-4-(7-bromo-6-dichloro-3-cyano-8-fluoroquinolin-4-yl)-2-methylpiperazine-1-carboxylate (9.0 g, yield 79%) as a yellow solid. LCMS (m/z): 519.2 (M + H).

**Synthesis of intermediate Int B2**

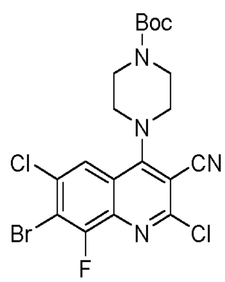

*tert*-Butyl 4-(7-bromo-2,6-dichloro-3-cyano-8-fluoroquinolin-4-yl)piperazine-1-carboxylate Intermediate Int B2 was synthesized by referring to the procedure as described in intermediate Int B1, using *tert*-butyl piperazine-1-carboxylate in place of *tert*-butyl (*R*)-2-methylpiperazine-1-carboxylate. LCMS (m/z): 503.2 (M + H).

**Synthesis of intermediate Int C1**

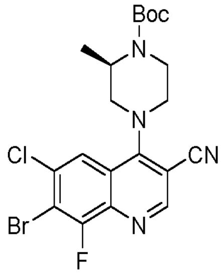

*tert*-Butyl (*R*)-4-(7-bromo-6-chloro-3-cyano-8-fluoroquinolin-4-yl)-2-methylpiperazine-1-carboxylate

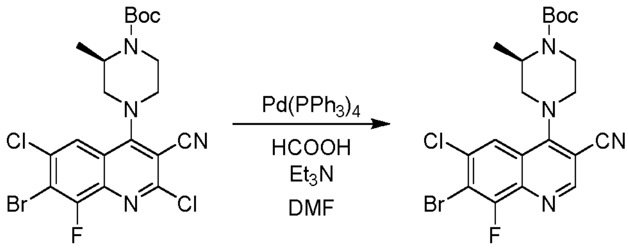


**Int B1** **Int C1**

To a round-bottom flask equipped with magnetic stirring bar were added *tert*-butyl (*R*)-4-(7-bromo-2,6-dichloro-3-cyano-8-fluoroquinolin-4-yl)-2-methylpiperazine-1-carboxylate (intermediate Int B1, 206 mg, 0.4 mmol), Pd $(PPh_3)_4$ (23 mg, 20 pmol), formic acid (55 mg, 1.2 mmol), and triethylamine (80.5 mg, 0.8 mmol), purged with nitrogen for three times, and added with DMF (4 mL). The reaction was heated to 50°C and stirred for 6 h. After the reaction was complete, the mixture was concentrated by rotary evaporator and the resulting crude was purified by FCC ($SiO_2$, EA/PE = 0-50%) to give *tert*-butyl (*R*)-4-(7-bromo-6-chloro-3-cyano-8-fluoroquinolin-4-yl)-2-methylpiperazine-1-carboxylate (140 mg, yield 73%). LCMS (m/z): 485.3 (M + H).

**Synthesis of intermediate Int C2**

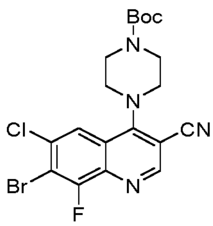

*tert*-Butyl 4-(7-bromo-6-chloro-3-cyano-8-fluoroquinolin-4-yl)piperazine-1-carboxylate

Intermediate Int C2 was synthesized by referring to the procedure as described in intermediate Int C1, using *tert*-butyl 4-(7-bromo-2,6-dichloro-3-cyano-8-fluoroquinolin-4-yl)piperazine-1-carboxylate (intermediate B2) in place of *tert*-butyl (R)-4-(7-bromo-2,6-dichloro-3-cyano-8-fluoroquinolin-4-yl)-2-methylpiperazine-1-carboxylate (intermediate Int B1). LCMS (m/z): 459.2, 471.2 (M + H).

**Synthesis of intermediate Int D**

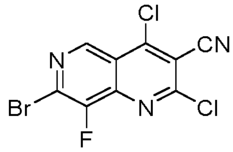

2,4,7-dichloro-8-fluoro-1,6-naphthyridine-3-carbonitrile

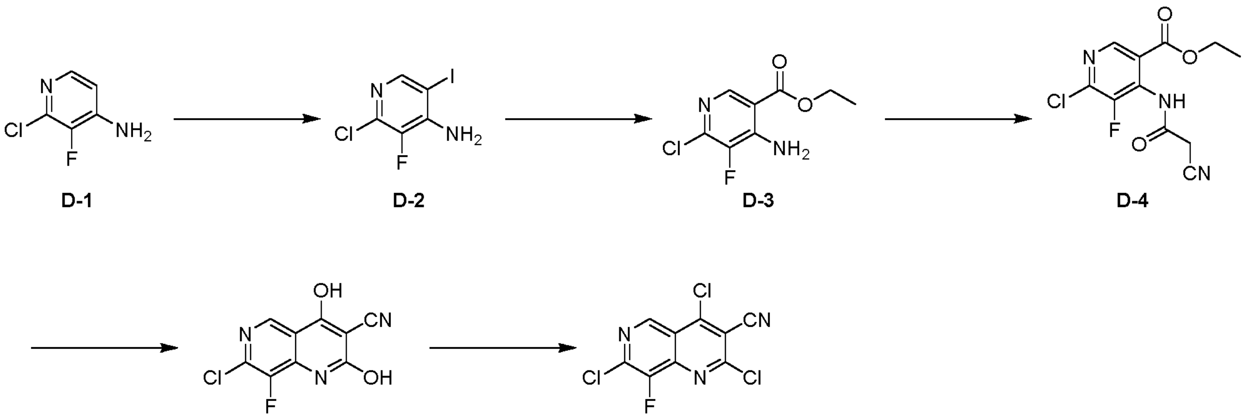

D-1 D-2 D-3 D-4

D-5 Int D

**Step A:** 2-Chloro-3-fluoro-5-iodopyridin-4-amine

2-Chloro-3-fluoropyridin-4-amine (3 g, 20.47 mmol) and NIS (6.91 g, 30.71 mmol) were dissolved in acetonitrile (30 mL) in a round-bottom flask at room temperature, and TsOH·$H_2O$ (400 mg, 2 mmol) was added under stirring. The reaction solution was heated to 70°C and allowed to react for 16 h. After TLC monitored the completion of the reaction, the reaction solution was cooled to room temperature, poured into saturated brine (100 mL), and extracted with ethyl acetate (25 mL × 3). The combined organic phases were washed with saturated aqueous $Na_2SO_3$ solution and brine successively, dried over $Na_2SO_4$, filtered, and concentrated to afford 2-chloro-3-fluoro-5-iodopyridin-4-amine (5.4 g, yield 97%) as a yellow solid. LCMS (m/z): 273.0 (M + H).

**Step B:** Ethyl 4-amino-6-chloro-5-fluoronicotinate

2-Chloro-3-fluoro-5-iodopyridin-4-amine (5.4 g, 19.82 mmol), Pd(dppf)$Cl_2$ (695 mg, 0.91 mmol), and triethylamine (555 mg, 49.6 mmol) were dispersed in absolute ethanol, heated to 90°C and allowed to react for 40 h under a carbon monoxide atmosphere. After the reaction was complete, the reaction was filtered through a pad of celite, and the filtrate was concentrated and purified by FCC ($SiO_2$, EA/DCM = 0-100%) to afford ethyl 4-amino-6-chloro-5-fluoronicotinate (3.5g, yield 81%) as a yellow solid. LCMS (m/z): 219.0 (M + H).

**Step C:** Ethyl 6-chloro-4-(2-cyanoacetamido)-5-fluoronicotinate

2-Cyanoacetic acid (778 mg, 9.15 mmol) and 2,2,2-trifluoroacetic anhydride (1.44 g, 6.86 mmol) were dissolved in acetonitrile (5 mL), and stirred at 55°C for 2 h. The reaction solution and ethyl 4-amino-6-chloro-5-fluoronicotinate (1.0 g, 4.57 mmol) were taken up into a 20 mL microwave tube, and microwaved at 105°C for 2 h. After the reaction was complete, the reaction solution was poured into water (25 mL), added with ethyl acetate (15 mL). The organic phase was washed with water, saturated aqueous sodium bicarbonate solution and saturated brine, dried over $Na_2SO_4$, concentrated and then purified by FCC ($SiO_2$, THF/DCM = 0-40%) to give ethyl 6-chloro-4-(2-cyanoacetamido)-5-fluoronicotinate (550 mg, yield 42%) as a yellow solid. LCMS (m/z): 286.0 (M + H).

**Step D:** 7-Chloro-8-fluoro-2,4-dihydroxy-1,6-naphthyridine-3-carbonitrile

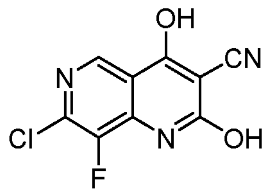

Ethyl 6-chloro-4-(2-cyanoacetamido)-5-fluoronicotinate (300 mg, 1.05 mmol) was dissolved in THF (10 mL), and stirred for 2 min in an ice bath before potassium tert-butoxide (236 mg, 2.10 mmol) was slowly added. The resulting mixture was stirred for 1 hour, with the ice bath gradually recovered to room temperature. LCMS indicated the reaction was complete. The reaction solution was added dropwise into a stirred saturated aqueous $NH_4Cl$ (50 mL) solution, and adjusted to pH about 7 with HCl (1 M), during which a white flocculent precipitate formed and was collected by filtration. The resulting solid was washed with water (10 mL) and petroleum ether (10 mL) and dried under vacuum to give 7-chloro-8-fluoro-2,4-dihydroxy-1,6-naphthyridine-3-carbonitrile (200 mg, yield 79%) as a white solid. LCMS (m/z): 240.1 (M + H).

**Step E:** 2,4,7-Trichloro-8-fluoro-1,6-naphthyridine-3-carbonitrile

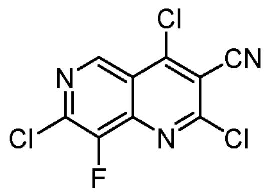

7-Chloro-8-fluoro-2,4-dihydroxy-1,6-naphthyridine-3-carbonitrile (200 mg, 0.83 mmol) was dissolved in $POCl_3$ (15 mL), heated at 120°C and stirred for overnight. TLC showed the reaction was complete. The reaction was cooled to room temperature, concentrated in vacuo to remove the most of phosphorus oxychloride, and acetonitrile (1 mL) was added. The resulting solution in acetonitrile was poured into saturated aqueous $NaHCO_3$ solution (30 mL), forming a yellow solid. The solid was filtered, collected and washed with water (10 mL) and petroleum ether (10 mL) respectively. Drying under vacuum affords 2,4,7-trichloro-8-fluoro-1,6-naphthyridine-3-carbonitrile (190 mg, yield 82%) as a yellow solid.

**Synthesis of intermediate Int E1**

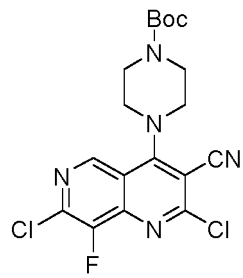

*tert*-Butyl 4-(2,7-dichloro-3 -cyano-8-fluoro-1,6-naphthyridin-4-yl)piperazine-1 -carboxylate

Int E1 was synthesized by referring to the procedure described in intermediate Int B1, using 2,4,7-trichloro-8-fluoro-1,6-naphthyridine-3-carbonitrile (intermediate Int D) in place of 7-bromo-2,4,6-trichloro-8-fluoroquinoline-3-carbonitrile (intermediate Int A) and using *tert*-butyl piperazine-1-carboxylate in place of *tert*-butyl (R)-2-methylpiperazine-1-carboxylate. LCMS (m/z): 426.3 (M + H).

**Synthesis of intermediate Int F**

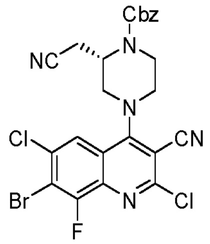

Benzyl (*S*)-4-(7-bromo-2,6-dichloro-3-cyano-8-fluoroquinolin-4-yl)-2-(cyanomethyl)piperazine-1-carboxylate

Int F was synthesized by referring to the procedure described for intermediate Int B1, using benzyl (*S*)-2-(cyanomethyl)piperazine-1-carboxylate in place of *tert*-butyl (*R*)-2-methylpiperazine-1-carboxylate. LCMS (m/z): 576.3, 578.3 (M + H).

**Example 1**

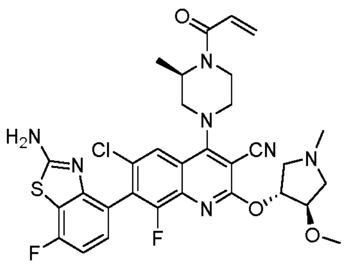

4-((*R*)-4-acryloyl-3-methylpiperazin-1-yl)-7-(2-amino-7-fluorobenzo[*d*]thiazol-4-yl)-6-chloro-8-fluoro-2-(((3*R*,4*R*)-4-methoxy-1-methylpyrrolidin-3-yl)oxy)quinoline-3-carbonitrile

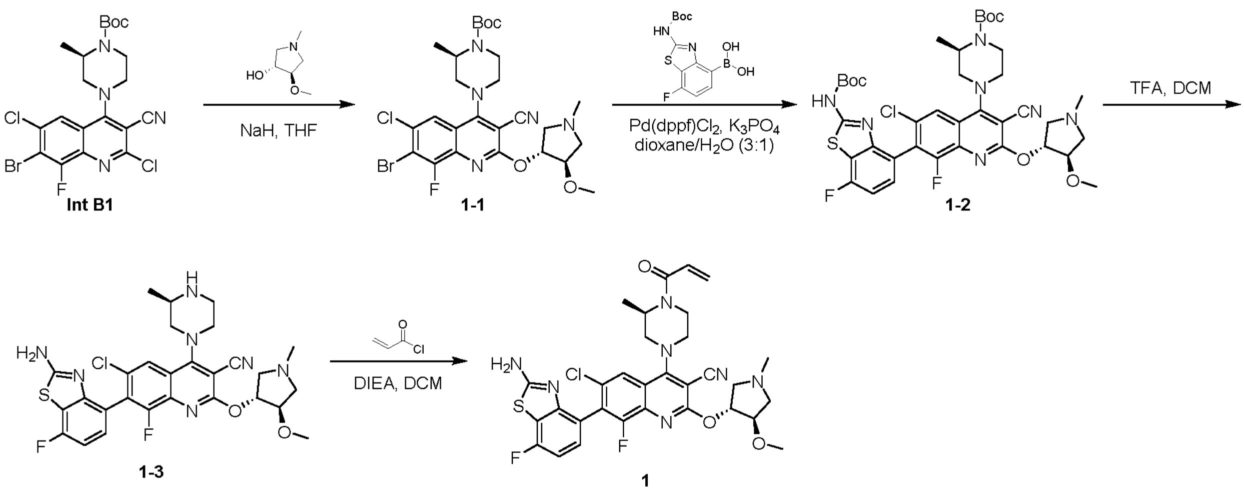

**Step A:** *tert*-Butyl (R)-4-(7-bromo-6-chloro-3-cyano-8-fluoro-2-(((3*R*,4*R*)-4-methoxy-1-methylpyrrolidin-3-yl)oxy)quinolin-4-yl)-2-methylpiperazine-1-carboxylate

NaH (60% w/w, 9.3 mg, 0.23mmol) was added to a stirred solution of (3*R*,4*R*)-4-methoxy-1-methylpyrrolidin-3-ol (27.8mg, 0.21mmol ) in THF (2 mL) at r.t., and the resulting mixture was stirred at room temperature for 10 min. *tert*-Butyl (*R*)-4-(7-bromo-2,6-dichloro-3-cyano-8-fluoroquinolin-4-yl)-2-methylpiperazine-1-carboxylate (Intermediate B1, 110mg, 0.21mmol) was added in one portion, and stirred at room temperature for 1 h. LCMS indicated Intermediate B1 was not completely reacted. Additional pre-stirred (5 min) solution of NaH (60% w/w, 5 mg, 0.12 mmol) and (3*R*,4*R*)-4-methoxy-1-methylpyrrolidin-3-ol (10 mg, 0.78 mmol) in THF (1 mL) was added. The resulting mixture was stirred for additional 30 min, and LCMS showed the reaction was complete. The reaction was quenched with saturated aqueous $NH_4Cl$ (2 mL), diluted with EA (30 mL). The resulting organic phase was washed with saturated NaCl (20 mL×3), dried over anhydrous $Na_2SO_4$, filtered, and concentrated to afford the crude *tert*-butyl (*R*)-4-(7-bromo-6-chloro-3-cyano-8-fluoro-2-(((3*R*,4*R*)-4-methoxy-1-methylpyrrolidin-3-yl)oxy)quinolin-4-yl)-2-methylpiperazine-1-carboxylate (130 mg, crude product) which was used directly in the next step. LCMS (m/z): 612.4 (M+H).

**Step B:** *tert*-Butyl (2*R*)-4-(7-(2-((*tert*-butoxycarbonyl)amino)-7-fluorobenzo[d]thiazol-4-yl)-6-chloro-3-cyano-8-fluoro-2-(((3*R*,4*R*)-4-methoxy-1-methylpyrrolidin-3-yl)oxy)quinolin-4-yl)-2-methylpiperazine-1-carboxylate

At room temperature, a microwave tube equipped with magnetic stirring bar was charged with *tert*-butyl (*R*)-4-(7-bromo-6-chloro-3-cyano-8-fluoro-2-(((3R,4R)-4-methoxy-1-methylpyrrolidin-3-yl)oxy)quinolin-4-yl)-2-methylpiperazine-1-carboxylate (170 mg, 0.28mmol), (2-((*tert*-butoxycarbonyl)amino)-7-fluorobenzo[d]thiazol-4-yl)boronic acid (112mg, 0.36mmol), Pd(dppf)$Cl_2$ (24 mg, 0.03mmol) and $K_3PO_4$ (104 mg, 0.49 mmol) in 1,4-dioxane/$H_2O$ (v/v=3:1, 1.6mL). The system was bubbled with nitrogen for 10 min, the reaction tube was sealed and heated at 100°C for 1 h under microwave. The reaction was cooled to room temperature, diluted with EA (20mL), washed with saturated aqueous NaCl solution (20mL×3), dried over anhydrous $Na_2SO_4$, and concentrated under reduced pressure. The residue was preliminarily purified by FCC ($SiO_2$, MeOH/DCM=0-20%) to afford *tert*-butyl (2*R*)-4-(7-(2-((tert-butoxycarbonyl)amino)-7-fluorobenzo[d]thiazol-4-yl)-6-chloro-3-cyano-8-fluoro-2-(((3*R*,4*R*)-4-methoxy-1-methylpyrrolidin-3-yl)oxy)quinolin-4-yl)-2-methylpiperazine-1-carboxylate (150mg, 62% purity), which was used directly in next step. LCMS (m/z): 800.6 (M+H).

**Step C:** 7-(2-Amino-7-fluorobenzo[d]thiazol-4-yl)-6-chloro-8-fluoro-2-(((3*R*,4*R*)-4-methoxy-1-methylpyrrolidin-3-yl)oxy)-4-((R)-3-methylpiperazin-1-yl) quinoline-3-carbonitrile

TFA (1 mL) was added dropwise to a stirred solution of tert-butyl (2*R*)-4-(7-(2-((*tert*-butoxycarbonyl)amino)-7-fluorobenzo [d]thiazol-4-yl)-6-chloro-3-cyano-8-fluoro-2-(((3*R*,4*R*)-4-methoxy-1-methylpyrrolidin-3-yl)oxy)quinolin-4-yl)-2-methylpiperazine-1-carboxylate (150 mg, 62% purity) in DCM (2 mL) at room temperature. The resulting mixture was stirred at room temperature for 30 min, and LCMS showed the reaction was complete. The reaction was concentrated under reduced pressure to remove the solvent TFA. A small amount of DCM was added and concentrated under reduced pressure again, which was repeated three times to remove the residual TFA, and the obtained crude product was directly used in the next step without purification.

**Step D:** 4-((*R*)-4-acryloyl-3-methylpiperazin-1-yl)-7-(2-amino-7-fluorobenzo[d]thiazol-4-yl)-6-chloro-8-fluoro-2-(((3*R*,4*R*)-4-methoxy-1-methylpyrrolidin-3-yl)oxy)quinoline-3-carbonitrile

The crude 7-(2-Amino-7-fluorobenzo[d]thiazol-4-yl)-6-chloro-8-fluoro-2-(((3*R*,4*R*)-4-methoxy-1-methylpyrrolidin-3-yl)oxy)-4-((R)-3-methylpiperazin-1-yl)quinoline-3-carbonitrile from Step C was dissolved in DCM (2 mL), cooled with ice-water bath, added with DIEA (103 uL, 0.62 mmol), and stirred for 5 mins before a solution of acryloyl chloride (10 uL, 0.12 mmol) in DCM (0.1 mL) was slowly added dropwise. After the addition was complete, the reacyion was stirred under an ice-water bath for additional 20 min. LCMS indicated the reaction was complete. $H_2O$ (5 mL) and EA (30 mL) were added to the reaction, the aqueous phase was removed by separation, and the organic phase was washed with a saturated aqueous NaCl solution (20 mLx3), dried over anhydrous $Na_2SO_4$, and concentrated. The crude was purified by preparative HPLC to give 4-((*R*)-4-acryloyl-3-methylpiperazin-1-yl)-7-(2-amino-7-fluorobenzo [*d*] thiazol-4-yl)-6-chloro-8-fluoro-2-(((3*R*,4*R*)-4-methoxy-1-methylpyrrolidin-3-yl)oxy)quinoline-3-carbonitrile (15.2 mg, 7.5% overall yield over four steps) as a white solid. $^1H$ NMR (400 MHz, Methanol-$d_4$) δ 8.12 - 7.99 (m, 1H), 7.33 - 7.22 (m, 1H), 7.07 - 6.97 (m, 1H), 6.87 (dd, *J* = 16.7, 10.7 Hz, $^1H$ ), 6.30 (d, *J* = 16.8 Hz, 1H), 5.83 (d, *J* = 10.7 Hz, 1H), 5.65 (s, 1H), 4.24 - 4.20 (m, 1H), 4.09 - 3.89 (m, 2H), 3.83 - 3.77 (m, 1H), 3.62-3.48 (m, 5H), 3.43 - 3.38 (m, 2H), 3.26 - 3.18 (m, 2H), 3.09 - 3.03 (m, 1H), 2.98 - 2.92 ( m, 1H), 2.60 (s, 3H), 1.46 (d, *J* = 6.8 Hz, 3H). $^{19}F$ NMR (376 MHz, Methanol-$d_4$) δ -114.94, -120.48. LCMS(m/z): 654.5 (M+H).

**Example 2**

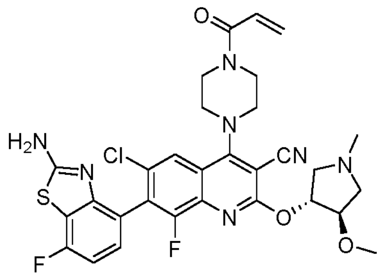

4-(4-Acryloylpiperazin-1-yl)-7-(2-amino-7-fluorobenzo[*d*]thiazol-4-yl)-6-chloro-8-fluoro-2-(((3*R*,4*R*)-4-methoxy-1-methylpyrrolidin-3-yl)oxy)quinoline-3-carbonitrile

Example 2 was synthesized by referring to the procedure described in Example 1, using *tert*-butyl 4-(7-bromo-2,6-dichloro-3-cyano-8-fluoroquinolin-4-yl)piperazine-1-carboxylate (Intermediate B2) in place of *tert*-butyl (*R*)-4-(7-bromo-2,6-dichloro-3-cyano-8-fluoroquinolin-4-yl)-2-methylpiperazine-1-carboxylate (Intermediate B1) in step A. $^1H$ NMR (400 MHz, DMSO-$d_6$) δ 8.02 - 7.87 (m, 3H), 7.24 (dt, *J* = 8.4, 5.1 Hz, 1H), 7.07 (t, *J* = 8.8 Hz, 1H), 6.90 (dd, *J* = 16.7, 10.5 Hz, 1H), 6.19 (dd, *J* = 16.7, 2.4 Hz, 1H), 5.75 (dd, *J* = 10.4, 2.4 Hz, 1H), 5.39 - 5.31 (m, 1H), 4.06 - 3.96 (m, 1H), 3.94-3.81 (m, 4H), 3.75 - 3.61 (m, 4H), 3.32 (s, 3H), 3.08 - 3.00 (m, 1H), 2.92 (dd, *J* = 11.0, 6.2 Hz, 1H), 2.73 - 2.64 (m, 1H), 2.33 (dt, *J* = 9.3, 4.3 Hz, 1H), 2.26 - 2.21 (m, 3H). $^{19}F$ NMR (376 MHz, DMSO-$d_6$) δ -112.27 (d, *J* = 4.4 Hz), -119.59 (d, *J* = 22.4 Hz). LCMS (m/z): 640.5 (M+H).

**Example 3**

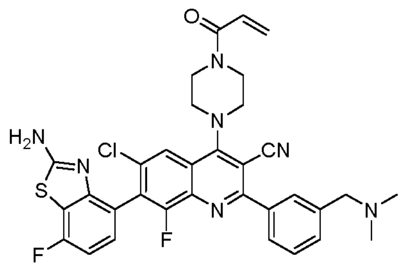

4-(4-Acryloylpiperazin-1-yl)-7-(2-amino-7-fluorobenzo[*d*]thiazol-4-yl)-6-chloro-2-(3-((dimethylamino)methyl)phenyl)-8-fluoroquinoline-3-carbonitrile

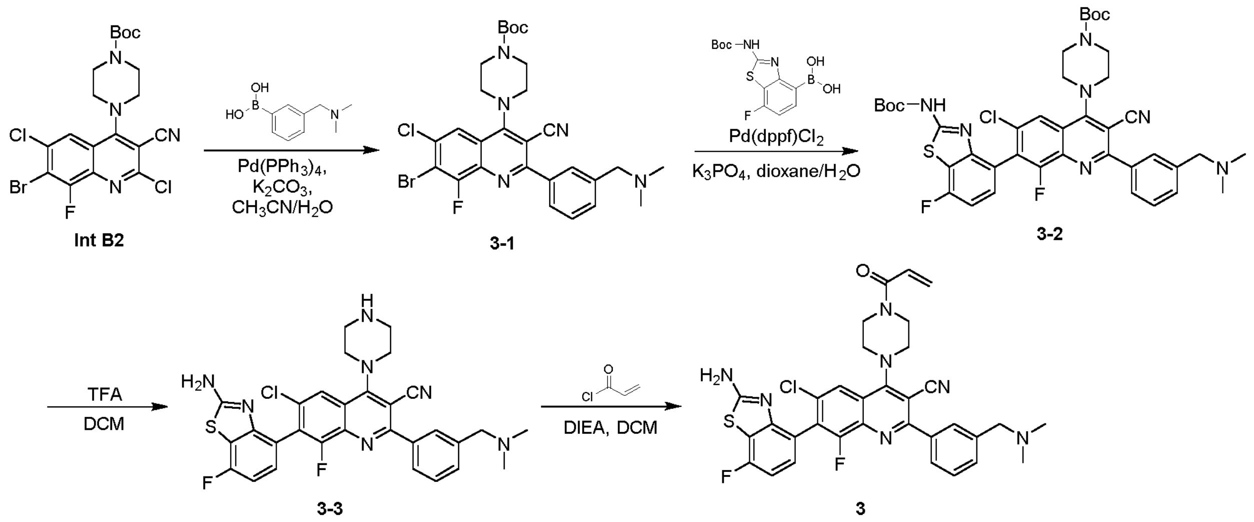


Int B2 3-1 3-2 3-3 3

**Step A:** *tert*-Butyl 4-(7-bromo-6-chloro-3-cyano-2-(3-((dimethylamino)methyl)phenyl)-8-fluoroquinolin-4-yl)piperazine-1-carboxylate

A microwave tube was charged with *tert*-butyl 4-(7-bromo-2,6-dichloro-3-cyano-8-fluoroquinolin-4-yl)piperazine-1-carboxylate (Intermediate B2, 300 mg, 0.60mmol), (3-((dimethylamino)methyl)phenyl)boronic acid (108mg, 0.60mmol), $Pd(PPh_3)_4$ (35mg, 0.03mmol), $K_2CO_3$ (166 mg, 1.2mmol), acetonitrile (3 mL) and water (1mL). The mixture was degassed and refilled with nitrogen for three times, and heated at 40°C for 1 h under microwave. The reaction was cooled to room temperature, concentrated, and the crude was purified by FCC ($SiO_2$, EA/PE=0-60%) to afford *tert*-butyl 4-(7-bromo-6-chloro-3-cyano-2-(3-((dimethylamino)methyl)phenyl)-8-fluoroquinolin-4-yl)piperazine-1-carboxylate (260 mg, yield 72%) as a yellow solid. LCMS (m/z): 602.4 (M+H).

**Step B to Step D:** 4-(4-Acryloylpiperazin-1-yl)-7-(2-amino-7-fluorobenzo[*d*]thiazol-4-yl)-6-chloro-2-(3-((dimethylamino)methyl)phenyl)-8-fluoroquinoline-3-carbonitrile

The subsequent synthesis of Example 3 was carried out by referring to the procedures described in Example 1, using *tert*-butyl 4-(7-bromo-6-chloro-3-cyano-2-(3-((dimethylamino)methyl)phenyl)-8-fluoroquinolin-4-yl)piperazine-1-carboxylate in place of *tert*-butyl (*R*)-4-(7-bromo-6-chloro-3-cyano-8-fluoro-2-(((3*R*,4*R*)-4-methoxy-1-methylpyrrolidin-3-yl)oxy)quinolin-4-yl)-2-methylpiperazine-1-carboxylate in step B. $^1H$ NMR (400 MHz, DMSO-$d_6$) $\delta$ 8.11 - 8.10 (m, 1H), 7.92 (s, 2H), 7.81 - 7.70 (m, 2H), 7.53 - 7.46 (m, 2H), 7.31 - 7.27 (m, 1H), 7.11 - 7.09 (m, 1H), 6.91 (dd, *J* = 16.6, 10.4 Hz, 1H), 6.22 - 6.17 (m, 1H), 5.77 - 5.74 (m, 1H), 3.98 - 9.84 (m, 4H), 3.83 - 3.70 (m, 4H), 3.51 - 3.46 (m, 2H), 2.24 - 2.13 (m, 6H). LCMS(m/z): 645.5 (M+H).

**Example 4**

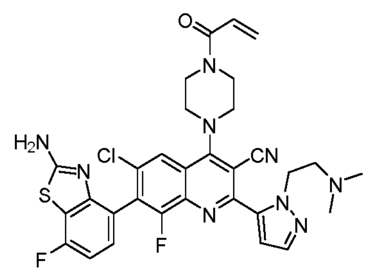

4-(4-Acryloylpiperazin-1-yl)-7-(2-amino-7-fluorobenzo[*d*]thiazol-4-yl)-6-chloro-2-(1-(2-(dimethylaminoH-pyrazol-5-yl)-8-fluoroquinoline-3-carbonitrile

Example 4 was synthesized by referring to the procedure described in Example 3, using (1-(2-(dimethylamino)ethyl)-1H-pyrazol-5-yl)boronic acid in place of (3-((dimethylamino)methyl)phenyl)boronic acid in Step A. $^1$H NMR (400 MHz, $CDCl_3$) δ 7.96 (s, 1H), 7.63 (s, 1H), 7.40 - 7.30 (m, 1H), 7.01 - 6.93 (m, 2H), 6.68-6.59 (m, 1H), 6.39 (d, *J* = 16.8 Hz, 1H), 6.26 (s, 2H), 5.80 (d, *J* = 10.6 Hz, 1H), 4.66 (t, *J* = 7.5 Hz, 2H), 4.04 - 3.71 (m, 8H), 3.39 (s, 1H), 2.98 (s, 1H), 2.38 (s, 6H). $^{19}$F NMR (376 MHz, $CDCl_3$) δ -111.07, -115.38. LCMS(m/z): 648.5 (M+H).

**Example 5**

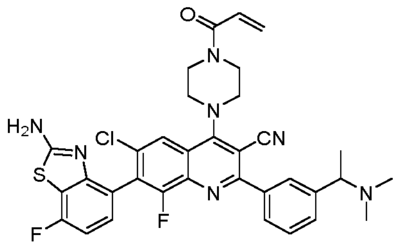

4-(4-Acryloylpiperazin-1-yl)-7-(2-amino-7-fluorobenzo[*d*]thiazol-4-yl)-6-chloro-2-(3-(1-(dimethylamino)ethyl)phenyl)-8-fluoroquinoline-3-carbonitrile

Example 5 was synthesized by referring to the procedure described in Example 3, using (3-(1-(dimethylamino)ethyl)phenyl)boronic acid in place of (3-((dimethylamino)methyl)phenyl)boronic acid in Step A. $^1$H NMR (400 MHz, MeOH-$d_4$) δ 8.16 (d, *J* = 1.6 Hz, 1H), 7.87 (s, 1H), 7.84 - 7.78 (m, 1H), 7.61 - 7.52 (m, 2H), 7.30 (dd, *J* = 8.5, 5.4 Hz, 1H), 7.09 - 6.99 (m, 1H), 6.89 (dd, *J* = 16.8, 10.6 Hz, 1H), 6.31 (dd, *J* = 16.7, 1.9 Hz, 1H), 5.84 (dd, *J* = 10.6, 1.9 Hz, 1H), 4.12 - 4.00 (m, 4H), 3.97 - 3.84 (m, 4H), 2.30 (s, 6H), 1.57 - 1.45 (m, 3H). $^{19}$F NMR (376 MHz, MeOH-$d_4$) δ -114.82,-118.65 (d, *J* = 9.3 Hz). LCMS (m/z): 658.5 (M+H).

**Example 6**

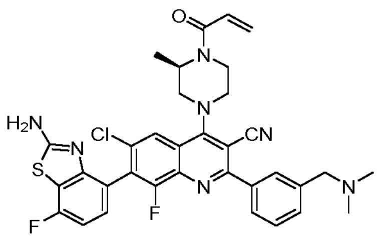

4-((*R*)-4-acryloyl-3-methylpiperazin-1-yl)-7-(2-amino-7-fluorobenzo[*d*]thiazol-4-yl)-6-chloro-2-(3-((dimethylamino)methyl)phenyl)-8-fluoroquinoline-3-carbonitrile

Example 6 was synthesized by referring to the procedure described for Example 3, using *tert*-Butyl (*R*)-4-(7-bromo-2,6-dichloro-3-cyano-8-fluoroquinolin-4-yl)-2-methylpiperazine-1-carboxylate (intermediate Int B1) in place of *tert*-butyl 4-(7-bromo-2,6-dichloro-3-cyano-8-fluoroquinolin-4-yl)piperazine-1-carboxylate (intermediate Int B2) in step A. $^1$H NMR (400 MHz, DMSO-$d_6$) δ 8.14 (d, *J* = 3.3 Hz, 1H), 7.92 (d, *J* = 19.9 Hz, 2H), 7.82 (s, 1H), 7.77 (d, J = 7.6 Hz , 1H), 7.56 - 7.46 (m, 2H), 7.33 - 7.27 (m, 1H), 7.13 - 7.06 (m, 1H), 6.94 - 6.84 (m, 1H), 6.23 - 6.14 (m, 1H), 5.78 - 5.72 (m, 1H), 4.95 -4.23 (m, 4H), 4.08 (t, *J* = 12.7 Hz, 1H), 4.00 - 3.90 (m, 1H), 3.77 (t, *J* = 12.7 Hz, 1H), 3.53 (s, 2H), 2.21 (s, 6H), 1.36 - 1.27 (m, 3H). LCMS (m/z): 658.5 (M+H).

**Example 7**

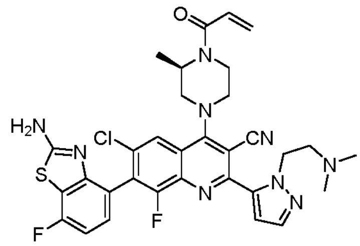

4-((*R*)-4-acryloyl-3-methylpiperazin-1-yl)-7-(2-amino-7-fluorobenzo[*d*]thiazol-4-yl)-6-chloro-2-(1-(2-(dimethylamino)ethyl)-1*H*-pyrazol-5-yl)-8-fluoroquinoline-3-carbonitrile

Example 6 was synthesized by referring to the procedure described for Example 3, using *tert*-butyl (*R*)-4-(7-bromo-2,6-dichloro-3-cyano-8-fluoroquinolin-4-yl)-2-methylpiperazine-1-carboxylate (intermediate Int B1) in place of *tert*-butyl 4-(7-bromo-2,6-dichloro-3-cyano-8-fluoroquinolin-4-yl)piperazine-1-carboxylate (intermediate Int B2), and using (1-(2-(dimethylamino)ethyl)-1H-pyrazol-5-yl)boronic acid in place of (3-((dimethylamino)methyl)phenyl)boronic acid in Step A. $^1$H NMR (400 MHz, DMSO-$d_6$) $\delta$ 8.14 (s, 1H), 7.90 (d, *J* = 11.8 Hz, 2H), 7.62 (d, *J* = 1.9 Hz, 1H), 7.29 (dd, *J* = 8.6, 5.3 Hz, 1H), 7.09 (t, *J* = 8.8 Hz, 1H), 6.94 - 6.83 (m, 2H), 6.17 (d, J = 16.7 Hz, 1H), 5.79 - 5.70 (m, 1H), 4.62 - 4.32 (m, 4H), 4.11 - 3.92 (m, 3H), 3.81 - 3.66 (m, 2H), 3.55 (s, 2H), 2.10-1.83 (m, 6H), 1.34 - 1.20 (m, 3H). LCMS (m/z): 662.5, (M+H).

**Example 8**

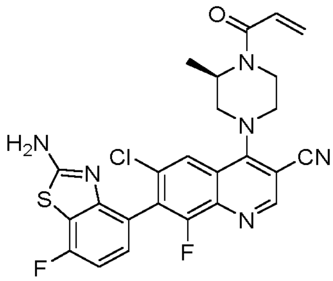

4-((*R*)-4-acryloyl-3-methylpiperazin-1-yl)-7-(2-amino-7-fluorobenzo[d]thiazol-4-yl)-6-chloro-8-fluoroquinoline-3-carbonitrile

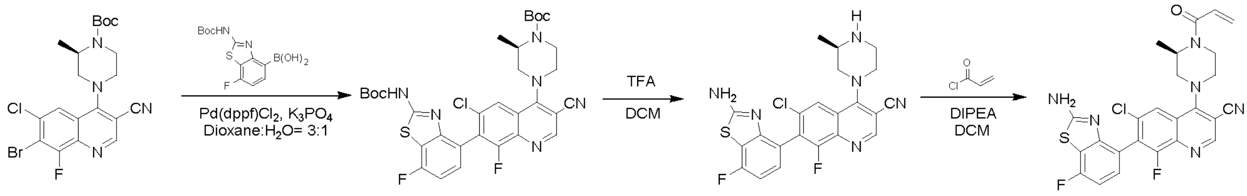


Int C1 8-1 8-2 8

**Step A:** *tert*-Butyl (2*R*)-4-(7-(2-((tert-butoxycarbonyl)amino)-7-fluorobenzo[*d*]thiazol-4-yl)-6-chloro-3-cyano-8-fluoroquinolin-4-yl)-2-methylpiperazine-1-carboxylate

A microwave tube was charged with *tert*-butyl (*R*)-4-(7-bromo-6-chloro-3-cyano-8-fluoroquinolin-4-yl)-2-methylpiperazine-1-carboxylate (Intermediate C1, 120 mg, 0.25mmol), (2-((*tert*-butoxycarbonyl)amino)-7-fluorobenzo[*d*]thiazol-4-yl)boronic acid (232 mg, 0.75mmol), Pd(dppf)$Cl_2$ (18.3 mg, 25$\mu$mol) and $K_3PO_4$ (211 mg, 1.0mmol), degassed and refilled with nitrogen for three times, added with 1,4-dioxane/water (v/v=3:1, 4ml), and the tube was placed into a microwave reactor, heated to 90°C and allowed to react for 1 h. After the reaction was complete, the system was cooled to room temperature, the solvent was dried by rotary evaporator, and the crude was purified by FCC ($SiO_2$, EA/PE=0-80%) to afford *tert*-butyl (2*R*)-4-(7-(2-((tert-butoxycarbonyl)amino)-7-fluorobenzo[*d*]thiazol-4-yl)-6-chloro-3-cyano-8-fluoroquinolin-4-yl)-2-methylpiperazine-1-carboxylate (92 mg, yield 47%) as a yellow solid. LCMS (m/z): 671.5 (M+H).

**Step B:** 7-(2-Amino-7-fluorobenzo[*d*]thiazol-4-yl)-6-chloro-8-fluoro-4-((*R*)-3-methylpiperazin-1-yl)quinoline-3-carbonitrile

TFA (2 mL) was added dropwise to a stirred solution of tert-butyl (2*R*)-4-(7-(2-((tert-butoxycarbonyl)amino)-7-fluorobenzo[*d*]thiazol-4-yl)-6-chloro-3-cyano-8-fluoroquinolin-4-yl)-2-methylpiperazine-1-carboxylate (92 mg, 0.14 mmol) in DCM (1 ml) at room temperature. The reaction was stirred for 1 h at r.t. and LCMS indicated the reaction was complete. The solvent was dried by rotary evaporator, added with a small amount of DCM and concentrated again. The procedure was repeated three times to remove the residual TFA to afford 7-(2-amino-7-fluorobenzo[*d*]thiazol-4-yl)-6-chloro-8-fluoro-4-((*R*)-3-methylpiperazin-1-yl)quinoline-3-carbonitrile, which was used directly in the next step. LCMS (m/z): 471.3 (M+H).

**Step C**: 4-((*R*)-4-acryloyl-3-methylpiperazin-1-yl)-7-(2-amino-7-fluorobenzo[d]thiazol-4-yl)-6-chloro-8-fluoroquinoline-3-carbonitrile

To a round bottom flask equipped with magnetic stirring bar was charged 7-(2-amino-7-fluorobenzo [d] thiazol-4-yl)-6-chloro-8-fluoro-4-((*R*)-3-methylpiperazine-1-yl)quinoline-3-carbonitrile (crude from Step B) and DCM (3ml). The reaction was cooled to 0°C in an ice-water bath, added with DIPEA (2m1), and then a solution of acryloyl chloride (11 mg, 0.12mmol) in DCM (1.0mL) was added dropwise. After the addition was complete, the reaction was stirred for additional 30 min in the ice-water bath. After the reaction was complete, the reaction was quenched with water (5 mL), extracted with DCM (10 mL×3), the combined organic phases were washed with saturated NaCl (10 mL×3), and dried over anhydrous $Na_2SO_4$. The reaction was concentrated by rotary evaporation, and purified by preparative HPLC to afford 4-((*R*)-4-acryloyl-3-methylpiperazin-1-yl)-7-(2-amino-7-fluorobenzo[*d*]thiazol-4-yl)-6-chloro-8-fluoroquinoline-3-carbonitrile (26 mg, yield 35%) as a white solid. $^1$H NMR (400 MHz, DMSO-$d_6$) δ 8.88 (s, 1H), 8.08 (dd, *J* = 4.3, 1.4 Hz, 1H), 7.92 (s, 2H), 7.38 - 7.24 (m, 1H), 7.12 - 7.04 (m, 1H), 6.86 (dd, *J* = 16.6, 10.5 Hz, 1H), 6.17 (dd, *J* = 16.6, 2.4 Hz, 1H), 5.81 - 5.68 (m, 1H), 4.47 (d, *J* = 125.4 Hz, 3H), 3.94 (t, *J* = 12.8 Hz, 1H), 3.87 - 3.64 (m, 2H), 3.56 - 3.41 (m, 1H), 1.49 - 1.19 (m, 3H). LCMS (m/z): 525.3 (M+H).

**Example 9**

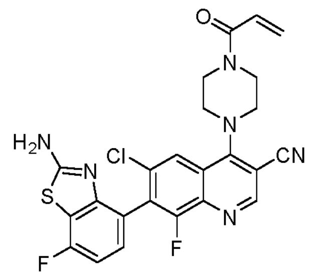

4-(4-Acryloylpiperazin-1-yl)-7-(2-amino-7-fluorobenzo[*d*]thiazol-4-yl)-6-chloro-8-fluoroquinoline-3-carbonitrile

Example 9 was synthesized by referring to the procedure described in Example 8, using *tert*-butyl 4-(7-bromo-6-chloro-3-cyano-8-fluoroquinolin-4-yl)piperazine-1-carboxylate (Intermediate Int C2) in place of *tert*-butyl (R)-4-(7-bromo-6-chloro-3-cyano-8-fluoroquinolin-4-yl)-2-methylpiperazine-1-carboxylate (Intermediate Int Cl) in step A. $^1$H NMR (400 MHz, Methanol-$d_4$) δ 8.78 (s, 1H), 8.10 (d, *J* = 1.8 Hz, 1H), 7.26 (dd, *J* = 8.4, 5.4 Hz, 1H), 7.06 - 6.97 (m, 1H), 6.87 (dd, *J* = 16.8, 10.7 Hz, 1H), 6.30 (dd, *J* = 16.8, 1.9 Hz, 1H), 5.83 (dd, *J* = 10.6, 1.9 Hz, 1H), 4.05 - 3.95 (m, 4H), 3.90-3.78 (m, 4H). $^{19}$F NMR (376 MHz, Methanol-$d_4$) δ -114.73, -119.40. LCMS (m/z): 511.3 (M+H).

**Example 10**

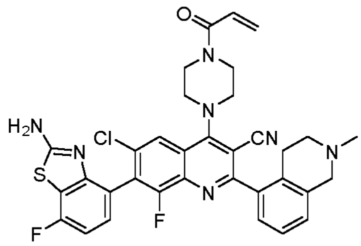

4-(4-Acryloylpiperazin-1-yl)-7-(2-amino-7-fluorobenzo[*d*]thiazol-4-yl)-6-chloro-8-fluoro-2-(2-methyl-1,2,3,4-tetrahydroisoquinolin-5-yl)quinoline-3-carbonitrile

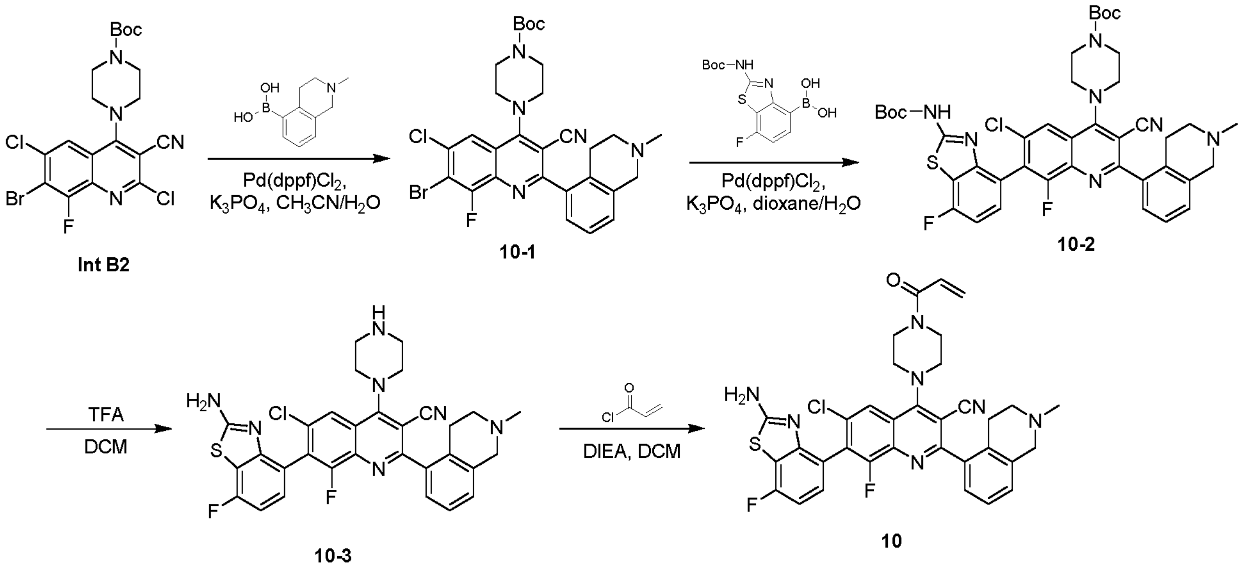


**Step A:** *tert*-Butyl 4-(7-bromo-6-chloro-3-cyano-8-fluoro-2-(2-methyl-1,2,3,4-tetrahydroisoquinolin-5-yl)quinolin-4-yl)piperazine-1-carboxylate

To a microwave tube equipped with a magnetic stirring bar was added *tert*-butyl 4-(7-bromo-2,6-dichloro-3-cyano-8-fluoroquinolin-4-yl)piperazine-1-carboxylate (Intermediate B2, 150 mg, 0.3mmol), (2-methyl-1,2,3,4-tetrahydroisoquinolin-5-yl)boronic acid (243 mg, 0.9mmol), $K_3PO_4$ (189 mg, 0.9mmol) in $CH_3CN/H_2O$ (v/v=3:1, 2mL). The reaction was purged with nitrogen for 5 min, added with Pd(dppf)$Cl_2$ (22 mg, 0.03 mmol), and purged with nitrogen for additional 5 min. The reaction was heated to 45°C for 4 hours under microwave and cooled to room temperature then. The reaction was added with EA (10 mL) and $H_2O$ (5 mL), and allowed to separate. The aqueous phase was extracted with EA (10 mL×2). the combined organic phases were washed with saturated NaCl (30mL), dried over anhydrous $Na_2SO_4$, filtered, and the filtrate was concentrated under reduced pressure to afford a crude which was purified by FCC ($SiO_2$, EA/PE=0-100%) to afford *tert*-butyl 4-(7-bromo-6-chloro-3-cyano-8-fluoro-2-(2-methyl-1,2,3,4-tetrahydroisoquinolin-5-yl)quinolin-4-yl)piperazine-1-carboxylate (80 mg, yield 44%) as a brown solid. LCMS (m/z): 614.4 (M+H).

**Step B to Step D:** 4-(4-Acryloylpiperazine-1-yl)-7-(2-amino-7-fluorobenzo[*d*]thiazol-4-yl)-6-chloro-8-fluoro-2-(2-methyl-1,2,3,4-tetrahydroisoquinoline-5-yl)quinoline-3-carbonitrile

The subsequent synthesis of Example 10 was carried out by referring to the procedures described for Example 1, using *tert*-butyl 4-(7-bromo-6-chloro-3-cyano-8-fluoro-2-(2-methyl-1,2,3,4-tetrahydroisoquinolin-5-yl)quinolin-4-yl)piperazine-1-carboxylate in place of *tert*-butyl (*R*)-4-(7-bromo-6-chloro-3-cyano-8-fluoro-2-(((3*R*,4*R*)-4-methoxy-1-methylpyrrolidin-3-yl)oxy)quinolin-4-yl)-2-methylpiperazine-1-carboxylate in step B. $^1$H NMR (400 MHz, Methanol-$d_4$) $\delta$ 8.15 (s, 1H), 7.36 - 7.22 (m, 4H), 7.01 (t, *J* = 8.8 Hz, 1H), 6.86 (dd, *J* = 16.8, 10.6 Hz, 1H), 6.29 (dd, *J* = 16.7, 1.9 Hz, 1H), 5.82 (dd, *J* = 10.5, 1.9 Hz, 1H), 4.57 (s, 2H), 3.99 (s, 4H), 3.85 (s, 4H ), 3.74 (s, 2H), 2.84 (d, *J* = 5.7 Hz, 1H), 2.73 (t, *J* = 5.9 Hz, 1H), 2.45 (s, 3H). LCMS(m/z): 654.5 (M +H).

**Example 11**

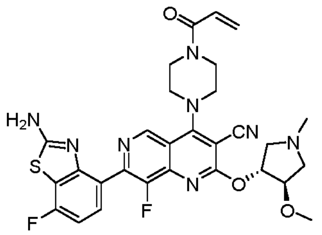

4-(4-Acryloylpiperazin-1-yl)-7-(2-amino-7-fluorobenzo [d] thiazol-4-yl)-8-fluoro-2-(((3*R*,4*R*)-4-methoxy-1 -methylpyrrolidin-3-yl)oxy)-1 ,6-naphthyridine-3-carbonitrile

Example 11 was synthesized by referring to the procedure described in Example 1, using *tert*-butyl 4-(2,7-dichloro-3-cyano-8-fluoro-1,6-naphthyridin-4-yl)piperazine-1-carboxylate (Intermediate E1) in place of *tert*-butyl (*R*)-4-(7-bromo-2,6-dichloro-3-cyano-8-fluoroquinolin-4-yl)-2-methylpiperazine-1-carboxylate (Intermediate B1) in Step A. $^{1}H$ NMR (400 MHz, DMSO-$d_6$) $\delta$ 9.14 (s, 1H), 7.95 (s, 2H), 7.44 (dd, *J* = 8.5, 5.8 Hz, 1H), 7.13 - 7.04 (m, 1H), 6.91 (dd, *J* = 16.6, 10.4 Hz, 1H), 6.19 (dd, *J* = 16.7, 2.4 Hz, 1H), 5.75 (dd, *J* = 10.4, 2.4 Hz, 1H), 5.47 - 5.39 (m, 1H), 4.08 - 3.99 (m, 1H), 3.89 (s, 2H), 3.83 (s, 6H), 3.34 (s, 3H), 3.08 (dd, *J* = 10.0, 6.4 Hz, 1H), 2.98 (dd, *J* = 11.1 , 6.2 Hz, 1H), 2.75 (dd, *J* = 11.3, 2.8 Hz, 1H), 2.40 (dd, *J* = 10.0, 4.9 Hz, 1H), 2.29 (s, 3H). $^{19}F$ NMR (376 MHz, DMSO-$d_6$) $\delta$ -111.71, -135.46. LCMS (m/z): 607.5 (M+H).

**Example 12**

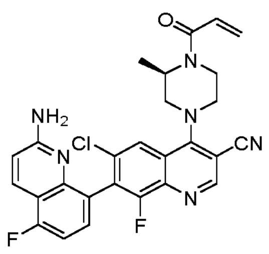

4-((*R*)-4-acryloyl-3-methylphenylpropylamine-1-yl)-2'-amino-6-chloro-5',8-difluoro-[7,8'-biquinoline]-3-carbonitrile

Example 12 was synthesized by referring to the procedure described in Example 8, using (5-fluoro-2-((4-methoxybenzyl)amino)quinolin-8-yl)boronic acid in place of (2-((*tert*-butoxycarbonyl)amino)-7-fluorobenzo[*d*]thiazol-4-yl)boronic acid in step A. LCMS (m/z): 519.1 (M+H).

**Example 13**

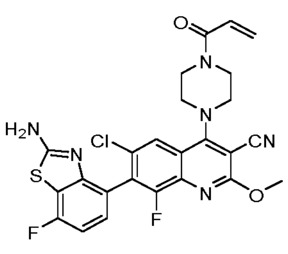

4-(4-Acryloylpiperazin-1-yl)-7-(2-amino-7-fluorobenzo[*d*]thiazol-4-yl)-6-chloro-8-fluoro-2-methoxyquinoline-3-carbonitrile

Example 13 was synthesized by referring to the procedure described for Example 1, using *tert*-butyl 4-(7-bromo-2,6-dichloro-3-cyano-8-fluoroquinolin-4-yl)piperazine-1-carboxylate (Intermediate Int B2) in place of *tert*-butyl (*R*)-4-(7-bromo-2,6-dichloro-3-cyano-8-fluoroquinolin-4-yl)-2-methylpiperazine-1-carboxylate (Intermediate Int B1), and using sodium methoxide in place of the mixture of (3*R*,4*R*)-4-methoxy-1-methylpyrrolidin-3-ol and NaH in Step A. $^1$H NMR (400 MHz, DMSO-$d_6$) δ 7.94 (d, *J* = 5.9 Hz, 3H), 7.25 (dd, *J* = 8.4, 5.6 Hz, 1H), 7.08 (t, *J* = 8.8 Hz, 1H), 6.91 (dd, *J* = 16.7, 10.5 Hz, 1H), 6.20 (dd, *J* = 16.7, 2.2 Hz, 1H), 5.76 (dd, *J* = 10.4, 2.2 Hz, 1H), 4.05 (s, 3H), 3.87 (d, *J* = 15.7 Hz, 4H), 3.68 (s, 4H). $^{19}$F NMR (376 MHz, DMSO) δ -112.27, -119.27. LCMS (m/z): 541.3 (M+H).

**Example 14**

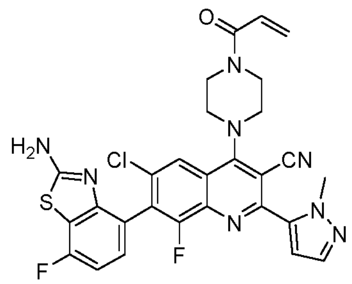

4-(4-Acryloylpiperazin-1-yl)-7-(2-amino-7-fluorobenzo[d]thiazol-4-yl)-6-chloro-8-fluoro-2-(1-methyl-1H-pyrazol-5-yl)quinoline-3-carbonitrile

Example 14 was synthesized by referring to the procedure described in Example 3, using (1-methyl-1H-pyrazol-5-yl)boronic acid in place of (3-((dimethylamino)methyl)phenyl)boronic acid in Step A. $^1$H NMR (400 MHz, DMSO-$d_6$) δ 8.12 (s, 1H), 7.94 (s, 2H), 7.61 (s, 1H), 7.29 (dd, *J* = 8.3, 5.7 Hz, 1H), 7.10 (t, *J* = 8.8 Hz, 1H), 7.00 - 6.86 (m, 2H), 6.20 (d, *J* = 16.7 Hz, 1H), 5.77 (d, *J* = 8.7 Hz, 1H), 4.04 (s, 3H), 3.90 (d, *J* = 14.7 Hz, 4H), 3.79 (s, 4H). $^{19}$F NMR (376 MHz, DMSO) δ -112.07, -118.03. LCMS (m/z): 591.4 (M+H).

**Example 15**

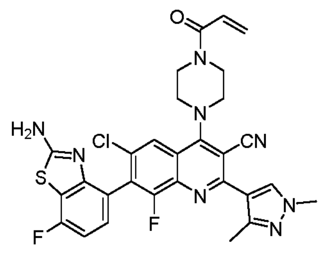

4-(4-Acryloylpiperazin-1-yl)-7-(2-amino-7-fluorobenzo[*d*]thiazol-4-yl)-6-chloro-2-(1,3-dimethyl-1*H*-pyrazol-4-yl)-8-fluoroquinoline-3-carbonitrile

Example 15 was synthesized by referring to the procedure described in Example 3, using (1,3-dimethyl-1H-pyrazol-4-yl)boronic acid in place of (3-((dimethylamino)methyl)phenyl)boronic acid in Step A. $^1$H NMR (400 MHz, DMSO-$d_6$) δ 8.30 (s, 1H), 8.05 (d, *J* = 1.5 Hz, 1H), 7.94 (s, 2H), 7.28 (dd, *J* = 8.5, 5.6 Hz, 1H), 7.09 (t, *J* = 8.8 Hz, 1H), 6.93 (dd, *J* = 16.7, 10.4 Hz, 1H), 6.20 (dd, *J* = 16.6, 2.4 Hz, 1H), 5.77 (dd, *J* = 10.4, 2.4 Hz, 1H), 3.89 (d, *J* = 11.9 Hz, 7H), 3.73 (d, *J* = 5.5 Hz, 4H), 2.41 (s, 3H). $^{19}$F NMR (376 MHz, DMSO) δ -112.23, -118.42. LCMS (m/z): 605.4 (M+H).

**Example 16**

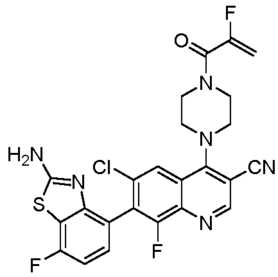

7-(2-Amino-7-fluorobenzo[*d*]thiazol-4-yl)-6-chloro-8-fluoro-4-(4-(2-fluoroacryloyl)piperazin-1-yl)quinoline-3-carbonitrile

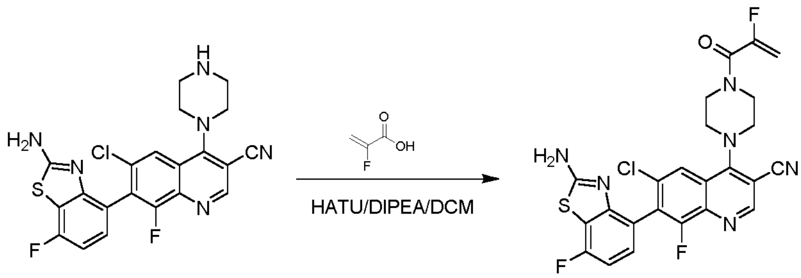


7-(2-Amino-7-fluorobenzo[d]thiazol-4-yl)-6-chloro-8-fluoro-4-(piperazin-1-yl)quinoline-3-carbonitrile (88 mg, 0.13mmol), 2-fluoroacrylic acid (13.3mg, 0.15mmol) and HATU (77 mg, 0.20mmol) were dissolved in DCM (5mL), and DIPEA (52 mg, 0.40mmol) was added dropwise to the reaction under stirring at room temperature. After the reaction was complete indicated by LCMS, 20 mL of water was added to the reaction system. The organic phase was separated, collected and concentrated to dry. The residue was purified by preparative HPLC to afford 7-(2-amino-7-fluorobenzo[*d*]thiazol-4-yl)-6-chloro-8-fluoro-4-(4-(2-fluoroacryloyl)piperazin-1-yl)quinoline-3-carbonitrile (9 mg, yield 13%) as a white solid. $^1$H NMR (400 MHz, DMSO-$d_6$) $\delta$ 8.89 (s, 1H), 8.06 (s, 1H), 7.94 (s, 2H), 7.29 (dd, *J* = 8.4, 5.6 Hz, 1H), 7.09 (t, *J* = 8.8 Hz, 1H), 5.38-5.33 (m, 1H), 5.32 (dd, *J* = 64, 4.1 Hz, 1H), 3.91-3.84 (m, 4H), 3.79-3.74 (m, 4H). $^{19}$F NMR (376 MHz, DMSO-$d_6$) $\delta$-105.25, -112.15, -117.60. LCMS: 529.3(M+H).

**Example 17**

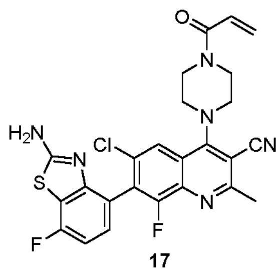

**17**

4-(4-Acryloylpiperazin-1-yl)-7-(2-amino-7-fluorobenzo [d] thiazol-4-yl)-6-chloro-8-fluoro-2-methylquinoline-3 -carbonitrile

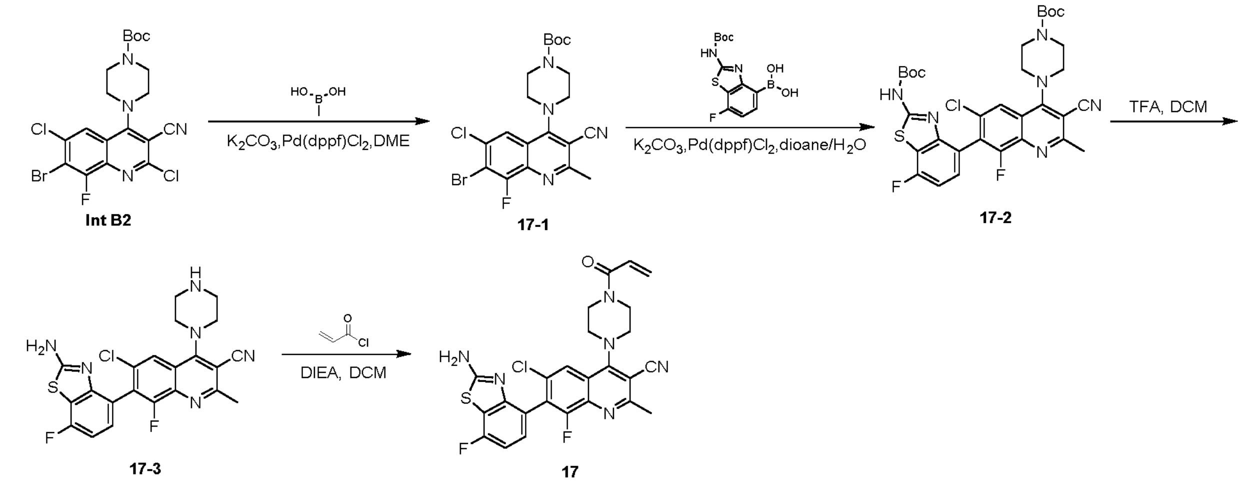


Int B2 17-1 17-2 17-3 17

**Step** A: *tert*-Butyl 4-(7-bromo-6-chloro-3-cyano-8-fluoro-2-methylquinolin-4-yl)piperazine-l-carboxylate

*tert*-Butyl 4-(7-bromo-2,6-dichloro-3-cyano-8-fluoroquinolin-4-yl)piperazine-1-carboxylate (Intermediate B2, 500mg , 0.99mmol), methylboronic acid (71mg, 1.19mmol) and $K_2CO_3$ (411mg, 2.98mmol) and DME (3mL) were charged into a reaction flask, which was purged by nitrogen for 3 min. Pd(dppf)$Cl_2$ (72mg, 0.099mmol) was added, and nitrogen was continued bubbling for additional 3 minutes. The flask was sealed, allowed to react at 90°C for 1 hour, and cooled to room temperature. EA (10 mL) and $H_2O$ (5 mL) were added for separation, and the aqueous phase was extracted with EA (20 mL×2). The combined organic phases were washed with saturated NaCl (30 mL), dried over anhydrous $Na_2SO_4$, filtered, and the filtrate was concentrated under reduced pressure to provide a crude. The crude was purified by FCC ($SiO_2$, EA/PE=0-100%) to afford *tert*-butyl 4-(7-bromo-6-chloro-3-cyano-8-fluoro-2-methylquinolin-4-yl)piperazine-1-carboxylate (120 mg, yield 25%) as a yellow solid. LCMS (m/z): 483.3 (M+H).

**Step B to Step D:** 4-(4-Acryloylpiperazin-1-yl)-7-(2-amino-7-fluorobenzo[*d*]thiazol-4-yl)-6-chloro-8-fluoro-2-methylquinoline-3-carbonitrile

The subsequent synthesis of Example 17 was carried out by referring to the synthesis of Example 1, using *tert*-butyl 4-(7-bromo-6-chloro-3-cyano-8-fluoro-2-methylquinolin-4-yl)piperazine-1-carboxylate in place of *tert*-butyl (*R*)-4-(7-bromo-6-chloro-3-cyano-8-fluoro-2-(((3*R*,4*R*)-4-methoxy-1-methylpyrrolidin-3-yl)oxy)quinolin-4-yl)-2-methylpiperazine-1-carboxylate in Step B. $^1H$ NMR (400MHz, DMSO-$d_6$) δ 8.04 - 8.01 (m, 1H), 7.94 (s, 2H), 7.27 (dd, *J* = 8.4, 5.6 Hz, 1H), 7.12 - 7.05 (m, 1H), 6.92 (dd, *J* = 16.7, 10.5 Hz, 1H), 6.20 (dd, *J* = 16.6, 2.4 Hz, 1H), 5.76 (dd, *J* = 10.5, 2.4 Hz, 1H), 3.93 - 3.82 (m, 4H), 3.69 (s, 4H), 2.74 (s, 3H). $^{19}F$ NMR (376 MHz, DMSO-$d_6$) δ -112.20, -118.13. LCMS (m/z): 525.4 (M+H).

**Example 18**

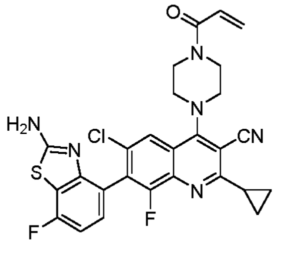

4-(4-Acryloylpiperazin-1-yl)-7-(2-amino-7-fluorobenzo[*d*]thiazol-4-yl)-6-chloro-2-cyclopropyl-8-fluoroquinoline-3-carbonitrile

Example 18 was synthesized by referring to the procedure described in Example 3, using cyclopropylboronic acid in place of (3-((dimethylamino)methyl)phenyl)boronic acid in Step A. $^1H$ NMR (400 MHz, DMSO-$d_6$) δ 8.01 (d, *J* =

1.5 Hz, 1H), 7.96 (s, 2H), 7.27 (dd, *J* = 8.5, 5.6 Hz, 1H), 7.13 - 7.05 (m, 1H), 6.94 (dd, *J* = 16.7, 10.4 Hz, 1H), 6.22 (dd, *J*= 16.6, 2.4 Hz, 1H), 5.78 (dd, *J*= 10.4, 2.4 Hz, 1H), 4.00 - 3.81 (m, 4H), 3.79 - 3.65 (m, 4H), 2.57 - 2.54 (m, 1H), 1.25 - 1.12 (m, 4H). LCMS: 551.5(M+H).

**Example 19**

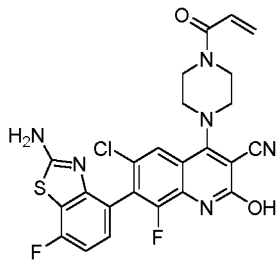

4-(4-Acryloylpiperazin-1-yl)-7-(2-amino-7-fluorobenzo[*d*]thiazol-4-yl)-6-chloro-8-fluoro-2-hydroxyquinoline-3-carbonitrile

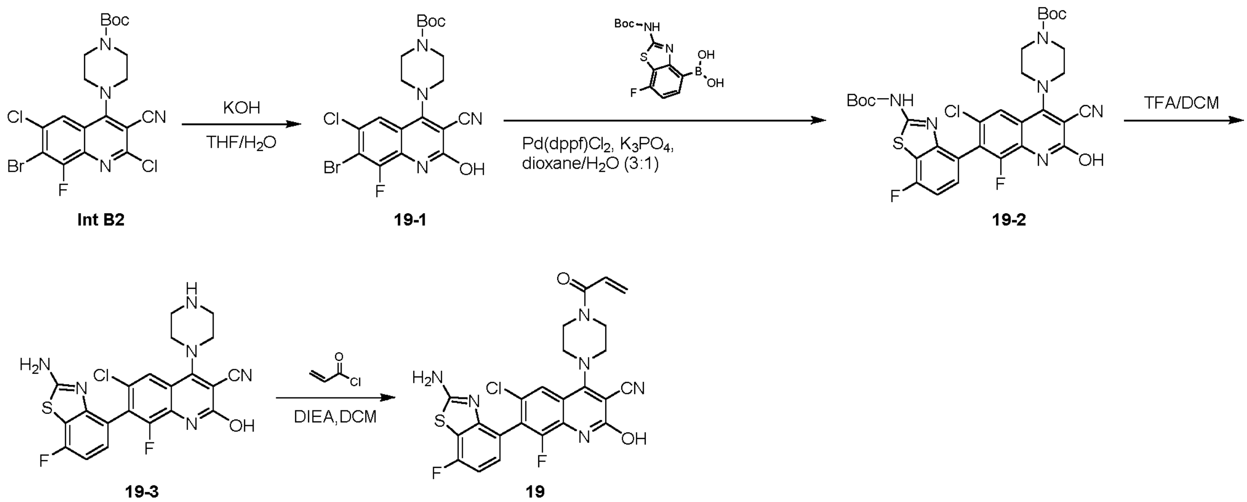


Int B2 19-1 19-2 19-3 19

**Step A:** *tert*-Butyl 4-(7-bromo-2,6-chloro-3-cyano-8-fluoro-2-hydroxyquinolin-4-yl)piperazine-1-carboxylate

KOH (44 mg, 0.78 mmol) was added to a stirred solution of tert-butyl 4-(7-bromo-2,6-dichloro-3-cyano-8-fluoroquinolin-4-yl)piperazine-1-carboxylate (200mg, 0.39mmol) in THF/ $H_2O$ (v/v=2:1, 4mL) at room temperature. The resulting mixture was stirred at 80°C for 3h and LCMS indicated the reaction was complete. The reaction was concentrated under reduced pressure to remove THF, poured into saturated aqueous $NH_4Cl$ solution (4mL), and carefully adjusted pH to 5-6 with1N hydrochloric acid. The product precipitated, was filtered, washed with water and petroleum ether each for twice, and concentrated under vacuum to afford *tert*-butyl 4-(7-bromo-2,6-chloro-3-cyano-8-fluoro-2-hydroxyquinolin-4-yl)piperazine-1-carboxylate, which was used directly in the next step. LCMS (m/z): 429.2, 431.1 (M-56).

**Step B to Step D:** 4-(4-Acryloylpiperazin-1-yl)-7-(2-amino-7-fluorobenzo[*d*]thiazol-4-yl)-6-chloro-8-fluoro-2-hydroxyquinoline-3-carbonitrile

The subsequent synthesis of Example 19 was carried out by referring to the synthesis of Example 1, using *tert*-butyl 4-(7-bromo-2,6-chloro-3-cyano-8-fluoro-2-hydroxyquinolin-4-yl)piperazine-1-carboxylate in place of *tert*-butyl (R)-4-(7-bromo-6-chloro-3-cyano-8-fluoro-2-(((3*R*,4*R*)-4-methoxy-1-methylpyrrolidin-3-yl)oxy)quinolin-4-yl)-2-methylpiperazine-1-carboxylate in Step B. $^1$HNMR (400 MHz, DMSO-$d_6$) $\delta$ 12.11 (s, 1H), 7.98 (s, 2H), 7.74 (s, 1H), 7.22 (dd, *J* = 8.5, 5.6 Hz, 1H), 7.11 - 7.04 (m 1H), 6.90 (dd, *J* = 16.6, 10.4 Hz, 1H), 6.19 (dd, *J* = 16.7, 2.3 Hz, 1H), 5.76 (dd, *J* =

10.4, 2.3 Hz, 1H), 3.85 (d, *J* = 19.0 Hz, 4H), 3.65 (s, 4H). $^{19}F$ NMR (376 MHz, DMSO) δ -112.03, -123.05. LCMS (m/z): 527.3 (M+H).

**Example 20**

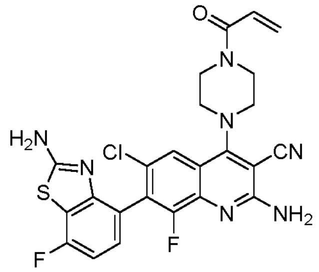

4-(4-Acryloylpiperazin-1-yl)-2-amino-7-(2-amino-7-fluorobenzo[*d*]thiazol-4-yl)-6-chloro-8-fluoroquinoline-3-carbonitrile

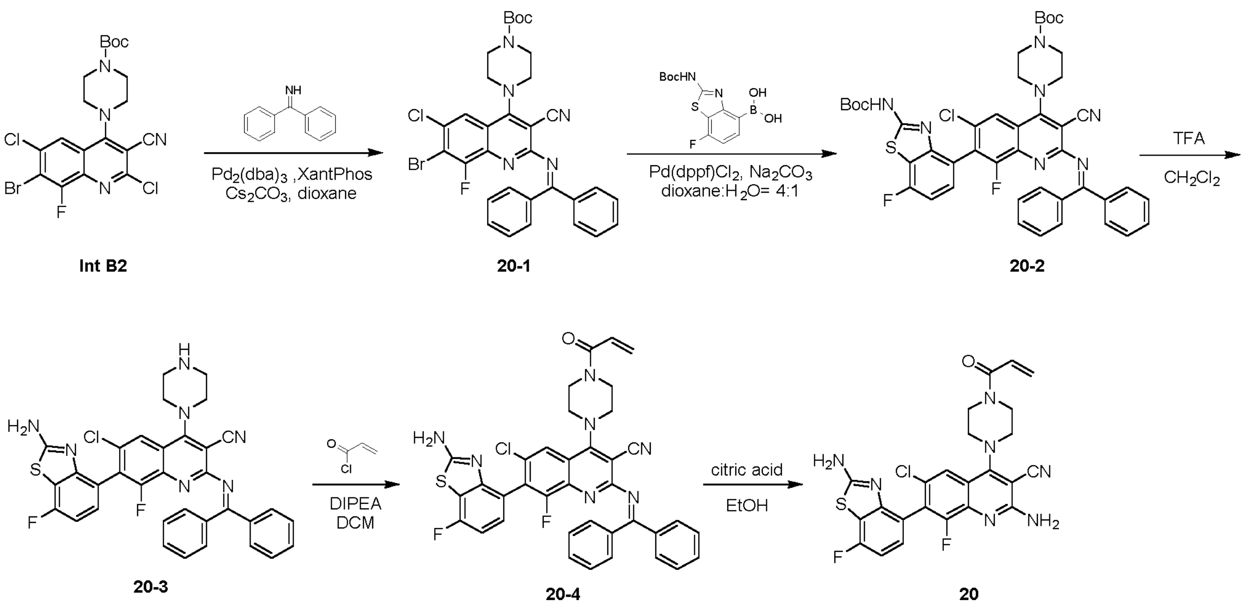


Int B2 → 20-1 → 20-2 → 20-3 → 20-4 → 20

**Step A:** *tert*-Butyl 4-(7-bromo-6-chloro-3-cyano-2-((diphenylmethylene)amino)-8-fluoroquinolin-4-yl)piperazine-1 -carboxylate

4-(4-Acryloylpiperazin-1-yl)-2-amino-7-(2-amino-7-fluorobenzo[*d*]thiazol-4-yl)-6-chloro-8-fluoroquinoline-3-carbonitrile (350mg, 694μmol), diphenylmethanimine (126mg, 694μmol), $Pd_2(dba)_3$ (64mg, 69.4 μmol), XantPhos (80mg, 139μmol), $C_{S2}CO_3$ (679mg, 2.08mmol) and dioxane (5mL) were added into a microwave tube, purged by nitrogen for 1 minute. Th tube was capped, heated to 80°C under microwave, and allowed to react for 1 h. After the reaction was complete, the reaction was cooled to room temperature, poured into water (50 mL), extracted with EA (50mL×3). The extraction liquids were collected, concentrated, and further purified by FCC ($SiO_2$, EA/PE=0-50%) to afford *tert*-butyl 4-(7-bromo-6-chloro-3-cyano-2-((diphenylmethylene)amino)-8-fluoroquinolin-4-yl)piperazine-1-carboxylate (280 mg, yield 62%) as a yellow solid. LCMS (m/z): 650.4 (M+H).

**Step B:** *tert*-Butyl 4-(7-(2-((tert-butoxycarbonyl)amino)-7-fluorobenzo[*d*]thiazol-4-yl)-6-chloro-3-cyano-2-((diphenylmethylene)amino)-8-fluoroquinolin-4-yl)piperazine-1-carboxylate

To a reaction tube equipped with magnetic stirring bar was charged *tert*-butyl 4-(7-bromo-6-chloro-3-cyano-2-((diphenylmethylene)amino)-8-fluoroquinolin-4-yl)piperazine-1-carboxylate (280mg, 431μmol), (2-((*tert*-butoxycarbonyl)amino)-7-fluorobenzothiazol-4-yl)boronic acid (135mg, 431μmol), Pd(dppf)$Cl_2$ (32mg, 43μmol), $Na_2CO_3$ (137mg, 1.29mmol) and 1,4-dioxane/$H_2O$ (v/v--4:1, 8 mL). The reaction was purged with nitrogen for 1 min and the tube was sealed, heated to 115°C under microwave for 2 h, cooled to room temperature, diluted with water (60 mL), extracted with EA (50 mL×3), dried over anhydrous $Na_2SO_4$ and concentrated under reduced pressure to give *tert*-butyl 4-(7-(2-((tert-butoxycarbonyl)amino)-7-fluorobenzo[*d*]thiazol-4-yl)-6-chloro-3-cyano-2-((diphenylmethylene)amino)-8-fluoroquinolin-4-yl)piperazine-1-carboxylate (310 mg, yield 86%). LCMS (m/z): 836.6 (M+H).

**Step C:** 7-(2-Amino-7-fluorobenzo[*d*]thiazol-4-yl)-6-chloro-2-((diphenylmethylene)amino)-8-fluoro-4-(piperazin-1-yl)quinoline-3-carbonitrile

TFA/$CH_2Cl_2$ (V/V=1:1, 15mL) was added to *tert*-butyl 4-(7-(2-((tert-butoxycarbonyl)amino)-7-fluorobenzo[*d*]thiazol-4-yl)-6-chloro-3-cyano-2-((diphenylmethylene)amino)-8-fluoroquinolin-4-yl)piperazine-1-carboxylate (310mg, 371μmol) at r.t., and the reaction was stirred for 0.5 h. LCMS showed the reaction was complete. The reaction was diluted with EA (10mL), pour into saturated aqueous $NaHCO_3$ solution (20mL), extracted with EA (30 mL×3), dried over anhydrous $Na_2SO_4$. The extraction liquids are collected to give 7-(2-amino-7-fluorobenzo[*d*]thiazol-4-yl)-6-chloro-2-((diphenylmethylene)amino)-8-fluoro-4-(piperazin-1-yl)quinoline-3-carbonitrile (140 mg, yield 59%). LCMS (m/z): 636.6 (M+H).

**Step D:** 4-(4-Acryloylpiperazin-1-yl)-7-(2-amino-7-fluorobenzo[*d*]thiazol-4-yl)-6-chloro-2-((diphenylmethylene)amino)-8-fluoroquinoline-3-carbonitrile

A solution of acryloyl chloride (24 mg, 264 μmol) in $CH_2Cl_2$ (0.5 mL) was added dropwise using a syringe to an ice-bath cooled solution of 7-(2-amino-7-fluorobenzo[*d*]thiazol-4-yl)-6-chloro-2-((diphenylmethylene)amino)-8-fluoro-4-(piperazin-1-yl)quinoline-3-carbonitrile (140mg, 220μmol) and DIPEA (85 mg, 660 μmol) in $CH_2Cl_2$ (5mL), and stirred for 10 minutes. LCMS indicated the reaction was complete. The reaction was poured into water (50mL), extracted with $CH_2Cl_2$ (30mL×3). The collected extraction liquids were concentrated to afford 4-(4-acryloylpiperazin-1-yl)-7-(2-amino-7-fluorobenzo[*d*]thiazol-4-yl)-6-chloro-2-((diphenylmethylene)amino)-8-fluoroquinoline-3-carbonitrile (150mg, crude) as a yellow solid. LCMS (m/z): 690.0 (M+H).

**Step E:** 4-(4-Acryloylpiperazin-1-yl)-2-amino-7-(2-amino-7-fluorobenzo[d]thiazol-4-yl)-6-chloro-8-fluoroquinoline-3-carbonitrile

4-(4-Acryloylpiperazin-1-yl)-7-(2-amino-7-fluorobenzo[*d*]thiazol-4-yl)-6-chloro-2-((diphenylmethylene)amino)-8-fluoroquinoline-3-carbonitrile (150 mg, 217 μmol) was dissolved in ethanol (30 mL) at room temperature, added with a saturated citric acid aqueous solution (1.5 mL), and heated to 60°C and stirred overnight. LCMS indicated the formation of the product. The reaction was concentrated, water (30 mL) was added and extracted with EA (50mL×3). The crude products obtained by concentration was separated by preparative HPCL to give 4-(4-acryloylpiperazin-1-yl)-2-amino-7-(2-amino-7-fluorobenzo[*d*]thiazol-4-yl)-6-chloro-8-fluoroquinoline-3-carbonitrile (11mg, yield 10%). $^1$HNMR (400 MHz, Chloroform-*d*) δ 7.71 (d, *J* = 1.6 Hz, 1H), 7.25 - 7.15 (m, 1H), 7.05 - 6.91 (m, 1H), 6.69 - 6.55 (m, 1H), 6.38 (dd, *J* = 16.7, 1.8 Hz, 1H), 5.80 (dd, *J* = 10.6, 1.8 Hz, 1H), 5.68 (s, 2H), 4.06 - 3.80 (m, 4H), 3.76 - 3.56 (m, 4H). $^{19}$F NMR (376 MHz, Chloroform-*d*) δ -111.62, -119.98. LCMS (m/z): 526.4 (M+H).

**Synthesis of Compounds 21-2 and 22-2**

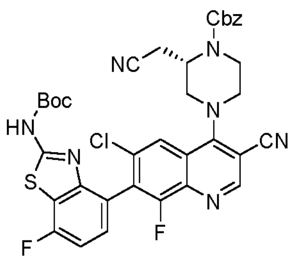

**21-2**

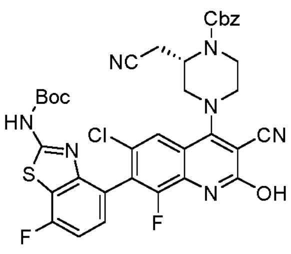

**22-2**

Benzyl (2*S*)-4-(7-(2-((*tert*-butoxycarbonyl)amino)-7-fluorobenzo[*d*]thiazol-4-yl)-6-chloro-3-cyano-8-fluoroquinolin-4-yl)-2-(cyanomethyl)piperazine-1-carboxylate (21-2) and benzyl (2*S*)-4-(7-(2-((*tert*-butoxycarbonyl)amino)-7-fluorobenzo[*d*]thiazol-4-yl)-6-chloro-3-cyano-8-fluoro-2-hydroxyquinolin-4-yl)-2-(cyanomethyl)piperazine-1 -carboxylate (22-2)

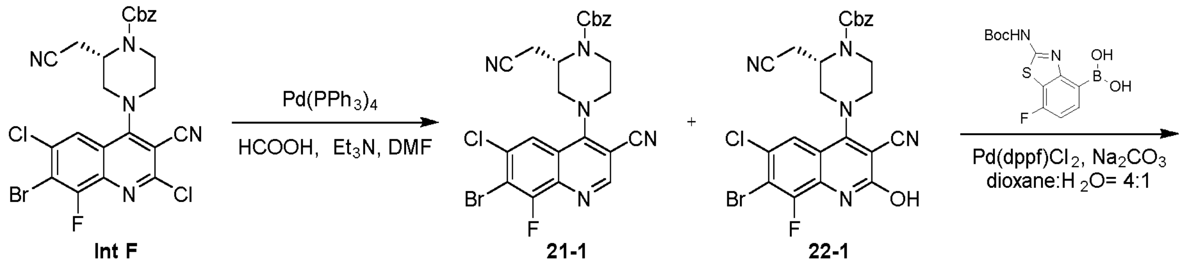


**Int F** **21-1** **22-1**

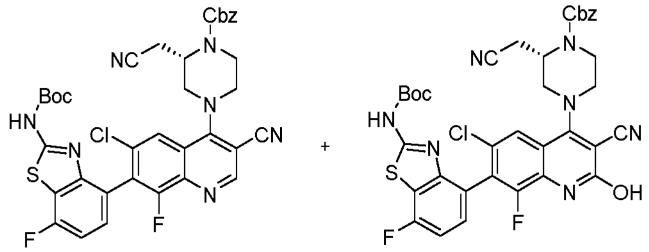


**21-2** **22-2**

**Step A:** Benzyl (*S*)-4-(7-bromo-6-chloro-3-cyano-8-fluoroquinolin-4-yl)-2-(cyanomethyl)piperazine-1-carboxylate (21-1) and benzyl (*S*)-4-(7-bromo-6-chloro-3-cyano-8-fluoro-2-hydroxyquinolin-4-yl)-2-(cyanomethyl)piperazine-1-carboxylate (22-1)

To a round bottom flask equipped with magnetic stirring bar was charged benzyl (*S*)-4-(7-bromo-2,6-dichloro-3-cyano-8-fluoroquinolin-4-yl)-2-(cyanomethyl)piperazine-1-carboxylate (Int F, 500mg, 866.2μmol), $Pd(PPh_3)_4$ (100mg, 87μmol), formic acid (159mg, 3.46mmol) and triethylamine (438mg, 4.33mmol). The flask was degassed and refilled with nitrogen for three times and DMF (15mL) was added then. The reaction was heated to 55°C and stirred for 6 h. After the reaction was complete, the reaction was cooled to room temperature, poured into water (150 mL). A yellow solid formed, and was filtered. The filtrate was washed with PE (15 mL) and $H_2O$ (15 mL), and the solids were collected to afford a mixture of benzyl (*S*)-4-(7-bromo-6-chloro-3-cyano-8-fluoroquinolin-4-yl)-2-(cyanomethyl)piperazine-1-carboxylate (21-1) and benzyl (*S*)-4-(7-bromo-6-chloro-3-cyano-8-fluoro-2-hydroxyquinolin-4-yl)-2-(cyanomethyl)piperazine-1-carboxylate (22-1) (530 mg). LCMS (m/z): 544.3 (M+H) (21-1) and LCMS (m/z): 558.3 (M+H) (22-1).

**Step B:** Benzyl (2S)-4-(7-(2-((*tert*-butoxycarbonyl)amino)-7-fluorobenzo[d]thiazol-4-yl)-6-chloro-3-cyano-8-fluoroquinolin-4-yl)-2-(cyanomethyl)piperazine-1-carboxylate (21-2) and benzyl (2*S*)-4-(7-(2-((*tert*-butoxycarbonyl)amino)-7-fluorobenzo[*d*]thiazol-4-yl)-6-chloro-3-cyano-8-fluoro-2-hydroxyquinolin-4-yl)-2-(cyanomethyl)piperazine-1-carboxylate (22-2)

To a reaction tube equipped with magnetic stirring bar was added the mixture of benzyl (*S*)-4-(7-bromo-6-chloro-3 -cyano-8-fluoroquinolin-4-yl)-2-(cyanomethyl)piperazine-1 -carboxylate (21-1) and benzyl (*S*)-4-(7-bromo-6-chloro-3-cyano-8-fluoro-2-hydroxyquinolin-4-yl)-2-(cyanomethyl)piperazine-1-carboxylate (22-1) (530mg, 976.43μmol), (2-((tert-butoxycarbonyl)amino)-7-fluorobenzo[d]thiazol-4-yl)boronic acid (335.24mg, 1.07mmol), $Pd(dppf)Cl_2$ (71.64mg, 97.64μmol), $Na_2CO_3$ (313mg, 2.93mmol) and 1,4-dioxane/$H_2O$ (V/V = 4:1, 10 mL). The reaction was bubbled with nitrogen for 1 min, and then sealed, heated to 115°C under microwave and allowed to react for 2 h. The reaction was cooled to room temperature, diluted with water (60 mL), and extracted with EA (50 mL×3). The combined extracts were dried over anhydrous $Na_2SO_4$, concentrated under reduced pressure and purified by FCC ($SiO_2$, EA/PE = 0-60%) to afford benzyl (2S)-4-(7-(2-((*tert*-butoxycarbonyl)amino)-7-fluorobenzo[d]thiazol-4-yl)-6-chloro-3-cyano-8-fluoroquinolin-4-yl)-2-(cyanomethyl)piperazine-1-carboxylate (21-2) (80 mg, yield 17%). LCMS (m/z): 730.5 (M+H); and benzyl (2S)-4-(7-(2-((*tert*-butoxycarbonyl)amino)-7-fluorobenzo[*d*]thiazol-4-yl)-6-chloro-3-cyano-8-fluoro-2-hydroxyquinolin-4-yl)-2-(cyanomethyl)piperazine-1-carboxylate (22-2) (380mg, yield 78%). LCMS (m/z): 746.5 (M+H), 646.4 (M-100+H).

**Example 21**

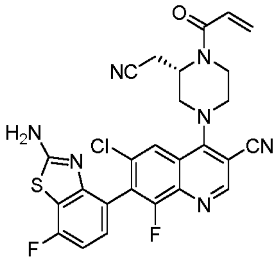

4-((*S*)-4-acryloyl-3-(cyanomethyl)piperazin-1-yl)-7-(2-amino-7-fluorobenzo[*d*]thiazol-4-yl)-6-chloro-8-fluoroquinoline-3-carbonitrile

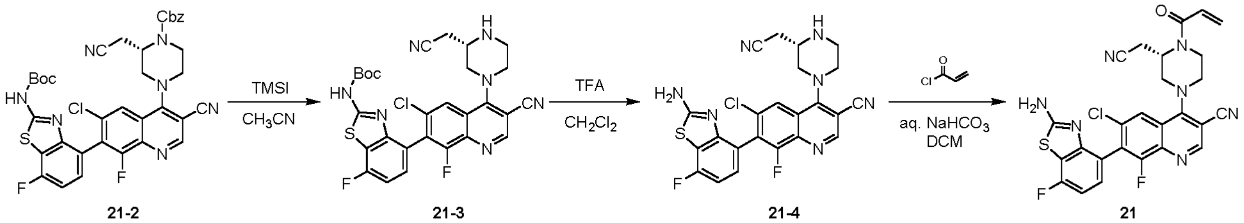


**Step A:** *tert*-Butyl (4-(6-chloro-3-cyano-4-((*S*)-3-(cyanomethyl)piperazin-1-yl)-8-fluoroquinolin-7-yl)-7-fluorobenzo[d]thiazol-2-yl)carbamate

TMSI (110 mg, 548 μmol) was added dropwise to a solution of benzyl (2*S*)-4-(7-(2-((*tert*-butoxycarbonyl)amino)-7-fluorobenzo[*d*]thiazol-4-yl)-6-chloro-3-cyano-8-fluoroquinolin-4-yl)-2-(cyanomethyl)piperazine-1-carboxylate (80 mg, 110 μmol) in MeCN (3 mL), and stirred at room temperature for 5 h., $Et_3N$ (1 mL) was added then and stirred for additional 10 min. LCMS indicated the reaction was complete. The reaction was poured into $H_2O$ (30mL), extracted with EA (30mL×3), and the extracts were combined to give *tert*-butyl (4-(6-chloro-3-cyano-4-((*S*)-3-(cyanomethyl)piperazin-1 -yl)-8-fluoroquinolin-7-yl)-7-fluorobenzo[d]thiazol-2-yl)carbamate (68mg, crude product). LCMS (m/z): 596.4 (M+H).

**Step B**: 7-(2-Amino-7-fluorobenzo[*d*]thiazol-4-yl)-6-chloro-4-((*S*)-3-(cyanomethyl)piperazin-1-yl)-8-fluoroquinoline-3 -carbonitrile

TFA/DCM (v/v=1:1, 5 mL) was added to a solution of *tert*-butyl (4-(6-chloro-3-cyano-4-((*S*)-3-(cyanomethyl)piperazin-1 -yl)-8-fluoroquinolin-7-yl)-7-fluorobenzo[d]thiazol-2-yl)carbamate (68mg, crude) at room temperature and stirred for 30 min at room temperature. LCMS indicated the reaction was complete. The reaction was concentrated to remove most of the solvents, diluted with EA (5mL), poured into saturated aqueous $NaHCO_3$ solution (20mL), and extracted with EA (30mL×3), and dried over anhydrous $Na_2SO_4$. The extracts were collected to give 7-(2-amino-7-fluorobenzo[*d*]thiazol-4-yl)-6-chloro-4-((*S*)-3-(cyanomethyl)piperazin-1-yl)-8-fluoroquinoline-3-carbonitrile (60 mg, crude). LCMS (m/z): 496.3 (M+H).

**Step C:** 4-((*S*)-4-acryloyl-3-(cyanomethyl)piperazin-1-yl)-7-(2-amino-7-fluorobenzo[*d*]thiazol-4-yl)-6-chloro-8-fluoroquinoline-3-carbonitrile

A solution of acryloyl chloride (11 mg, 121 μmol) in DCM (0.5 mL) was added dropwise using a syringe to a mixed solution of 7-(2-amino-7-fluorobenzo[*d*]thiazol-4-yl)-6-chloro-4-((5)-3-(cyanomethyl)piperazin-1-yl)-8-fluoroquinoline-3-carbonitrile (60 mg, crude), saturated aqueous $NaHCO_3$ solution (1.5 mL) and DCM (3mL) under ice bath, and stirred for 10 min. LCMS showed that the reaction was complete. The reaction was poured into water (30mL), extracted

with DCM (20mL×2). The combined extracts were concentrated, and the resulting crude was further purified by preparative HPLC to afford 4-((*S*)-4-acryloyl-3-(cyanomethyl)piperazin-1-yl)-7-(2-amino-7-fluorobenzo[*d*]thiazol-4-yl)-6-chloro-8-fluoroquinoline-3-carbonitrile (7mg, 10% overall yield in three steps) as a white solid. $^1$H NMR (400 MHz, Methanol-$d_4$) δ 8.77 (s, 1H), 8.07 (d, *J* = 1.6 Hz, 1H), 7.26 - 7.11 (m, 1H), 7.00 - 6.88 (m, 1H), 6.82 (s, 1H), 6.26 (dd, *J* = 16.7, 1.9 Hz, 1H), 5.80 (dd, *J* = 10.7, 1.8 Hz, 1H), 5.24 (d, *J* = 24.8 Hz, 1H), 4.21 (s, 1H), 4.10 - 3.71 (m, 4H), 3.43 (d, *J*= 12.4 Hz, 1H), 3.15 (s, 1H), 3.01 - 2.86 (m, 1H). $^{19}$F NMR (376 MHz, Methanol-$d_4$) δ -114.73, -118.76. LCMS (m/z): 550.4 (M+H).

**Example 22**

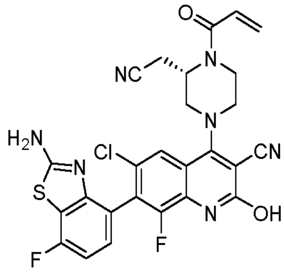

4-((*S*)-4-acryloyl-3-(cyanomethyl)piperazin-1-yl)-7-(2-amino-7-fluorobenzo[*d*]thiazol-4-yl)-6-chloro-8-fluoro-2-hydroxyquinoline-3-carbonitrile

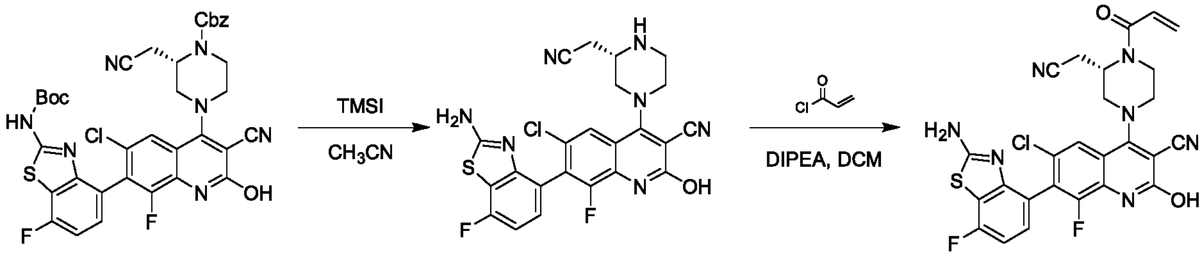


**22-2** **22-3** **22**

**Step A:** 7-(2-Amino-7-fluorobenzo[*d*]thiazol-4-yl)-6-chloro-4-((*S*)-3-(cyanomethyl)piperazin-1-yl)-8-fluoro-2-hydroxyquinoline-3-carbonitrile

TMSI (510 mg, 2.55 mmol) was added dropwise to a mixed solution of benzyl (2*S*)-4-(7-(2-((*tert*-butoxycarbonyl)amino)-7-fluorobenzo[*d*]thiazol-4-yl)-6-chloro-3-cyano-8-fluoro-2-hydroxyquinolin-4-yl)-2-(cyanomethyl)piperazine-1-carboxylate (380mg, 510μmol) in MeCN (10 mL), and stirred at room temperature for 5 h. $Et_3N$ (3 mL) was added then and the reaction was stirred for additional 10 min. LCMS showed the completion of the reaction. The reaction was poured into $H_2O$ (30mL), extracted with EA (50mL×2), and the extracts were combined to afford 7-(2-amino-7-fluorobenzo[*d*]thiazol-4-yl)-6-chloro-4-((*S*)-3-(cyanomethyl)piperazin-1-yl)-8-fluoro-2-hydroxyquinoline-3-carbonitrile (200mg, yield 76%) as a yellow solid. LCMS (m/z): 512.3 (M+H).

**Step B:** 4-((*S*)-4-acryloyl-3-(cyanomethyl)piperazin-1-yl)-7-(2-amino-7-fluorobenzo[*d*]thiazol-4-yl)-6-chloro-8-fluoro-2-hydroxyquinoline-3-carbonitrile

A solution of acryloyl chloride (38 mg, 430 μmol) in $CH_2Cl_2$ (0.5 mL) was added dropwise using a syringe to amixed solution of 7-(2-amino-7-fluorobenzo[*d*]thiazol-4-yl)-6-chloro-4-((*S*)-3-(cyanomethyl)piperazin-1-yl)-8-fluoro-quinoline-3-carbonitrile (200mg, 391μmol), DIPEA (151mg, 1.17mmol ) and DCM (5 mL) under ice bath, and stirred for 10 minutes. LCMS showed the reaction was complete, the reaction was poured into water (50 mL), extracted with DCM (30 mL×3). The combined extracts was concentrated and the resulting crude was further purified by preparative HPLC to afford 4-((*S*)-4-acryloyl-3-(cyanomethyl)piperazin-1-yl)-7-(2-amino-7-fluorobenzo[*d*]thiazol-4-yl)-6-chloro-8-fluoro-2-hydroxyquinoline-3-carbonitrile (52 mg, yield 24%) as a yellow solid. $^1$H NMR (400 MHz, Methanol-$d_4$) δ 7.83 (s, 1H), 7.27 - 7.16 (m, 1H), 7.04 - 6.95 (m, 1H), 6.88 (s, 1H), 6.32 (dd, *J* = 16.6, 1.9 Hz, 1H), 5.86 (dd, *J* = 10.6, 1.9 Hz, 1H),

5.48 - 5.12 (m, 1H), 5.02 (s, 1H), 4.09 - 3.80 (m, 4H), 3.53 - 3.39 (m, 2H), 3.05 - 2.87 (m, 1H). $^{19}F$ NMR (376 MHz, DMSO-d6) δ-112.18, -123.14. LCMS (m/z): 566.4 (M+H).

**Examples 23-31**

The following compounds were prepared in a manner similar to the above:

| Example | Structure | LCMS | Example | Structure | LCMS |
|---|---|---|---|---|---|
| 23 | 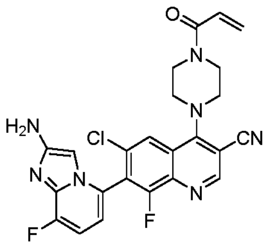 | 494.1 | 24 | 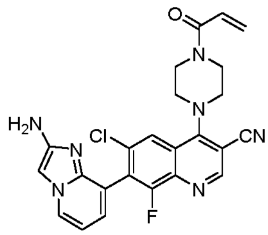 | 476.2 |
| 25 | 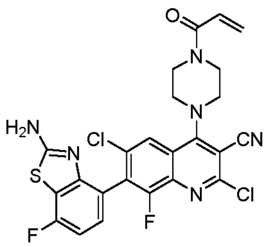 | 545.0 | 26 | 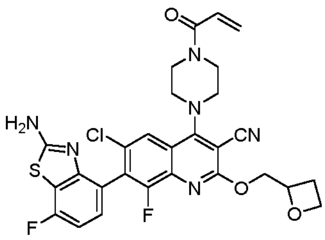 | 597.1 |
| 27 | 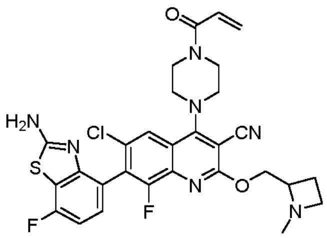 | 610.2 | 28 | 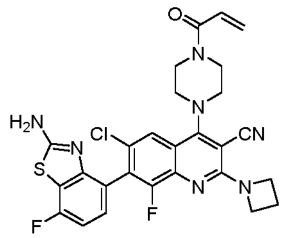 | 566.1 |
| 29 | 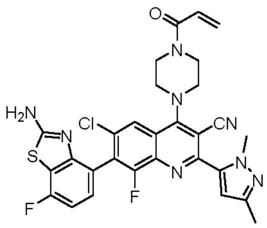 | 605.1 | 30 | 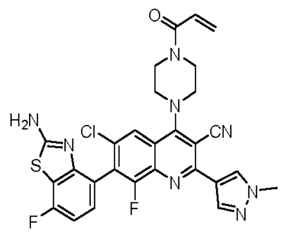 | 591.1 |
| 31 | 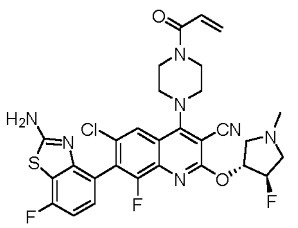 | 628.2 | | | |

**Example 32**

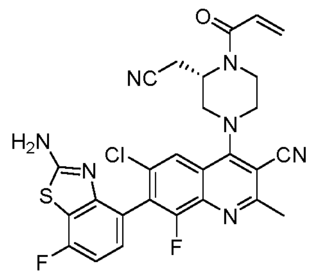

4-((*S*)-4-acryloyl-3-(cyanomethyl)piperazin-1-yl)-7-(2-amino-7-fluorobenzo[*d*]thiazol-4-yl)-6-chloro-8-fluoro-2-methylquinoline-3-carbonitrile

Example 32 was synthesized by referring to the procedure described in Example 17, using benzyl (*S*)-4-(7-bromo-2,6-dichloro-3-cyano-8-fluoroquinolin-4-yl)-2-(cyanomethyl)piperazine-1-carboxylate (Intermediate F) in place of *tert*-butyl 4-(7-bromo-2,6-dichloro-3-cyano-8-fluoroquinolin-4-yl)piperazine-1-carboxylate (Intermediate B2) in Step A. LCMS (m/z): 564.2 (M+H).

**Example 33**

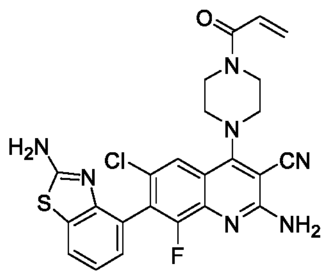

4-(4-Acryloylpiperazin-1-yl)-2-amino-7-(2-aminobenzo[*d*]thiazol-4-yl)-6-chloro-8-fluoroquinoline-3-carbonitrile

Example 33 was synthesized by referring to the procedure described in Example 20, using (2-((*tert*-butoxycarbonyl)amino)benzo[*d*]thiazol-4-yl)boronic acid in place of (2-((*tert*-butoxycarbonyl)amino)-7-fluorobenzo[*d*]thiazol-4-yl)boronic acid in Step B. $^1$H NMR (400 MHz, DMSO-$d_6$) δ 7.80 - 7.69 (m, 2H), 7.63 (s, 2H), 7.19 - 7.01 (m, 4H), 6.91 (dd, *J* = 16.6, 10.5 Hz, 1H), 6.19 (dd, *J* = 16.6, 2.4 Hz, 1H), 5.75 (dd, *J* = 10.3, 2.4 Hz, 1H), 3.97 - 3.76 (m, 4H), 3.66 - 3.49 (m, 4H). LCMS (m/ z): 508.4 (M+H).

**Example 34**

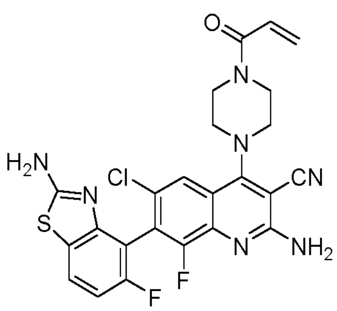

4-(4-Acryloylpiperazin-1-yl)-2-amino-7-(2-amino-5-fluorobenzo[*d*]thiazol-4-yl)-6-chloro-8-fluoroquinoline-3-carbonitrile

Example 34 was synthesized by referring to the procedure described in Example 20, using (2-((*tert*-butoxycarbonyl)amino)-5-fluorobenzo[*d*]thiazol-4-yl)boronic acid in place of (2-((*tert*-butoxycarbonyl)amino)-7-fluorobenzo[*d*]thiazol-4-yl)boronic acid in Step B. $^1H$ NMR (400 MHz, DMSO-$d_6$) δ 8.00 - 7.67 (m, 4H), 7.24 - 7.08 (m, 2H), 7.08 - 6.99 (m, 1H), 6.91 (dd, *J* = 16.6, 10.5 Hz, 1H), 6.19 (d, *J* = 16.6 Hz, 1H), 5.76 (d, *J* = 10.8 Hz, 1H), 3.99 - 3.73 (m, 4H), 3.73 - 3.46 (m, 4H). LCMS(m/z): 526.4 (M+H).

**Example 35**

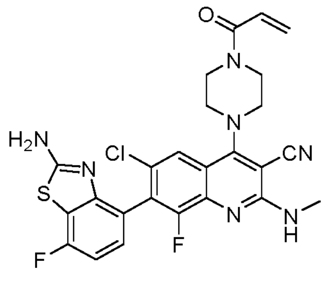

4-(4-Acryloylpiperazin-1-yl)-7-(2-amino-7-fluorobenzo[*d*]thiazol-4-yl)-6-chloro-8-fluoro-2-(methylamino)quinoline-3-carbonitrile

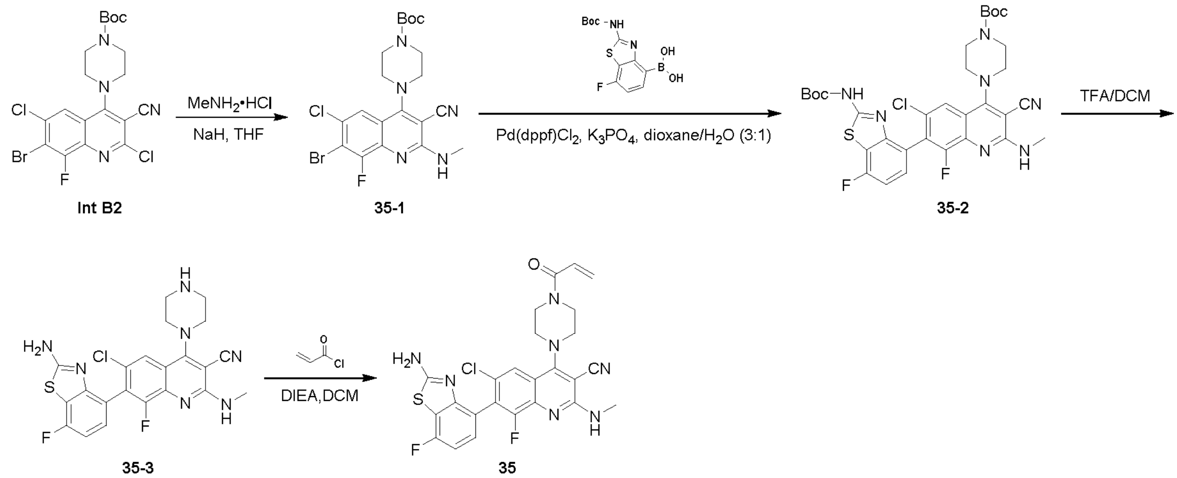

Int B2
35-1
35-2
35-3
35

**Step A:** *tert*-Butyl 4-(7-bromo-6-chloro-3-cyano-8-fluoro-2-(methylamino)quinolin-4-yl)piperazine-1-carboxylate

NaH (143mg, 3.6mmol) was added to a solution of methylamine hydrochloride (121mg, 1.8mmol) in THF (8mL), and stirred for 10 minutes at room temperature. *tert*-Butyl 4-(7-bromo-2,6-dichloro-3-cyano-8-fluoroquinolin-4-yl)piperazine-1-carboxylate (300mg, 0.60mmol) was then added to the mixed solution for reaction for 2 hours at room temperature. LCMS detected the generation of the product, the reaction mixture was added into $H_2O$ (30 mL) and extracted with EA (50 mL×3). The combined organic phases were washed with saturated brine, dried over anhydrous $Na_2SO_4$, to give *tert*-butyl 4-(7-bromo-6-chloro-3-cyano-8-fluoro-2-(methylamino)quinolin-4-yl)piperazine-1-carboxylate (200 mg, yield 67%) as a yellow solid. LCMS (m/z): 499.6 (M+H).

**Step B to Step D:** 4-(4-Acryloylpiperazin-1-yl)-7-(2-amino-7-fluorobenzo[*d*]thiazol-4-yl)-6-chloro-8-fluoro-2-(methylamino)quinoline-3-carbonitrile

The subsequent synthesis of Example 35 was carried out as described in Example 19, using *tert*-butyl 4-(7-bromo-6-chloro-3-cyano-8-fluoro-2-(methylamino)quinolin-4-yl)piperazine-1-carboxylate in place of *tert*-butyl 4-(7-bromo-2,6-dichloro-3-cyano-8-fluoro-2-hydroxyquinolin-4-yl)piperazine-1-carboxylate in Step B. LCMS (m/z): 540.4 (M+H).

**Example 36**

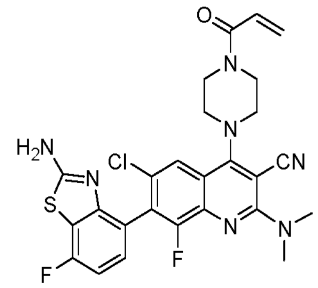

4-(4-Acryloylpiperazin-1-yl)-7-(2-amino-7-fluorobenzo[*d*]thiazol-4-yl)-6-chloro-2-(dimethylamino)-8-fluoroquinoline-3-carbonitrile

Example 36 was synthesized by referring to the procedure described in Example 35, using dimethylamine hydrochloride in place of methylamine hydrochloride in Step A. $^{1}$H NMR (400 MHz, DMSO-$d_6$) δ 7.91 (s, 2H), 7.80 (s, 1H), 7.23 - 7.20 (m, 1H), 7.08 -7.03 (m, 1H), 6.94 - 6.87 (m, 1H), 6.19 (d, *J* = 1.6 Hz, 1H), 5.76 (d, *J* = 1.2 Hz, 1H), 3.87 (d, *J* = 1.6 Hz, 4H), 3.64 (s, 4H), 3.16 (s, 6H). $^{19}$F NMR (376 MHz, DMSO-$d_6$) δ -112.51, -120.42. LCMS (m/z): 554.5 (M+H).

**Example 37**

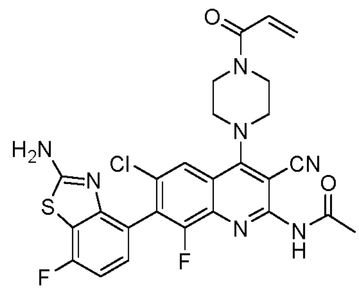

*N*-(4-(4-acryloylpiperazin-1-yl)-7-(2-amino-7-fluorobenzo[*d*]thiazol-4-yl)-6-chloro-3-cyano-8-fluoroquinolin-2-yl)acetamide

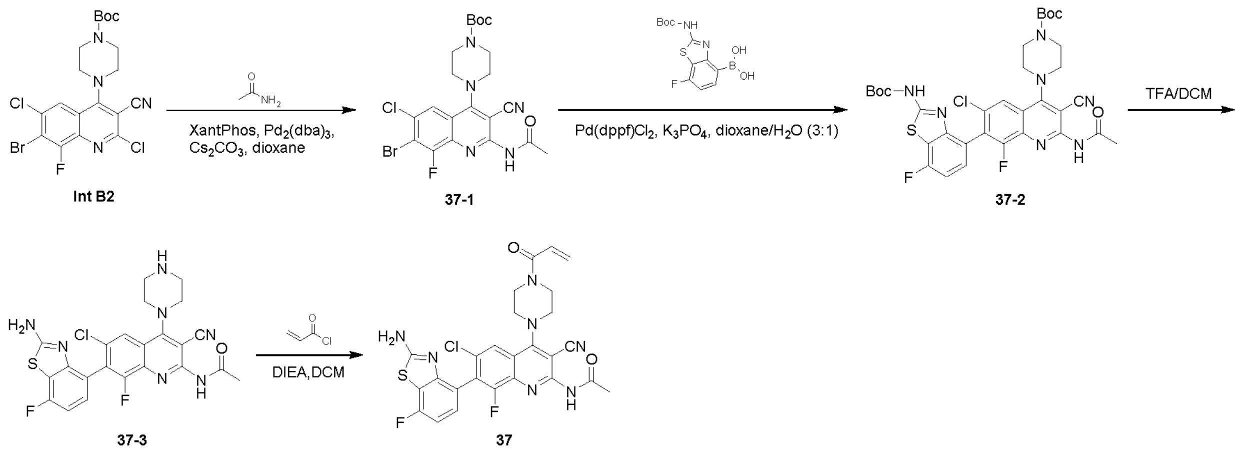


Int B2

37-1

37-2

37-3

37

**Step A:** *tert*-Butyl 4-(2-acetamido-7-bromo-6-chloro-3-cyano-8-fluoroquinolin-4-yl)piperazine-1-carboxylate

To a round-bottomed flask equipped with magnetic stirring bar was added *tert*-butyl 4-(7-bromo-2,6-dichloro-3-cyano-8-fluoroquinolin-4-yl)piperazine-1-carboxylate (600 mg, 1.2 mmol), acetamide (77 mg, 1.3 mmol), $Cs_2CO_3$ (1.16 g, 3.6 mmol) and dioxane (10 mL) at room temperature. After sparging with nitrogen, $Pd_2(dba)_3$ (109mg, 0.12mmol) and Xantphos (65mg, 0.12mmol) were added, further purged with nitrogen, heated to 90°C and stirred overnight. LCMS monitored the reaction was complete. The reaction solution was poured into water, extracted with ethyl acetate, and the combined organic phases were dried over anhydrous sodium sulfate, and concentrated under reduced pressure to give the crude product, which was purified by FCC ($SiO_2$, EA/PE=0-100%) to afford *tert*-butyl 4-(2-acetamido-7-bromo-6-chloro-3-cyano-8-fluoroquinolin-4-yl)piperazine-1-carboxylate (205mg, yield 33%). LCMS (m/z): 526.1 (M+H).

**Step B to Step D**: *N*-(4-(4-acryloylpiperazin-1-yl)-7-(2-amino-7-fluorobenzo[*d*]thiazol-4-yl)-6-chloro-3-cyano-8-fluoroquinolin-2-yl)acetamide

The subsequent synthesis of Example 37 was carried out as described in Example 19, using tert-butyl 4-(2-acetamido-7-bromo-6-chloro-3-cyano-8-fluoroquinolin-4-yl)piperazine-1-carboxylate in place of *tert*-butyl 4-(7-bromo-2,6-dichloro-3-cyano-8-fluoro-2-hydroxyquinolin-4-yl)piperazine-1-carboxylate in Step B. $^1H$ NMR (400 MHz, DMSO-$d_6$) $\delta$ 11.01 (s, 1H), 8.03 (d, *J* = 1.5 Hz, 1H), 7.97 - 7.90 (m, 2H), 7.27 (dd, *J* = 8.4, 5.6 Hz, 1H), 7.13 - 7.04 (m, 1H), 6.91 (dd, *J* = 16.6, 10.4 Hz, 1H), 6.19 (dd, *J* = 16.7, 2.4 Hz, 1H), 5.76 (dd, *J* = 10.4, 2.4 Hz, 1H), 3.97 - 3.81 (m, 4H), 3.74 - 3.60 (m, 4H), 2.13 (s, 3H). $^{19}F$ NMR (376 MHz, DMSO-$d_6$) $\delta$ -112.16, -118.37. LCMS (m/ z): 568.1 (M+H).

**Example 38**

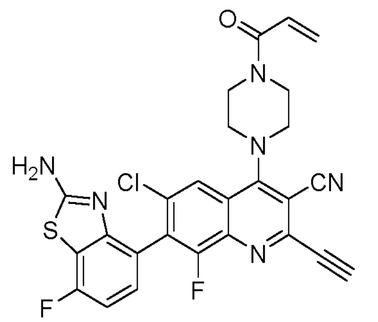

4-(4-Acryloylpiperazin-1-yl)-7-(2-amino-7-fluorobenzo[*d*]thiazol-4-yl)-6-chloro-2-ethynyl-8-fluoroquinoline-3-carbonitrile

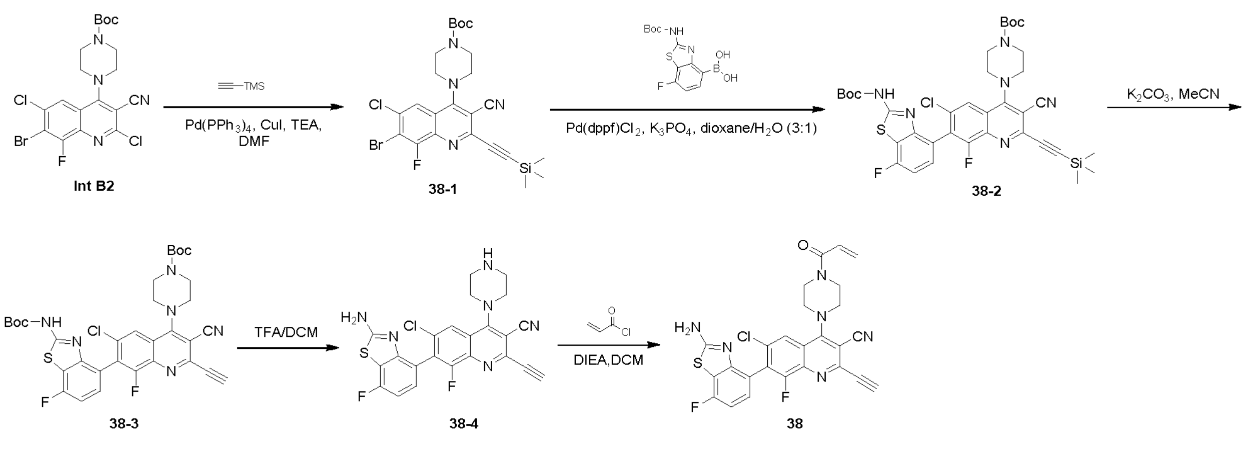

**Step A:** *tert*-Butyl 4-(7-bromo-6-chloro-3-cyano-8-fluoro-2-((trimethylsilyl)ethynyl)quinolin-4-yl)piperazine-1-carboxylate

*tert*-Butyl 4-(7-bromo-2,6-dichloro-3-cyano-8-fluoroquinolin-4-yl)piperazine-1-carboxylate (500 mg, 0.99mmol), ethynyltrimethylsilane (292mg, 3.0mmol), $Pd(PPh_3)_4$ (115mg, 0.099mmol), CuI (38mg, 0.20mmol) and TEA (301mg, 3.0mmol) were added in DMF (10mL) at room temperature, degassed and refilled with nitrogen for three times, and reacted at 70°C for 16 hours. After the product was detected, the reaction solution was poured into $H_2O$ (100mL), extracted with EA (100mL×2), washed with saturated brine, dried over anhydrous $Na_2SO_4$, concentrated, and purified by FCC ($SiO_2$, EA/PE=0-50%) to give *tert*-butyl 4-(7-bromo-6-chloro-3-cyano-8-fluoro-2-((trimethylsilyl)ethynyl)quinolin-4-yl)piperazine-1-carboxylate (350mg, yield 62%) as a yellow solid. LCMS (m/z): 566.0 (M+H).

**Step B:** *tert*-Butyl 4-(7-(2-((tert-butoxycarbonyl)amino)-7-fluorobenzo[*d*]thiazol-4-yl)-6-chloro-3-cyano-8-fluoro-2-((trimethylsilyl)ethynyl)quinolin-4-yl)piperazine-1-carboxylate

To a vial equipped with magnetic stirring bar was added *tert*-butyl 4-(7-bromo-6-chloro-3-cyano-8-fluoro-2-((trimethylsilyl)ethynyl)quinolin-4-yl)piperazine-1-carboxylate (210mg, 0.37mmol), (2-((*tert*-butoxycarbonyl)amino)-7-fluorobenzo[d]thiazol-4-yl)boronic acid (139mg, 0.45 mmol), $K_2CO_3$ (154 mg, 1.1 mmol) and dioxane/water (v/v=3:1, 5 mL) at room temperature. The vial was degassed by sparging with nitrogen, Pd(dppf)$Cl_2$ (27mg, 0.037mmol) was added, and purged with nitrogen again. The reaction was heated to 90°C and stirred for 1 h. After cooling to room temperature, the reaction solution was poured into water, extracted with ethyl acetate, and the combined organic phases were dried over anhydrous sodium sulfate, concentrated under reduced pressure to give a crude product which was purified by FCC ($SiO_2$, EA/PE=0-100%) to give *tert*-butyl 4-(7-(2-((tert-butoxycarbonyl)amino)-7-fluorobenzo[*d*]thiazol-4-yl)-6-chloro-3-cyano-8-fluoro-2-((trimethylsilyl)ethynyl)quinolin-4-yl)piperazine-1-carboxylate (90 mg, yield 32%). LCMS (m/z): 753.2 (M+H).

**Step C:** *tert*-Butyl 4-(7-(2-((*tert*-butoxycarbonyl)amino)-7-fluorobenzo[*d*]thiazol-4-yl)-6-chloro-3-cyano-2-ethynyl-8-fluoroquinolin-4-yl)piperazine-1 -carboxylate

A mixture of *tert*-butyl 4-(7-(2-((tert-butoxycarbonyl)amino)-7-fluorobenzo[*d*]thiazol-4-yl)-6-chloro-3-cyano-8-fluoro-2-((trimethylsilyl)ethynyl)quinolin-4-yl)piperazine-1-carboxylate (90 mg, 0.12 mmol), $K_2CO_3$ (33 mg, 0.24 mmol) and acetonitrile (2 mL) was stirred at room temperature for 1 h. LCMS monitored the reaction was complete. The reaction was poured into water, and extracted with EA. The combined organic phases were washed with brine, dried over anhydrous sodium sulfate, filtered and concentrated to give tert-butyl 4-(7-(2-((*tert*-butoxycarbonyl)amino)-7-fluorobenzo[*d*]thiazol-4-yl)-6-chloro-3-cyano-2-ethynyl-8-fluoroquinolin-4-yl)piperazine-1-carboxylate (70 mg, crude product), which was directly used in the next step without further purification. LCMS (m/z): 681.2 (M+H).

**Steps D and E:** 4-(4-Acryloylpiperazin-1-yl)-7-(2-amino-7-fluorobenzo[*d*]thiazol-4-yl)-6-chloro-2-ethynyl-8-fluoroquinoline-3-carbonitrile

The steps of removal of the protecting group and introduction of acryloyl chloride involved in the subsequent synthesis of Example 38 were carried out by referring to the procedures described in Example 1. LCMS (m/z): 535.1 (M+H).

**Example 39**

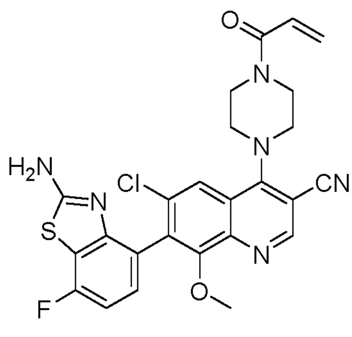

4-(4-Acryloylpiperazin-1-yl)-7-(2-amino-7-fluorobenzo[*d*]thiazol-4-yl)-6-chloro-8-methoxyquinoline-3-carbonitrile

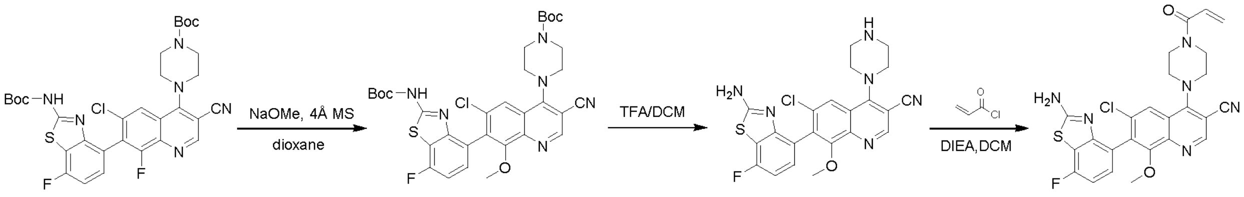


39-1 39-2 39-3 39

**Step A:** *tert*-Butyl 4-(7-(2-((*tert*-butoxycarbonyl)amino)-7-fluorobenzo[*d*]thiazol-4-yl)-6-chloro-3-cyano-8-methoxyquinolin-4-yl)piperazine-1 -carboxylate

To a reaction vial equipped with magnetic stirring bar was added *tert*-butyl 4-(7-(2-((*tert*-butoxycarbonyl)amino)-7-fluorobenzo[*d*]thiazol-4-yl)-6-chloro-3-cyano-8-fluoroquinolin-4-yl)piperazine-1-carboxylate (70mg, 0.11mmol), sodium methoxide (20mg, 0.37mmol), 4Å molecular sieves (100mg) and dioxane (2mL). The vial was sealed, heated to 110°C and stirred for 8h. After the reaction was complete, the reaction was diluted with EA (20 mL), washed with water (15 mL) and saturated brine (15 mL) successively, dried over anhydrous sodium sulfate, filtered and concentrated under reduced pressure. The resulting crude was purified by FCC ($SiO_2$, EA/PE=0-80%) to afford *tert*-butyl 4-(7-(2-((*tert*-butoxycarbonyl)amino)-7-fluorobenzo[d]thiazol-4-yl)-6-chloro-3-cyano-8-methoxyquinolin-4-yl)piperazine-1-carboxylate (42 mg, yield 59%). LCMS (m/z): 669.1 (M+H).

**Steps B and C:** 4-(4-Acryloylpiperazin-1-yl)-7-(2-amino-7-fluorobenzo[*d*]thiazol-4-yl)-6-chloro-8-methoxyquinoline-3-carbonitrile

The steps of removal of protecting group and introduction of acryloyl chloride involved in the subsequent synthesis of Example 39 were carried out by referring to the procedures as described in Example 1. $^1$H NMR (400 MHz, DMSO-$d_6$) $\delta$ 8.86 (s, 1H), 7.96 (s, 1H), 7.89 - 7.82 (m, 2H), 7.16 (dd, *J* = 8.4, 5.7 Hz, 1H), 7.07 - 7.01 (m, 1H), 6.91 (dd, *J* = 16.7, 10.5 Hz, 1H), 6.19 (dd, *J* = 16.7, 2.4 Hz, 1H), 5.75 (dd, *J* = 10.4, 2.4 Hz, 1H), 3.94 - 3.82 (m, 4H), 3.78 (s, 3H), 3.70 - 3.64 (m, 4H). $^{19}$F NMR (376 MHz, DMSO-$d_6$) $\delta$ -113.34. LCMS(m/z): 523.1 (M+H).

**Activity Examples**

**Example 1: Inhibitory activity of the compounds of the present disclosure on the proliferation of KRas G12C mutant cells**

This assay verified the inhibitory activity of the compounds of the present disclosure on the proliferation of human non-small cell lung cancer cells NCI-H358 with KRas G12C mutation by using Promega's CellTiter-Glo® Luminescent Cell Viability Assay kit.

[Test materials] : NCI-H358 cell line (Cell Resource Center, IBMS, CAMS/PUMC, Item number: 3111C0001CCC000470), 96-well transparent flat-bottomed black-walled cell culture plate (Greiner Bio one, Cat#655096), RPMI-1640 Medium (GE, Cat# SH30809.01), Fetal Bovine Serum FBS (Thermo Fisher, Cat #10099-141), CellTiter-Glo® Luminescent Cell Viability Assay Kit (Promega, Cat#G7573), PBS (Solarbio, Cat#P1020), Trypsin (Thermo Fisher, Cat#25200072), DMSO (Sigma, Cat #D2650), Methyl cellulose (SIGMA, Cat #9004-67-5).

(Experimental procedure] : 180 μL of cell suspension (RPMI1640 solution with 10% FBS and 1 % methyl cellulose) was charged to a 96-well cell culture plate at a density of 1500 cells/well. A control group containing no cells, no compounds and only 3D complete medium (RPMI 1640 solution with 1! Methyl cellulose and 10% FBS) (i.e. culture medium control) and a control group containing no compounds but cells (i.e. cell control) were set. AMG510 or the following reference compounds were used as positive controls. The cell plate was placed in a cell incubator overnight. A 10-fold drug solution (RPMI1640 solution with 10% FBS and 1% DMSO) was prepared at a concentration of 10 μM, and 20 μL of this solution was added to each well of the 96-well plate seeded with cells, so that the final concentration of the compounds in each well was 1 μM, and the DMSO concentration was 0.1%. Three replicate wells were set up for each compound. The solutions of AMG510 or the following reference compounds were prepared in the same way and added to the positive control wells. The cell plate was placed in a cell incubator and cultured for 120 h. For endpoint detection, the CellTiter-Glo reagent was melted, and the cell plate was moved to room temperature to equilibrate for 30 minutes, added with 100 μL of CellTiter-Glo to each well, and shaken on an orbital shaker for 5 minutes to fully lyse the

cells. The plate was kept at room temperature for 20 minutes to stabilize the luminescence signal, and the luminescence value of each well was full wavelength scanned with a multi-functional microplate reader (Molecular Devices, Spectramax M3 microplate reader).

[Test samples] The compounds of Examples 1-39, and reference compound A

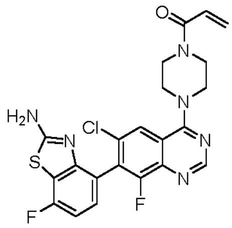

(prepared and characterized according to the method described in WO2020/081282 A1) and reference compound B

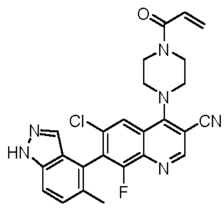

(prepared and characterized according to the method described in WO2015054572).

[Data Analysis] The following formula was used to calculate the cell inhibition rate under the action of each concentration of the compounds:

$$\text{Inhibition rate\%} = [1-(\text{Lum}_{\text{Drug to be tested}} - \text{Lum}_{\text{Culture medium control}})/(\text{Lum}_{\text{Cell control}} - \text{Lum}_{\text{Culture medium control}})]\times 100\%$$

IC50 values were calculated using GraphPad Prism 7.0 software, nonlinear Sigmoidal regression was used to fit the data to obtain a dose-response curve.

[Experimental results] The compounds of the present disclosure showed satisfactory anti-cell proliferation activities on NCI-H358 human non-small cell lung cancer cells with KRas G12C mutation. Specifically, the examples tested all showed anti-proliferation activities, and the $IC_{50}$ values are generally <1 μM, such as <0.5 μM, <0.1 μM, preferably <50 nM, more preferably <20 nM, most preferably <10 nM, such as Examples 17, 20, 32, 33, 34, and 37 all showed an $IC_{50}$ value of <50nM, and Examples 1, 2, 3, 5, 6, 8, 9, 10 and 39 showed an $IC_{50}$ value of <20nM. The data for some representative Examples are shown in Table 1.

Table 1. Inhibitory activity of representative examples on NCI-H358 cell proliferation (1 μM inhibition rate and $IC_{50}$)

| Compound | Inhibition rate% (1μM) | $IC_{50}$ (μM) |
|---|---|---|
| Example 1 | 94.7 | 0.015 |
| Example 2 | 97.6 | 0.003 |
| Example 3 | 97.7 | 0.009 |
| Example 4 | 94.8 | 0.053 |
| Example 5 | 94.9 | 0.007 |
| Example 6 | 95.0 | 0.017 |
| Example 7 | 90.0 | 0.26 |
| Example 8 | 94.0 | 0.018 |
| Example 9 | 98.8 | 0.0086 |
| Example 10 | 96.1 | 0.018 |

(continued)

| Compound | Inhibition rate% (1μM) | $IC_{50}$ (μM) |
|---|---|---|
| Ref. Comp. A | 96.9 | 0.011 |
| Ref. Comp. B | / | 0.35 |

**Example 2: Pharmacokinetic properties of the compounds of the present disclosure in rats**

2.1 Pharmacokinetic properties of some compounds of the present disclosure evaluated by Cassette pharmacokinetic experiments in rats.

[Test materials] : Male SD rats, age: 6-8 weeks, body weight 220-250g, purchased from Zhaoyan (Suzhou) New Drug Research Center Co., Ltd.; Tolbutamide (Aladdin, Cat#H1401054); Sulfobutyl-β-cyclodextrin (Captisol, Shandong Binzhou Zhiyuan Biological Technology Co., Ltd, Cat# 20191013); Propylene glycol (15)-stearate (Solutol, Meilun Bio, Cat#S0206A); DMSO (Vetec Company, Cat#WXBD0293V); acetonitrile (Sigma-Aldrich, Cat#WXBD1744V); Methanol (Sigma-Aldrich, Cat#WXBD2831V).

[Experimental procedure] : The compound combination was prepared in a solvent of 5%DMSO/10%Solutol/85% (20%Captisol), and the final concentration of each compound was 1mg/mL. The SD rats were treated with the compound preparation in 1 mL/kg by tail vein IV injection, and blood samples were collected at the time points of 5min, 15min, 30min, 1h, 2h, 4h, 8h, and 24h via external jugular vein puncture, centrifuged at low temperature for 20 minutes to collect plasma samples, which were stored at -80°C until analysis.

[Sample analysis] : Establishing LC-MS/MS compound analysis method

Standard curve preparation: 20μL of 1 mg/mL DMSO stock solution for each compound was aspirated and transferred to 900μL of 50% methanol working solution, and the resulting solution was serially diluted to obtain a standard curve working solution with concentrations of 20000, 10000, 5000, 1000, 500, 100, 50, 20 and 10ng/mL. Then 5μL of this standard curve working solution was mixed with 45 μL rat blank plasma to give a standard curve with concentrations of 2000, 1000, 500, 100, 50, 10, 5, 2 and 1ng/mL for quantification of unknown samples.

Sample pretreatment: 50μL of unknown plasma sample or standard curve sample was mixed with 250μL of acetonitrile containing internal standard as a precipitant. The plasma protein was precipitated, extracted for test compounds, and centrifuged at low temperature for 20 minutes. The supernatant was taken, mixed with 0.1% formic acid aqueous solution, and 5 μL of the resulting solution was aspirated for blood concentration analysis.

[Data processing] : A standard curve was plotted using mass spectrometry software, to quantify unknown samples, and pharmacokinetic parameters are calculated by Winnonlin 8.2 using the drug concentration of unknown samples at each time point.

[Experimental results] : The results show that the compounds of the present disclosure exhibited good or even improved pharmacokinetic properties in the cassette PK study.

Table 2.

| Example | $T_{1/2}$(h) | AUC0-t (ng·hr/mL) | Cl (mL/min/kg) |
|---|---|---|---|
| 1 | 2.87 | 657 | 20.9 |
| 9 | 0.67 | 1047 | 15.8 |
| 10 | 2.66 | 592 | 27.4 |
| 13 | 1.37 | 1946 | 8.58 |
| 14 | 1.58 | 1096 | 15.1 |
| 15 | 1.03 | 717 | 23.3 |
| 16 | 3.5 | 2787 | 5.93 |
| Ref. Comp. A | 1.00 | 623 | 27.1 |
| AMG510 | 0.49 | 231 | 71.8 |

Compound AMG510:

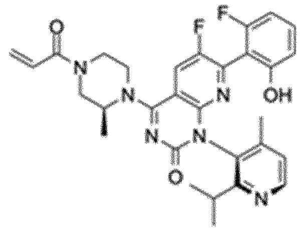

,

prepared according to the method described in Lanman B et al., J. Med. Chem. 2020, 63, 52-65.

2.2 Pharmacokinetic properties of representative examples of the present disclosure

[Test materials] Same as above 2.1.

[Experimental procedure] : IV injection administration was performed on rats according to the similar procedure described in 2.1 above, with a drug concentration of 3 mg/mL, and the drug preparation, administration and sample collection were performed by reference to 2.1 above. For the oral administration group, the drug was formulated to a 3 mg/mL suspension in 0.5% methylcellulose MC-400cp (Aladdin, M112866), and gavage administered in 10mL/kg. Blood samples were collected at the time points of 0.25, 0.5, 1, 2, 4, 6, 8 and 24 hours after administration via external jugular vein puncture, centrifuged at low temperature for 20 minutes, and the plasma samples were collected and stored at -80°C until analysis.

The subsequent sample analysis and data processing are similar to those described in 2.1.

[Experimental results]: The results show that Example 9 has excellent pharmacokinetic properties, see Table 3.

Table 3

| IV | | | | | | PO | | | | | |
|---|---|---|---|---|---|---|---|---|---|---|---|
| **Dose (mg/kg)** | **T1/2 (hr)** | **$AUC_{0\rightarrow t}$ (ng hr/mL)** | **$AUC_{0\rightarrow\infty}$ (ng hr/mL)** | **Vd (L/kg)** | **Cl (mL/min/kg)** | **Dose (mg/kg)** | **$T_{max}$ (hr)** | **$C_{max}$ (ng/mL)** | **$AUC_{0\rightarrow t}$ (ng hr/mL)** | **$AUC_{0\rightarrow\infty}$ (ng hr/mL** | **F (%)** |
| 3 | 0.67 | 3310 | 3314 | 0.63 | 15.6 | 30 | 1.33 | 2393 | 7776 | 7873 | 23.8 |

**Example 3: Antitumor activity of the compounds of the present disclosure in human non-small cell lung cancer NCI-H358 xenograft mouse model**

Tumor growth inhibitory activity of the compounds of the present disclosure was evaluated and verified in a human non-small cell lung cancer NCI-H358 xenograft mouse model.

[Test materials] : NCI-H358 cell line carrying KRAS G12C mutation, provided by KYinno Biotechnology Co., Ltd. (from ATCC, Cat#CRL-5807). NPSG mice, female, provided by Beijing Phenotek Biotechnologies Inc.

[Experimental procedure] : $5\times10^6$ NCI-H358 cells (containing 50% Matrigel) were inoculated subcutaneously on the right flank of 6-8 weeks old female NPSG mice, with the inoculation volume of 0.1 mL. When the average tumor size reached 160-220 $mm^3$, the mice were grouped randomly according to tumor size and body weight, upon which the mice were treated with compounds on the same day designated as Day 0. The mice were gavage administrated once daily at 10 mg/kg or solvent control (50mM citrate buffer containing 10% cyclodextrin, pH5.0). Tumor volume and body weight were measured twice a week during the study. The tumor volume was calculated by the formula $V=D\times d\times d/2$, wherein D and d refer to the long and short diameter of the tumor respectively. Average tumor inhibition rate was calculated by $TGI\% = [(C_{\text{Average value}} - C_{0\text{ Average value}}) - (T_{\text{Average value}} - T_{0\text{ Average value}})]/(C_{\text{Average value}} - C_{0\text{ Average value}})^*$ 100%, where T and C are the tumor volumes of the treated group and control group, respectively, and $T_0$ and $C_0$ are the initial tumor volumes of the treated group and control group, respectively.

[Experimental results] : The results are shown in Table 4, the representative examples of the present disclosure can significantly inhibit the growth of NCI-H358 tumor. Compared with the reference compound AMG510, the compounds of the present disclosure showed a comparable or improved antitumor effect under the same dosage. The body weight of the mice in each group had no significant change.

Table 4

| Group | Tumor volume ($mm^3$) (day 14) | TGI(%) |
|---|---|---|
| Solvent control group | 1170 | / |
| Example 1 | 478.4 | 71 |
| Example 2 | 497.3 | 69 |
| Example 8 | 519.2 | 67 |
| AMG510 | 525.1 | 66 |

In addition, using the same materials and procedures as above, the tumor inhibition activity of Example 9 at different doses in the above-mentioned non-small cell lung cancer mouse model and its effect on body weight were also investigated. The results are shown in Figure 1. It can be seen that the representative examples showed excellent tumor inhibition activity without significant side effects on body weight.

**Example 4: Inhibition of the compounds of the present disclosure on cytochrome P450 enzyme system**

The inhibitory effect of the compounds of the present disclosure on the cytochrome P450 enzyme system was investigated in this assay.

[Test materials] : Human liver microsomes (Corning, Cat#452161); Reduced nicotinamide adenine dinucleotide phosphate (NADPH, MCE, Cat#HY-F0003/CS-4998); Phenacetin, Diclofenac, α-naphthoflavone, Omeprazole and Ketoconazole were purchased from TCI; S-Mephenytoin and testosterone were purchased from CAYMAN; Midazolam was purchased from Bioreclamation IVT; Quinidine was purchased from Damas-beta; Sulfaphenazole was purchased from MCE; Bufuralol was purchased from TRC.

[Experimental procedure] : Preparation of 0.1M potassium phosphate buffer (K-buffer): 100 mM potassium phosphate buffer (K-buffer) was prepared with potassium dihydrogen phosphate and dipotassium hydrogen phosphate, and adjusted to pH 7.4.

Preparation of 400× test compound and reference inhibitor solutions: 8 μL of 10 mM stock solution of the test compounds was mixed with 12μL of acetonitrile. Preparation of inhibitor cocktail solution for CYP1A2, CYP2C9 and CYP2D6: 12μL of 1mM α-naphthoflavone, 10μL of 40mM Sulfaphenazole, 10μL of 10mM Quinidine and 8μL DMSO were mixed. Preparation of inhibitor cocktail solution for CYP3A4 and CYP2C19: 8 μL of DMSO solution was mixed with 12 μL of acetonitrile.

Preparation of 4×NADPH potassium phosphate solution: 66.7 mg of NADPH was added to 10mL of 0.1M K-buffer pH7.4. Preparation of 4×substrate potassium phosphate solution: the different substrates were prepared to 4-fold concentration test solutions with 10mL 0.1M K-buffer according to the concentration requirements.

Preparation of 0.2mg/mL human liver microsomes (HLM) solution: 10μL of 20mg/mL human liver microsomes was added to 990μL K-buffer, and the solution was stored in ice bath until use.

600 μL of 0.2mg/mL HLM was added to a 96-well plate, followed by the addition of 3 μL of a 400-fold test compound solution; 200 μL of 0.2 mg/mL HLM was added to a 96-well plate, and 1 μL of the diluted positive control inhibitor solution was added. 30μL of the compound solution mixed with human liver microsomes was added into a 96-well plate, and 15μL of substrate solution was added then. The solutions obtained above, and the prepared NADPH solution were pre-incubated at 37°C for 5 min. 15 μL of the pre-warmed NADPH solution was added to the reaction plate, mixed well, to initiate the reaction. The reaction plate was incubated at 37°C, for 5 minutes (3A4), for 10 minutes (1A2, 2C9, 2D6), for 45 minutes (2C19). At the end of the reaction, 120 μL of acetonitrile containing internal standard was added to quenched the reaction. Samples were vortexed for 10 min, centrifuged at 5594 g for 15 min, and prepared for LC-MS/MS analysis.

[Experimental results] : The experimental results show (Table 5) that, at the test concentration, compared with AMG510 and reference compound A, the representative compounds of the present disclosure had no significant inhibitory effect on the CYP subtypes critical for drug metabolism, showing better drug-drug interaction safety.

Table 5

| Example | CYP inhibition rate (%) at 10 μM concentration | | | | | |
|---|---|---|---|---|---|---|
| | 1A2 | 2C9 | 2C19 | 2D6 | CYP3A4 (midazolam) | CYP3A4 (testosterone) |
| 8 | 17.61 | 49.91 | 31.21 | 13.83 | 21.97 | 16.93 |
| 9 | 13.59 | 42.49 | 34.92 | -11.44 | 23.99 | 30.91 |
| Ref. Comp. A | 72.97 | 52.40 | 74.27 | 32.59 | 11.09 | 10.91 |
| AMG510 | 10.34 | 26.24 | 11.07 | 23.67 | 63.68 | 24.75 |

**Example 5: Inhibition effect of the compounds of the present disclosure on proliferation of a series of KRas mutant cells**

In this assay, the CellTiter-Glo (CTG) kit of Promega was used to evaluate the anti-proliferation activities of the compounds of the present disclosure on 12 KRas mutant tumor cell lines.

[Test materials] : RPMI1640 medium (Hyclone, Cat#SH30809.01); fetal bovine serum (FBS) (Gibco, Cat#10099-141); phosphate buffer PBS (Solarbio, Cat#P1020-500); DMSO (Sigma, Cat#D8418-1L); detection kit CTG (Promega, Cat#G7573); 96-well cell culture plate (Thermo, Cat# 3100 Series); Vibrator (QILINBEIER, Cat#QB-9001); Incubator (Thermo Scientific, Cat#Model 3100 series); microscope (OLYMPUS, Cat#CKX41SF); multi-function microplate reader (BMG LABTECH, Cat#CLARIOstar® Plus); biological safety cabinet (Thermo, Cat# Model 1300 Series A2). All cell lines used in the following assay were purchased from KYinno Biotechnology Co., Ltd.

[Experimental procedure] :

| Cell line | Mutation type | Tissue source | Growth characteristics | Complete medium |
|---|---|---|---|---|
| H23 | KRas-G12C | Lung | Adherent | RPMI-1640 + 10% FBS |
| H1373 | KRas-G12C | Lung | Adherent | RPMI-1640 + 10% FBS |
| MIA-Paca-2 | KRas-G12C | Pancreas | Adherent | DMEM+10%FBS |
| SW837 | KRas-G12C | Colorectal | Adherent | RPMI-1640 + 10% FBS |
| Kyse-410 | KRas-G12C | Esophageal | Adherent | RPMI-1640 + 10% FBS |
| HCT116 | KRas-G13D | Colorectal | Adherent | RPMI-1640 + 10% FBS |
| T84 | KRas-G13D | Colorectal | Adherent | DMEM+10%FBS |
| A549 | KRasG12S | Lung | Adherent | DMEM+10%FBS |

Each of the above cell lines was cultured in the indicated complete medium at 37°C, 5% $CO_2$, and the cells in the logarithmic growth phase were harvested and counted using a platelet counter. The cell viability was detected by the trypan blue exclusion method to ensure that the cell viability was above 90%. Cell density was adjusted using complete medium, and then inoculated into 96-well cell culture plates, at 90 μL and a total of 3000 cells per well. The cells in the 96-well plate were cultured at 37°C and 5% $CO_2$.

10× the test compound solution in culture medium was prepared, the top concentration was 10 μM, serially diluted at a dilution factor of 3.16 to afford 9 concentrations. 10 μL of each diluted compound solution was transferred to the corresponding experimental well of the 96-well cell plate and each concentration was set three replicate wells. The cells in the 96-well plate that had been dosed were incubated at 37°C and 5% $CO_2$ for 72 hours, and then CTG analysis was performed.

The CTG reagent was melted, and the cell plate was moved to room temperature to equilibrate for 30 minutes. 100 μL of CTG solution was added to each well, and shaken on an orbital shaker for 5 minutes to fully lyse the cells. The plate was kept at room temperature for 20 minutes to stabilize the luminescence signal, and the luminescence value of each well was scanned and the data was collected. GraphPad Prism 7.0 software was used to analyze the data, and the nonlinear Sigmoidal regression was used to fit the data to obtain a dose-response curve. The relative and absolute IC50 values and the maximum inhibition rate were calculated according to conventional methods well known to those skilled in the art.

$$\text{Inhibition rate\%} = [1- (\text{Lum}_{\text{Drug to be tested}} - \text{Lum}_{\text{Culture medium control}})/(\text{Lum}_{\text{Cell control}} - \text{Lum}_{\text{Culture medium control}})]\times 100\%$$

[Experimental results] :

Table 6.

| | | | Relative IC50(uM) | | Absolute IC50(uM) | | Maximum inhibition% | |
|---|---|---|---|---|---|---|---|---|
| Cell Line | Mutation | Cancer Type | Ex. 9 | Ref. Comp. A | Ex. 9 | Ref. Comp. A | Ex. 9 | Ref. Comp. A |
| H23 | $KRas^{G12C}$ | Lung cancer | 0.205 | 0.32 | 0.273 | 0.521 | 94.38 | 85.92 |
| H1373 | $KRas^{G12C}$ | Lung cancer | 1.639 | 1.918 | 1.506 | 3.24 | 80.29 | 65.19 |
| MIA-PaCa-2 | $KRas^{G12C}$ | Pancreatic cancer | 0.036 | 0.041 | 0.081 | 0.092 | 85.93 | 74.54 |
| SW837 | $KRas^{G12C}$ | Rectal cancer | 0.017 | 0.018 | 0.431 | >10 | 59.99 | 48.61 |
| Kyse-410 | $KRas^{G12C}$ | Esophageal cancer | 2.036 | 5.631 | 1.573 | 7.239 | 77.5 | 58.34 |
| HCT116 | $KRas^{G13D}$ | Colorectal cancer | 5.889 | >10 | 7.063 | >10 | 62.01 | 29.26 |
| T84 | $KRas^{G13D}$ | Colon cancer | >10 | >10 | >10 | >10 | 14.73 | 4.59 |
| A549 | $KRas^{G12S}$ | Lung cancer | >10 | >10 | >10 | >10 | 44.1 | 19.67 |

The results in Table 6 show that the representative compounds of the present disclosure exhibited an anti-proliferation activity superior to that of the reference compound A in a series of KRas tumor cells and had good selectivity.

**Example 6: Inhibition of the compounds of the present disclosure on proliferation of KRas G12C Inhibitor-resistant cell model**

Acquired resistance is one of the key factors that affect the treatment efficacy of KRas G12C inhibitors. In this assay, the potential application value of the compounds of the present disclosure in a known resistant model was evaluated by measuring in an acquired resistance model of KRas G12C inhibitor Adagrasib (Engl J Med 2021; 384:2382-93).

In this assay, the CellTiter-Glo (CTG) kit provided by Promega was used to evaluate the effect of the tested compounds on the cell proliferation of 5 KRas cell strains (with acquired resistance to the KRas G12C inhibitor Adagrasib) with the reference compound A and AMG510 as control compounds.
[Test materials] : The experimental materials and equipments used in this assay are the same as those listed in Example 5 above. All cell lines used in this assay were provided by KYinno Biotechnology Co., Ltd.

[Experimental procedure] :

| Cell Line | Cell Line Type | Growth Characteristics | Complete Medium |
|---|---|---|---|
| BaF3-KRas-G12C-Y96C | BA/F3 Cell | suspension | RPMI-1640 + 10% FBS |
| BaF3-KRas-G12C-Y96D | BA/F3 Cell | suspension | RPMI-1640 + 10% FBS |
| BaF3-KRas-G12C-R68S | BA/F3 Cell | suspension | RPMI-1640 + 10% FBS |
| BaF3-KRas-G12C-H95Q | BA/F3 Cell | suspension | RPMI-1640 + 10% FBS |
| BaF3-KRas-G12C-H95D | BA/F3 Cell | suspension | RPMI-1640 + 10% FBS |

Following the same procedures and conditions as Example 5, the inhibitory activity of the test compounds on cell growth was investigated, and the results are shown in Table 7.

Table 7.

| Cell Line | IC50 (uM) | | |
|---|---|---|---|
| | Ex. 9 | Ref. Comp. A | AMG510 |
| BaF3-KRas-G12C-Y96C | 0.31 | 0.55 | >10 |
| BaF3-KRas-G12C-Y96D | 1.29 | 3.24 | >10 |
| BaF3-KRas-G12C-R68S | 0.56 | 1.79 | 2.03 |
| BaF3-KRas-G12C-H95Q | 0.091 | 0.12 | 0.081 |
| BaF3-KRas-G12C-H95D | 0.10 | 0.15 | 0.11 |

The results show that the compounds of the present disclosure exhibited an inhibitory activity superior to those of the reference compound A and AMG510 in the above resistant cell modela.

**Example 7: In vivo pharmacodynamic study of the compounds of the present disclosure on a subcutaneous xenograft tumor model of human pancreatic cancer Mia PaCa-2 cells in BALB/c nude mouse**

The in vivo efficacy of the representative compounds of the present disclosure in the subcutaneous xenograft tumor model of human pancreatic cancer Mia PaCa-2 cells was evaluated.
[Test materials] : BALB/c nude mice of 6-8 weeks, female, Zhejiang Vital River Laboratory Animal Technology Co., Ltd. Human pancreatic cancer Mia PaCa-2 cells (ATCC, Cat#: CRL-1420). Matrigel (Matrigel, Corning, Cat. No. 356234).
[Experimental procedure] : 0.2mL ($5\times10^6$) Mia PaCa-2 cells (plus Matrigel, volume ratio 1:1) were inoculated subcutaneously on the right back dorsal of each mouse. When the average tumor volume reached about 137mm$^3$, the mice were randomly divided into experimental groups (solvent control group; AMG510 10mpk; Example 9 compound 3mpk; Example 9 compound 10mpk; Example 9 compound 30mpk) with 8 mice in each group, and gavage dosing was initiated once a day with the vehicle of 0.5% methylcellulose (400cp) aqueous solution. During the assay, tumor volumes and body weights were measured twice a week.
The tumor volume was calculated according to the following formula: $V = 0.5a \times b^2$, where $a$ and $b$ represent the long and short diameters of the tumor, respectively.
The tumor growth inhibition TGI (%) of the test compounds was calculated according to the following formula: TGI (%)=[1-(Average tumor volume of the treatment group at the end of dosing - Average tumor volume of the treatment group at the beginning of dosing)/(Average tumor volume of the solvent control group at the end of dosing - Average

tumor volume of the solvent control group at the beginning of dosing)] × 100%.

[Experimental results] : The results are shown in Table 8 and Figure 2, showing that the representative example compounds exhibited excellent target-related tumor suppressive activity on human pancreatic cancer Mia PaCa-2, superior to that of the reference compound AMG5 10, and there was no significant change in body weight in each group.

Table 8.

| Group | Tumor volume ($mm^3$) (Day 21) | TGI(%) |
|---|---|---|
| Solvent Control | 1202 | / |
| Example 9 (30mpk) | 162 | 97.7% |
| Example 9 (10mpk) | 246 | 89.8% |
| Example 9 (3mpk) | 665 | 50.4% |
| AMG510 (10mpk) | 432 | 72.3% |

## Claims

1. A compound of formula (I),

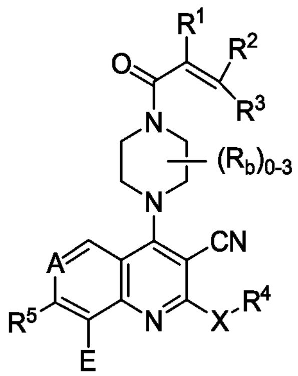

(I)

wherein,

A is selected from C-$R_a$ or N, wherein $R_a$ is selected from halogen, CN, nitro, $C_{3-6}$ cycloalkyl or $C_{1-6}$ alkyl optionally substituted by halogen;
$R^1$, $R^2$ and $R^3$ are each independently selected from H, halogen or $C_{1-6}$ alkyl, wherein the alkyl is optionally substituted by a group independently selected from halogen, $-N(R_c)_2$, $-OR_c$ or 3-8 membered heterocycloalkyl;
$R_b$ at each occurrence is independently selected from H, halogen, CN, or $C_{1-6}$ alkyl optionally substituted by halogen or CN;
X is selected from a bond, -O- or -NH-;
$R^4$ is selected from H, halogen, $C_{1-6}$ alkyl, $-C_{0-6}$ alkyl-$C_{6-10}$ aryl, $-C_{0-6}$ alkyl-$C_{3-8}$ cycloalkyl, $-C_{0-6}$ alkyl-$C_{3-8}$ cycloalkenyl, $-C_{0-6}$ alkyl-3-8 membered heterocycloalkyl, $-C_{0-6}$ alkyl-3-8 membered heterocycloalkenyl, $-C_{0-6}$ alkyl-5-10 membered heteroaryl, wherein the alkyl, aryl, cycloalkyl, cycloalkenyl, heterocycloalkyl, heterocycloalkenyl and heteroaryl groups are optionally substituted by one or more groups independently selected from halogen, $C_{1-6}$ alkyl optionally substituted by halogen, $-(CR_cR_c)_{0-6}-SR_c$, $-(CR_cR_c)_{0-6}-(CO)_{0-1}-OR_c$, $-(CR_cR_c)_{0-6}-(CO)_{0-1}-N(R_c)_2$, wherein the-$(CR_cR_c)_{0-6}-(CO)_{0-1}-N(R_c)_2$ attached to the aryl, cycloalkyl, cycloalkenyl, heterocycloalkyl, heterocycloalkenyl or heteroaryl, via the group connected to N and together with the ring atom to which it is attached and the adjacent atom, optionally form a 4-7 membered nitrogen-containing heterocyclic ring;
$R_c$ at each occurrence is independently selected from H or $C_{1-6}$ alkyl optionally substituted by halogen, or wherein two $R_c$ attached to the same carbon or nitrogen atom, together with the carbon atom or nitrogen atom to which they are attached, independently form a 3-6 membered cyclic group;
E is selected from halogen, $-O-R_d$ or $-N(R_d)_2$-, wherein each $R_d$ is independently H or $C_{1-6}$ alkyl optionally substituted by halogen;

$R^5$ is

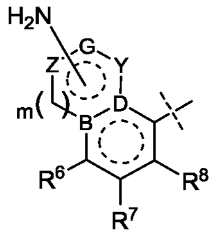

;

m is 0 or 1;

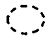 represents an aromatic ring;
B and D are each independently selected from N or C;
Z, G and Y are each independently selected from C, N, O or S;
$R^6$, $R^7$ and $R^8$ are each independently selected from H, halogen and $C_{1-6}$ alkyl optionally substituted by halogen;
with the proviso that B and D are not both N; and at most three of Z, G, Y, D and B are not C;
or an isomer, a pharmaceutically acceptable salt or a solvate thereof.

**2.** The compound of formula (I) according to claim 1 or an isomer, a pharmaceutically acceptable salt or a solvate thereof, wherein A is N or C-$R_a$, wherein $R_a$ is selected from halogen, preferably Cl.

**3.** The compound of formula (I) according to claim 1 or 2 or an isomer, a pharmaceutically acceptable salt or a solvate thereof, wherein $R^1$ is selected from H or halogen, preferably H or F.

**4.** The compound of formula (I) according to any one of claims 1-3 or an isomer, a pharmaceutically acceptable salt or a solvate thereof, wherein $R_b$ is H; or there is one $R_b$ and $R_b$ is CN or $C_{1-6}$ alkyl optionally substituted by CN; or there are two $R_b$ and $R_b$ is $C_{1-6}$ alkyl.

**5.** The compound of formula (I) according to any one of claim 1-4 or an isomer, a pharmaceutically acceptable salt or a solvate thereof, wherein $R^4$ is selected from H, halogen, $C_{1-6}$ alkyl, phenyl, $C_{3-6}$ cycloalkyl, 4-6 membered heterocycloalkyl comprising 1, 2 or 3 heteroatoms independently selected from N, O or S and 5-6 membered heteroaryl comprising 1, 2 or 3 heteroatoms independently selected from N, O or S, wherein the alkyl, phenyl, cycloalkyl, heterocycloalkyl and heteroaryl are optionally substituted by 1, 2 or 3 groups independently selected from halogen, $C_{1-6}$ alkyl optionally substituted by halogen, $-(CR_cR_c)_{0-6}-OR_c$ and $-(CR_cR_c)_{0-6}-N(R_c)_2$, where $R_c$ at each occurrence is independently selected from H or $C_{1-6}$ alkyl.

**6.** The compound of formula (I) according to any one of claims 1-5 or an isomer, a pharmaceutically acceptable salt or a solvate thereof, wherein E is halogen, preferably F, or E is $-OR_d$, $R_d$ is $C_{1-6}$ alkyl optionally substituted by halogen.

**7.** The compound of formula (I) according to any one of claims 1-6 or an isomer, a pharmaceutically acceptable salt or a solvate thereof, wherein $R^5$ is

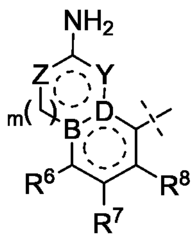

,

wherein at most two of Z, Y, B and D are not C, selected from

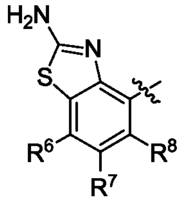,
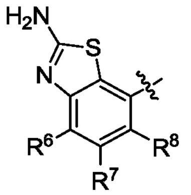,
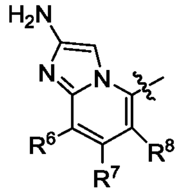,

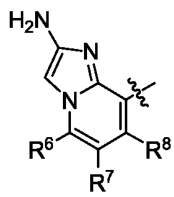,
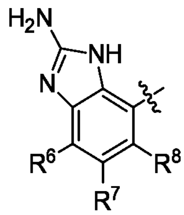,
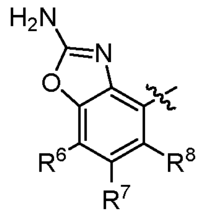,
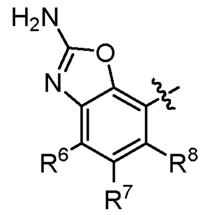,
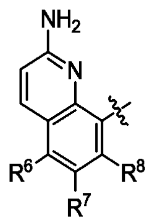,
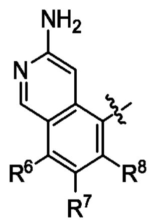,

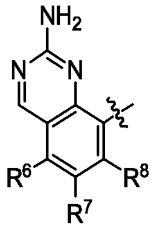,

preferably $R^5$ is

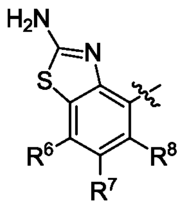.

**8.** The compound of formula (I) according to claim 7 or an isomer, a pharmaceutically acceptable salt or solvate thereof, wherein at least one of $R^6$, $R^7$ and $R^8$ is halogen, preferably F.

**9.** The compound of formula (I) according to claim 7 or an isomer, a pharmaceutically acceptable salt or a solvate thereof, wherein $R^5$ is selected from

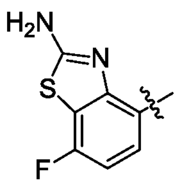,
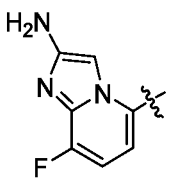,
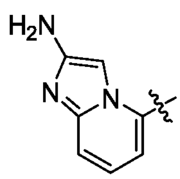,

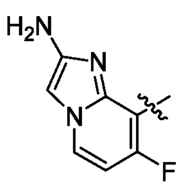,
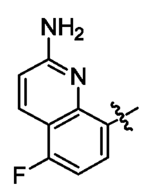,
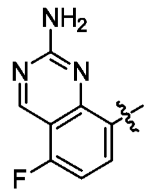;

preferably $R^5$ is

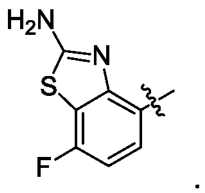

**10.** A compound of formula (II) or an isomer, a pharmaceutically acceptable salt or a solvate thereof,

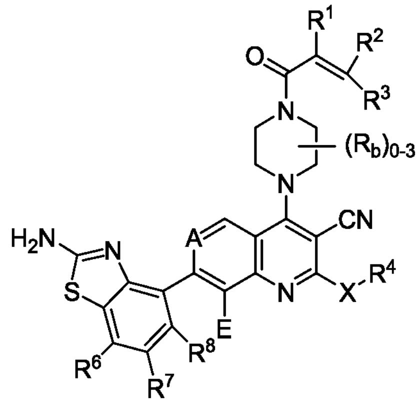

(II)

wherein:

A is selected from C-$R_a$ or N, wherein $R_a$ is selected from halogen;
$R^1$, $R^2$ and $R^3$ are each independently selected from H, halogen or $C_{1-6}$ alkyl;
$R_b$ at each occurrence is independently selected from H, halogen, CN, or $C_{1-6}$ alkyl optionally substituted by halogen or CN;
X is selected from a bond, -O- or -NH-;
$R^4$ is selected from H, halogen, $C_{1-6}$ alkyl, -$C_{0-3}$ alkyl-$C_{6-10}$ aryl, -$C_{0-3}$ alkyl-$C_{3-8}$ cycloalkyl, -$C_0$-3 alkyl-$C_{3-8}$ cycloalkenyl, -$C_{0-3}$ alkyl-3-8 membered heterocycloalkyl comprising 1, 2 or 3 heteroatoms independently selected from N, O or S, -$C_{0-3}$ alkyl-3-8 membered heterocycloalkenyl comprising 1, 2 or 3 heteroatoms independently selected from N, O or S, -$C_{0-3}$ alkyl-5-10 membered heteroaryl comprising 1, 2 or 3 heteroatoms independently selected from N, O or S, wherein the alkyl, aryl, cycloalkyl, cycloalkenyl, heterocycloalkyl, heterocycloalkenyl and heteroaryl are optionally substituted by 1, 2 or 3 groups independently selected from halogen, $C_{1-6}$ alkyl optionally substituted by halogen, -$(CR_cR_c)_{0-6}$-$OR_c$, -$(CR_cR_c)_{0-6}$-$N(R_c)_2$, where the -$(CR_cR_c)_{0-6}$-$N(R_c)_2$ attached to the aryl, cycloalkyl, cycloalkenyl, heterocycloalkyl, heterocycloalkenyl or heteroaryl, via the group connected to N and together with the ring atom to which it is attached and the adjacent atom, optionally form a 4-7 membered nitrogen-containing heterocyclic ring;
$R_c$ at each occurrence is independently selected from H or $C_{1-6}$ alkyl optionally substituted by halogen;
E is selected from halogen, -O-$R_d$ or -N$(R_d)_2$-, wherein each $R_d$ is independently H or $C_{1-6}$ alkyl optionally substituted by halogen;
$R^6$, $R^7$ and $R^8$ are each independently selected from H, halogen and $C_{1-6}$ alkyl optionally substituted by halogen;

or an isomer, a pharmaceutically acceptable salt or a solvate thereof.

**11.** The compound of formula (II) according to claim 10 or an isomer, a pharmaceutically acceptable salt or a solvate thereof, wherein A is C-$R_a$, wherein $R_a$ is halogen, preferably Cl.

**12.** The compound of formula (II) according to claim 10 or an isomer, a pharmaceutically acceptable salt or a solvate thereof, wherein at least one of $R^6$, $R^7$ and $R^8$ is halogen, preferably F.

**13.** The compound of formula (II) according to claim 12 or an isomer, a pharmaceutically acceptable salt or a solvate

thereof, wherein $R^6$ is F, and $R^7$ and $R^8$ are H.

**14.** The compound of formula (II) according to claim 10 or an isomer, a pharmaceutically acceptable salt or a solvate thereof, wherein E is halogen, preferably F.

**15.** The compound of formula (II) according to claim 10 or an isomer, a pharmaceutically acceptable salt or a solvate thereof, wherein E is $-O-R_a$, and $R_d$ is $C_{1-6}$ alkyl optionally substituted by halogen.

**16.** The compound of formula (II) according to any one of claims 10-15 or an isomer, a pharmaceutically acceptable salt or a solvate thereof, wherein $R^4$ is selected from H, halogen, $C_{1-6}$ alkyl, phenyl, $C_{3-6}$ cycloalkyl, 4-6 membered heterocycloalkyl comprising 1, 2 or 3 heteroatoms independently selected from N, O or S, and 5-6 membered heteroaryl comprising 1, 2 or 3 heteroatoms independently selected from N, O or S, wherein the alkyl, aryl, cycloalkyl, heterocycloalkyl and heteroaryl are optionally substituted by 1, 2 or 3 groups independently selected from halogen, $C_{1-6}$ alkyl optionally substituted by halogen, $-(CR_cR_c)_{0-6}-OR_c$ and $-(CR_cR_c)_{0-6}-N(R_c)_2$, which the $-(CR_cR_c)_{0-6}-N(R_c)_2$ attached to the aryl, cycloalkyl, cycloalkenyl, heterocycloalkyl, heterocycloalkenyl or heteroaryl, via the group connected to N and together with the ring atom to which it is attached and the adjacent atom, optionally form a 4-7 membered nitrogen-containing heterocyclic ring.

**17.** A compound selected from

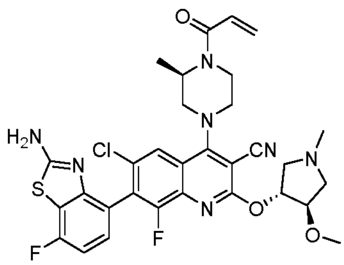
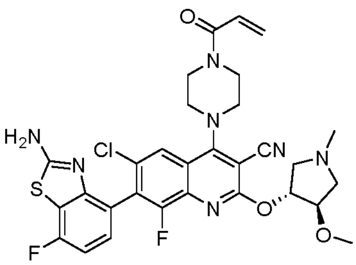
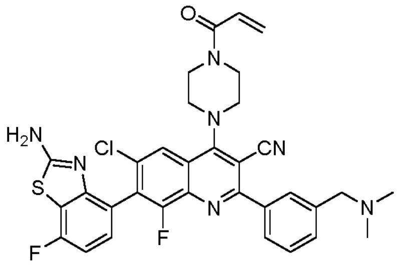

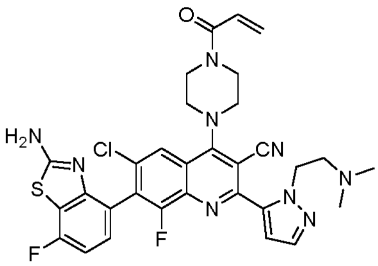
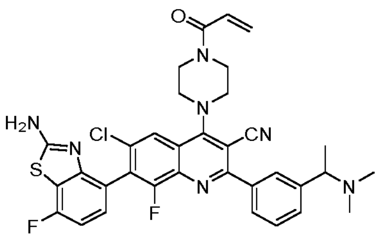
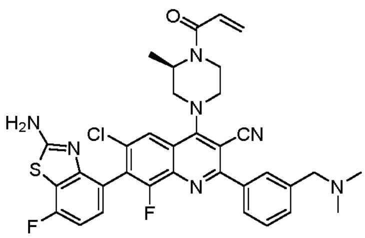

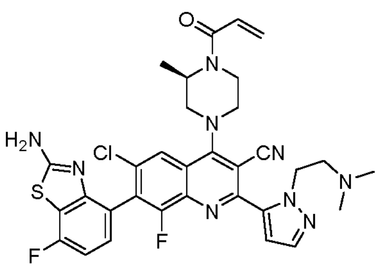
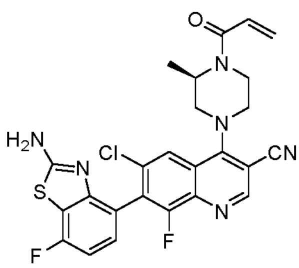
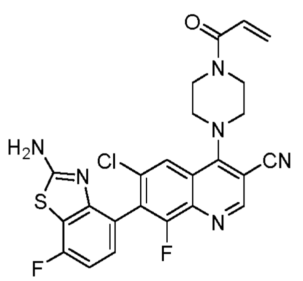

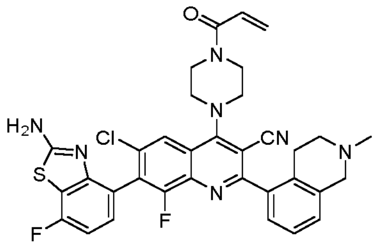
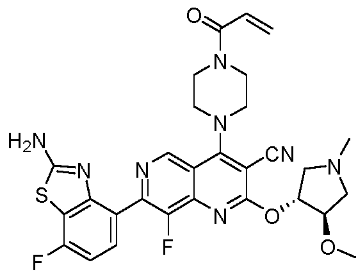
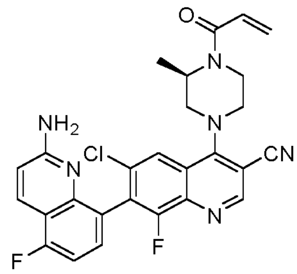

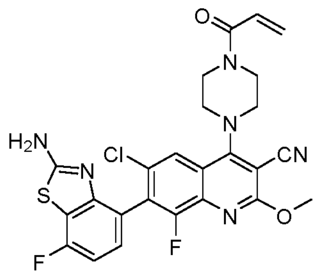
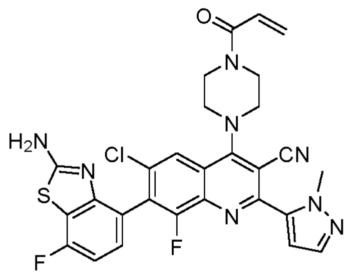
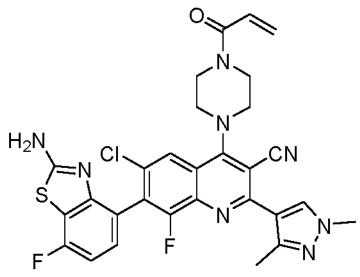

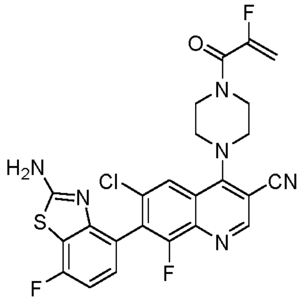
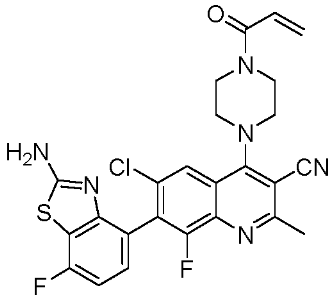
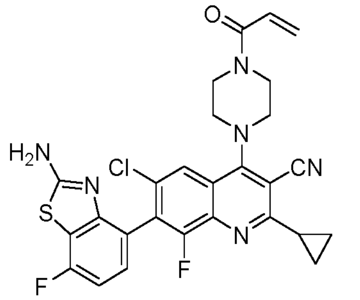

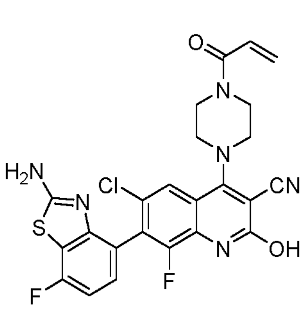
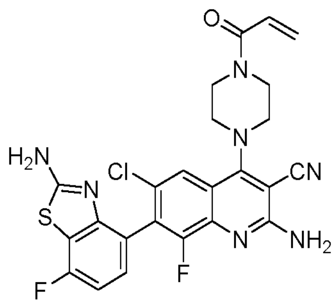
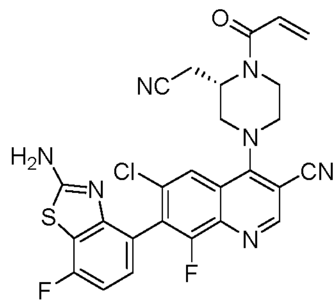

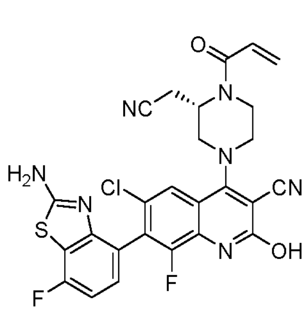
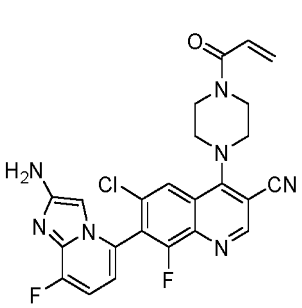
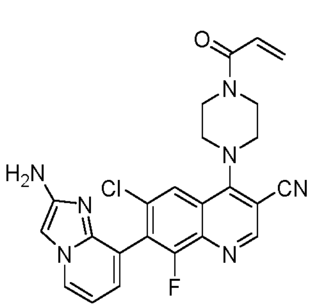

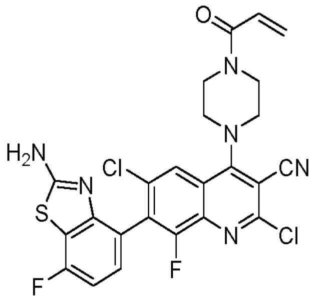
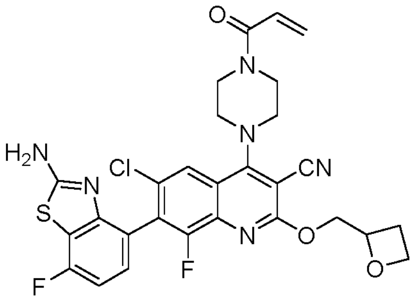
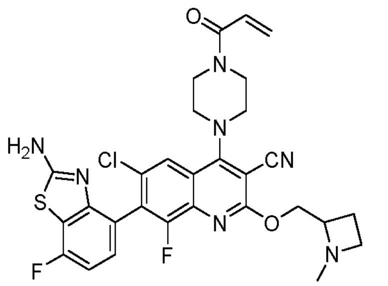
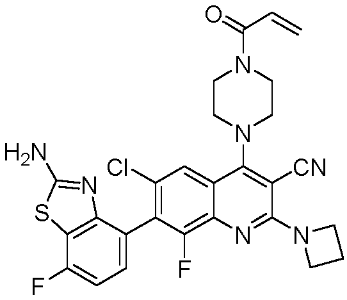
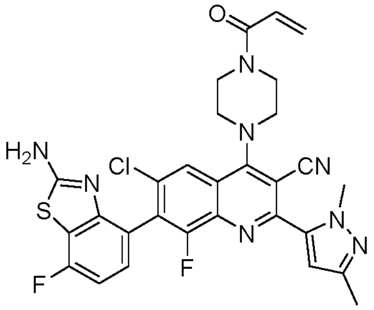
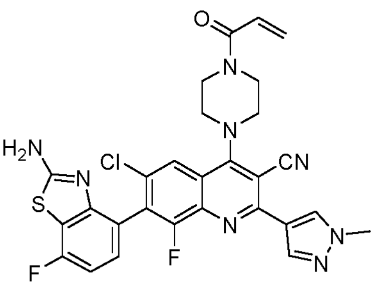
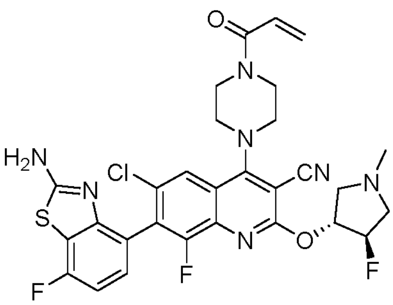
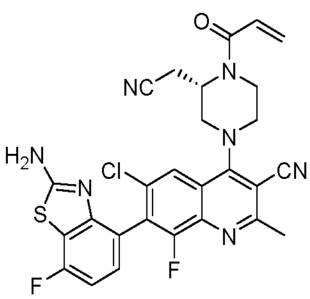
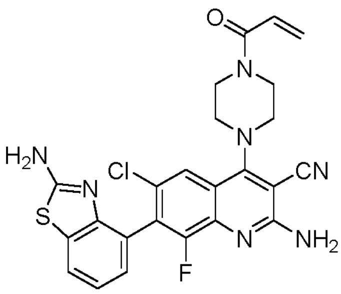
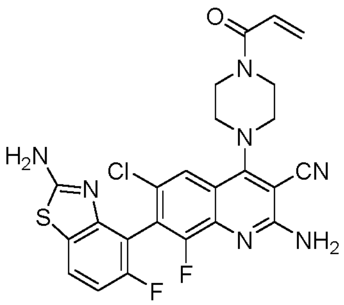
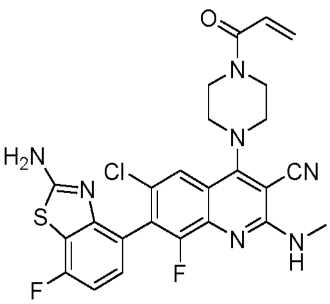
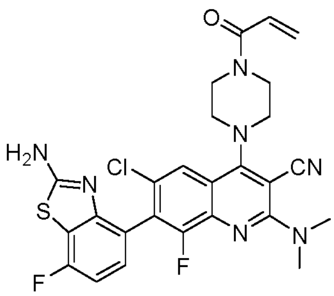
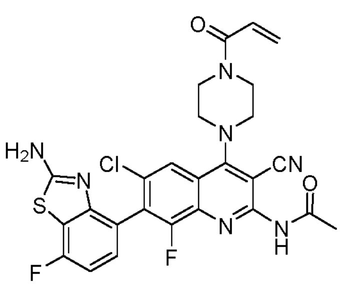
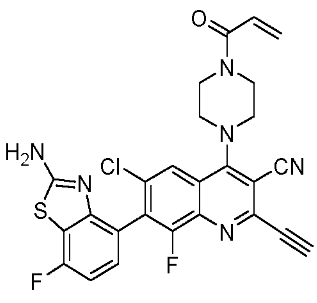
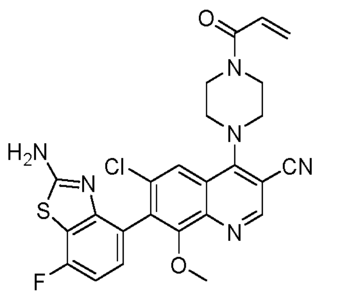

or an isomer, a pharmaceutically acceptable salt or a solvate thereof.

**18.** A pharmaceutical composition comprising the compound according to any one of claims 1-17, an isomer or a

pharmaceutically acceptable salt or solvate thereof, and a pharmaceutically acceptable excipient.

**19.** The compound according to any one of claims 1-17, an isomer or a pharmaceutically acceptable salt or a solvate thereof for use as medicine, preferably for the treatment and/or prevention of a KRas mutation, preferably KRas G12C mutation-mediated disease.

**20.** A method for treating and/or preventing a disease mediated by a Ras mutation, preferably KRas G12C, comprising administering a therapeutically effective amount of the compound according to any one of claims 1-17, an isomer or a pharmaceutically acceptable salt or a solvate thereof or the pharmaceutical composition according to claim 18.

**21.** Use of the compound according to any one of claims 1-17, an isomer or a pharmaceutically acceptable salt or a solvate thereof or the pharmaceutical composition according to claim 18 for preventing or treating a disease mediated by a KRas mutation, preferably KRas G12C mutation.

**22.** Use of the compound according to any one of claim 1-17, an isomer or a pharmaceutically acceptable salt or a solvate thereof or the pharmaceutical composition according to claim 18 in the manufacture of a medicament for preventing or treating a disease mediated by a KRas mutation, preferably KRas G12C mutation.

**23.** The use according to claim 21 or 22, wherein the disease mediated by a KRas mutation, preferably KRas G12C mutation is selected from lung cancer, lung adenocarcinoma, bone cancer, pancreatic cancer, skin cancer, head and neck cancer, skin or intraocular melanoma, uterine cancer, ovary cancer, rectum cancer, cancer in anal region, stomach cancer, colon cancer, breast cancer, carcinoma of fallopian tube, endometrial cancer, cervical cancer, carcinoma of vagina, carcinoma of vulva, Hodgkin's disease, esophagus cancer, carcinoma of small intestine, carcinoma of endocrine system, thyroid cancer, parathyroid cancer, adrenal gland cancer, soft tissue sarcoma, urethral cancer, penis cancer, prostate cancer, chronic or acute leukemia, lymphocytic lymphoma, bladder cancer, carcinoma of kidney or ureter, renal cell carcinoma, carcinoma of renal pelvis, central nervous system tumor (CNS), primary CNS lymphoma, spine tumor, brainstem glioma, or pituitary adenoma.

**24.** The use according to claim 23, wherein the disease mediated by a KRas mutation, preferably KRas G12C mutation, is selected from lung adenocarcinoma, lung cancer, colon cancer, rectum cancer, pancreatic cancer, cholangiocarcinoma, endometrial cancer, ovarian cancer, leukemia; most preferably selected from lung adenocarcinoma, colon cancer, rectum cancer, pancreatic cancer, cholangiocarcinoma.

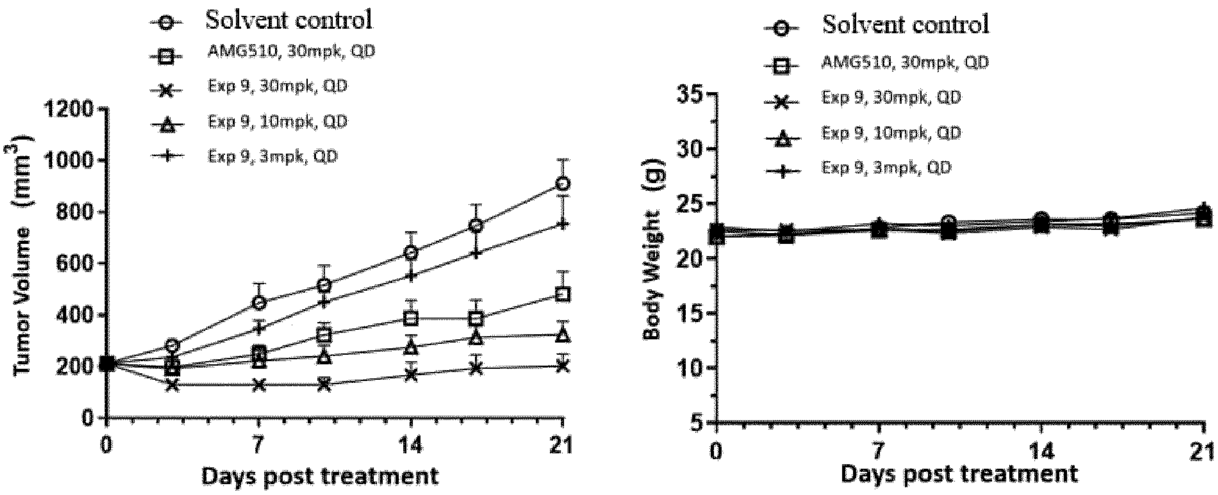


**Figure 1**

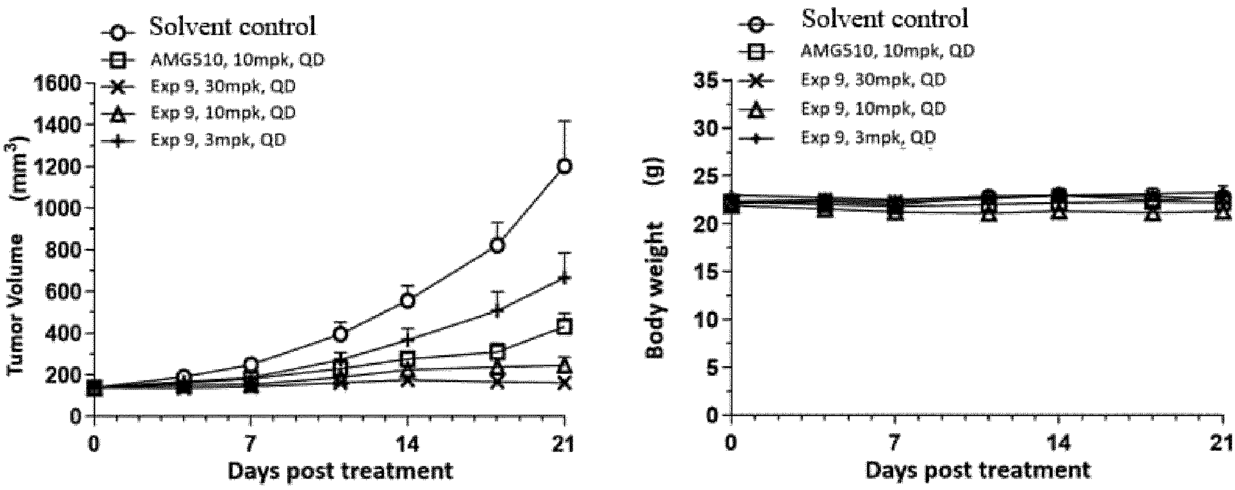


**Figure 2**

## INTERNATIONAL SEARCH REPORT

International application No.
**PCT/CN2021/128977**

**A. CLASSIFICATION OF SUBJECT MATTER**

C07D 471/00(2006.01)i； C07D 471/04(2006.01)i； C07D 401/00(2006.01)i； C07D 401/02(2006.01)i； A61K 31/517(2006.01)i； A61K 31/496(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNTXT; CNABS; DWPI; SIPOABS; CNKI; Web of Science; STNext: 肿瘤, 癌, KRas突变, KRas G12C突变, 泰励生物科技, 尚尔昌, tumor, cancer, KRas, KRas G12C, 基于式I化合物的结构检索, a structural search based on the compound of formula I

**C. DOCUMENTS CONSIDERED TO BE RELEVANT**

| Category* | Citation of document, with indication, where appropriate, of the relevant passages | Relevant to claim No. |
|---|---|---|
| PX | CN 113563323 A (SHANGHAI LINGDA BIOMEDICAL CO., LTD. et al.) 29 October 2021 (2021-10-29)<br>claims 1-13 | 1-24 |
| PY | CN 112142735 A (SHANGHAI LINGDA BIOMEDICAL CO., LTD. et al.) 29 December 2020 (2020-12-29)<br>claims 1 and 7-10, and description, embodiment 14 | 7-24 |
| X | CN 110869357 A (ARAXES PHARMA L.L.C.) 06 March 2020 (2020-03-06)<br>claims 1-42, and description, compound 1 in a table | 1-6, 18-24 |
| Y | CN 110869357 A (ARAXES PHARMA L.L.C.) 06 March 2020 (2020-03-06)<br>claims 1-42, and description, compound 1 in the table | 7-24 |
| X | CN 106488910 A (ARAXES PHARMA L.L.C.) 08 March 2017 (2017-03-08)<br>claims 1-95, and description, compounds 269, 291, 293, 308, 310, and 336 in the table | 1-6, 18-24 |
| Y | CN 106488910 A (ARAXES PHARMA L.L.C.) 08 March 2017 (2017-03-08)<br>claims 1-95, and description, compounds 269, 291, 293, 308, 310, and 336 in the table | 7-24 |
| Y | WO 2020081282 A1 (ELI LILLY AND COMPANY) 23 April 2020 (2020-04-23)<br>claims 1-4 and 11-17 | 7-24 |

✓ Further documents are listed in the continuation of Box C. ✓ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 January 2022** | **29 January 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/128977**

**C. DOCUMENTS CONSIDERED TO BE RELEVANT**

| Category* | Citation of document, with indication, where appropriate, of the relevant passages | Relevant to claim No. |
|---|---|---|
| Y | CN 110869358 A (ARAXES PHARMA L.L.C.) 06 March 2020 (2020-03-06) claims 1-45, and description, compounds 2, 3, 5-8, 12, 28, 37, 38, 48-50, 53-54, 58-59, 66-74, 78-80, and 90-93 | 7-24 |
| A | CN 107849022 A (ARAXES PHARMA L.L.C.) 27 March 2018 (2018-03-27) claims 1, 3, 6-8, 11-16, and 89-102 | 1-24 |
| A | CN 108779097 A (ARAXES PHARMA L.L.C.) 09 November 2018 (2018-11-09) claims 1-86 | 1-24 |

Form PCT/ISA/210 (second sheet) (January 2015)

**INTERNATIONAL SEARCH REPORT**

International application No. **PCT/CN2021/128977**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **20-21**
because they relate to subject matter not required to be searched by this Authority, namely:

[1] Claim 20 sets forth a method for treating and/or preventing diseases mediated by Ras mutations, preferably KRas G12C; and claim 21 sets forth a use of the compound according to any one of claims 1-17, an isomer or pharmaceutically acceptable salt or solvate thereof, or the pharmaceutical composition according to claim 18, in the prevention or treatment of diseases mediated by Ras mutations, preferably KRas G12C. Said claims relate to a method for treatment of a human body or animal body, belonging to the subject matter defined in PCT Rule 39.1(iv) that does not warrant a search.

[2] This search report is completed on the basis of the following amendment of claims 20-21: a use of the compound according to any one of claims 1-17, an isomer or pharmaceutically acceptable salt or solvate thereof, or the pharmaceutical composition according to claim 18, in the preparation of a drug for the prevention or treatment of diseases mediated by Ras mutations, preferably KRas G12C.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/128977**

| Patent document cited in search report | Publication date (day/month/year) | Patent family member(s) | Publication date (day/month/year) |
|---|---|---|---|
| CN 113563323 A | 29 October 2021 | WO 2021218110 A1 | 04 November 2021 |
| CN 112142735 A | 29 December 2020 | None | |
| CN 110869357 A | 06 March 2020 | TW 201906832 A | 16 February 2019 |
| | | US 2019062314 A1 | 28 February 2019 |
| | | US 10736897 B2 | 11 August 2020 |
| | | WO 2018218071 A1 | 29 November 2018 |
| | | EP 3630746 A1 | 08 April 2020 |
| | | JP 2020521741 A | 27 July 2020 |
| CN 106488910 A | 08 March 2017 | NI 201600049 A | 20 May 2016 |
| | | NO 20160646 A1 | 19 April 2016 |
| | | EP 3055290 A1 | 17 August 2016 |
| | | EP 3055290 B1 | 02 October 2019 |
| | | CA 2926328 A1 | 16 April 2015 |
| | | BR 112016008016 A2 | 12 September 2017 |
| | | BR 112016008016 B1 | 19 January 2021 |
| | | EA 201690752 A1 | 29 July 2016 |
| | | EA 033689 B1 | 18 November 2019 |
| | | EA 033689 B9 | 29 April 2020 |
| | | PH 12016500538 A1 | 13 June 2016 |
| | | PH 12016500538 B1 | 13 June 2016 |
| | | AU 2014331794 A1 | 21 April 2016 |
| | | AU 2014331794 B2 | 04 April 2019 |
| | | AU 2014331794 C1 | 12 September 2019 |
| | | EP 3636639 A1 | 15 April 2020 |
| | | IL 244699 D0 | 21 April 2016 |
| | | IL 244699 A | 30 November 2020 |
| | | SG 11201602662 Y A | 30 May 2016 |
| | | MX 2016004360 A | 19 August 2016 |
| | | KR 20160076519 A | 30 June 2016 |
| | | WO 2015054572 A1 | 16 April 2015 |
| | | JP 2016532656 A | 20 October 2016 |
| | | JP 6559123 B2 | 14 August 2019 |
| WO 2020081282 A1 | 23 April 2020 | JP 6824475 B1 | 03 February 2021 |
| | | JP 2021505640 A | 18 February 2021 |
| | | TW 202033518 A | 16 September 2020 |
| | | CN 112805281 A | 14 May 2021 |
| | | US 2020115375 A1 | 16 April 2020 |
| | | US 10968214 B2 | 06 April 2021 |
| | | EP 3867251 A1 | 25 August 2021 |
| | | AR 116604 A1 | 26 May 2021 |
| CN 110869358 A | 06 March 2020 | KR 20200010306 A | 30 January 2020 |
| | | PH 12019550248 A1 | 11 January 2021 |
| | | BR 112019024674 A2 | 16 June 2020 |
| | | EP 3630745 A2 | 08 April 2020 |
| | | WO 2018218070 A2 | 29 November 2018 |
| | | WO 2018218070 A3 | 07 February 2019 |
| | | US 2021024501 A1 | 28 January 2021 |
| | | US 2019062313 A1 | 28 February 2019 |
| | | US 10745385 B2 | 18 August 2020 |

Form PCT/ISA/210 (patent family annex) (January 2015)

**INTERNATIONAL SEARCH REPORT**
**Information on patent family members**

International application No.
**PCT/CN2021/128977**

| Patent document cited in search report | Publication date (day/month/year) | Patent family member(s) | Publication date (day/month/year) |
|---|---|---|---|
| | | AU 2018271990 A1 | 12 December 2019 |
| | | JP 2020521742 A | 27 July 2020 |
| | | TW 201900633 A | 01 January 2019 |
| | | CA 3063440 A1 | 29 November 2018 |
| CN 107849022 A | 27 March 2018 | EP 3280708 A1 | 14 February 2018 |
| | | EP 3280708 B1 | 01 September 2021 |
| | | US 2019284144 A1 | 19 September 2019 |
| | | US 10829458 B2 | 10 November 2020 |
| | | MX 2017012979 A | 28 November 2017 |
| | | AU 2016245864 A1 | 26 October 2017 |
| | | AU 2016245864 B2 | 22 October 2020 |
| | | AU 2016245864 C1 | 09 September 2021 |
| | | BR 112017021869 A2 | 11 December 2018 |
| | | JP 2018513853 A | 31 May 2018 |
| | | KR 20180005178 A | 15 January 2018 |
| | | TW 201702232 A | 16 January 2017 |
| | | CA 2981530 A1 | 13 October 2016 |
| | | UY 36612 A | 30 November 2016 |
| | | HK 1248231 A1 | 12 October 2018 |
| | | JP 2021098721 A | 01 July 2021 |
| | | JO 3707 B1 | 31 January 2021 |
| | | US 2016297774 A1 | 13 October 2016 |
| | | US 10246424 B2 | 02 April 2019 |
| | | WO 2016164675 A1 | 13 October 2016 |
| | | WO 2016164675 A8 | 11 May 2017 |
| | | EA 201792214 A1 | 31 January 2018 |
| CN 108779097 A | 09 November 2018 | EP 3377481 A1 | 26 September 2018 |
| | | WO 2017087528 A1 | 26 May 2017 |
| | | KR 20180081596 A | 16 July 2018 |
| | | US 2017197945 A1 | 13 July 2017 |
| | | US 10414757 B2 | 17 September 2019 |
| | | US 2020181123 A1 | 11 June 2020 |
| | | US 11021470 B2 | 01 June 2021 |
| | | TW 201726656 A | 01 August 2017 |
| | | JP 2018533611 A | 15 November 2018 |
| | | AU 2016355433 A1 | 28 June 2018 |
| | | AU 2016355433 B2 | 20 May 2021 |
| | | AU 2016355433 B9 | 03 June 2021 |
| | | MX 2018005967 A | 29 August 2018 |
| | | EA 201891191 A1 | 28 December 2018 |
| | | EA 038635 B1 | 27 September 2021 |
| | | EA 038635 B9 | 26 October 2021 |
| | | CA 3005089 A1 | 26 May 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 10256421
- US 20190144444 A1
- WO 2019110751 A1
- WO 2020081282 A1
- WO 2015054572 A


### Non-patent literature cited in the description


- **ALAMGEER et al.** *Current Opin Pharmacol.,* 2013, vol. 13, 394-401
- **ZHI TAN et al.** *Mini-Reviews in Medicinal Chemistry,* 2016, vol. 16, 345-357
- **CHANG, E.H. et al.** *Proceedings of the National Academy of Sciences of the United States of America,* 1982, vol. 79 (16), 4848-4852
- **MCCORMICK, F. et al.** *Clinical Cancer Research,* 2015, vol. 21 (8), 1797-1801
- **DUAN NI et al.** *Pharmacology & Therapeutics, https://doi.org/10.1016/j.pharmthera.2019.06.007*
- **JOHN P. O'BRYAN et al.** *Pharmacological Research,* 2019, vol. 139, 503-511
- McGraw-Hill Dictionary of Chemical Terms. McGraw-Hill Book Company, 1984
- **ELIEL, E. ; WILEN, S.** Stereochemistry of Compounds. John Wiley & Sounds, Inc, 1994
- **BUNDGARD, H.** Design of Prodrugs. Elsevier, 1985, 7-9, 21-24
- **HIGUCHI, T. et al.** *ACS Symposium Series,* vol. 14
- **EDWARD B. ROCHE (B. ROCHE.** Bioreversible Carriers in Drug Design. Pharmaceutical Association & Pergamon Press, 1987
- **ANSEL, HOWARD C. et al.** Ansel's Pharmaceutical Dosage Forms and Drug Delivery Systems. Lippincott, Williams& Wilkins, 2004
- **E.L. ELIEL ; S. H. WILEN ; L.N. MANDER'S.** Stereochemistry of Organic Compounds. Wiley-Interscience, 1994
- **T. W. GREENE ; P. G. M. WUTS.** Protective Groups in Organic Synthesis. John Wiley & Sons, 1999
- **LANMAN B et al.** *J. Med. Chem.,* 2020, vol. 63, 52-65
- *Engl J Med,* 2021, vol. 384, 2382-93